# EUROPEAN PATENT APPLICATION

(11) **EP 1 988 168 A1**
(43) Date of publication of application: **05.11.2008**
(21) Application number: 07008887.7
(22) Date of filing: 02.05.2007
(51) Int. Cl.: C12N 15/82, C12N 15/70, C12N 15/52, C07K 14/415

(54) **Means and methods for providing ribulose bisphosphate-carboxylase-oxygenase with improved properties**

(71) Applicant: Max-Planck-Gesellschaft zur Förderung der Wissenschaften e.V., 80539 München (DE)
(72) Inventor: Hayer-Hartl, Manajit, 82288 Kottgeisering (DE); Hartl, Ulrich F., 82288 Kottgeisering (DE); Bracher, Andreas, 80336 München (DE); Saschenbrecker, Sandra, 82166 Gräfelfing (DE)
(74) Representative: Vossius & Partner

(57) **Abstract**

Described are means and methods for providing ribulose 1,5-bisphosphate-carboxylase-oxygenase (Rubisco) with improved properties. In particular, described are methods for expressing plant Rubisco in prokaryotic host cells, to methods for screening for plant Rubisco with improved properties in prokaryotic host cells, methods for producing Rubisco molecules with improved properties, to the thus produced Rubisco molecules, methods for producing transgenic plants expressing a heterologous Rubisco molecule and the corresponding transgenic plants.

## Description

The present invention relates to means and methods for providing ribulose 1,5-bisphosphate-carboxylase-oxygenase (Rubisco) with improved properties. In particular, the present invention relates to methods for expressing plant Rubisco in prokaryotic host cells, to methods for screening for plant Rubisco with improved properties in prokaryotic host cells, to methods for producing Rubisco molecules with improved properties, to the thus produced Rubisco molecules, to methods for producing transgenic plants expressing a heterologous Rubisco molecule and to the corresponding transgenic plants.

Rubisco catalyses the initial steps of two opposing reaction pathways; photosynthetic carbon fixation (CO₂ as the substrate) and photorespiration (O₂ as the substrate) (for review see Andrews and Lorimer (The Biochemistry of plants: a comprehensive treatise 10 (1987), 131-218)). The photosynthetic fixation of CO₂ results in the synthesis of usable sugars (Gutteridge and Gatenby (Plant Cell 7 (1995), 809-819)) and thus is responsible for plant growth and yield. Three structural forms of assembly of otherwise closely homologous Rubisco subunits are known (Andersson and Taylor (Arch. Biochem. Biophys. 414 (2003), 130-140); Tabita, (Phosynth. Res. 60 (1999), 1-28): Form I found mainly in plants and cyanobacteria but also in some proteobacteria is a hexadecamer, denoted as RbcL₈S₈, containing eight large subunits (RbcL; MW 50-55 kDa) and eight small subunits (RbcS: MW 12-18 kDa). The oligomer has a cylindrical shape with a diameter of -110 Å and a height of 100 Å. Four small subunits cap the top and bottom of eight large subunits, which form a tetramer of dimers. The simpler form II Rubisco found in some photosynthetic bacteria *(Rhodospirillum rubrum,* purple nonsulfur bacteria) and dinoflagellates is a dimer of RbcL subunits. Form III, found in a thermophilic archaeon, consists of five RbcL dimers. Non-assembled large subunits of plant Rubsico were observed to cofractionate with the chloroplast chaperonin 60 (also known as Rubisco Subunit Binding Protein), suggesting a critical role of chaperonin in Rubisco assembly (Barraclough and Ellis (Biochim. Biophys. Acta. 608 (1980), 18-31); Roy (Plant Cell 1 (1989), 1035-1042)).

Molecular chaperones fulfill essential functions in protein biogenesis and quality control in all types of cells (Frydman (Ann. Rev. Biochem. 70 (2001), 603-647); Hartl and Hayer-Hartl (Science 295 (2002), 1852-1858)). Although the term 'molecular chaperone' was coined to describe a role in assisting oligomeric protein assembly (Ellis (Trends Biochem. Sci 31 (2006), 395-401)), it is now commonly thought that chaperones are primarily involved with mediating polypeptide chain folding, essentially by preventing misfolding and aggregation of newly-synthesized and stress-denatured polypeptides. These mechanisms are well established, particularly for the Hsp70s and the chaperonins (Bukau et al. (Cell 125 (2006), 443-451); Hartl and Hayer-Hartl, loc. cit.). In contrast, how cells ensure the efficient assembly of folded subunits into oligomeric complexes is not well understood. Although the majority of proteins reside in oligomeric structures, surprisingly few examples of specific assembly chaperones are known (Ellis, *loc. cit.).* Hexadecameric ribulose 1,5-bisphosphate carboxylase/oxygenase (Rubisco) in chloroplasts, the most abundant enzyme in nature, has been an important paradigm in studies of protein assembly. Indeed, this process was initially thought to be mediated by the chaperonin system (Gatenby and Ellis (Ann. Rev. Cell Biol. 6 (1990), 125-149); Roy (Plant Cell 1 (1989), 1035-1042)). However, a direct role in subunit assembly is not consistent with present models of chaperonin function and attempts to reconstitute Rubisco with chaperonin alone have failed (Tabita, (Photosynth. Res. 60 (1999), 1-28)), suggesting that additional factors must participate in this process. Chaperonins are large cylindrical complexes with ATPase activity. The GroEL-type chaperonins (Cpn60s) of bacteria, chloroplasts and mitochondria consist of two heptameric rings of -60 kDa subunits, each forming a central cavity for the binding of non-native protein substrate (Frydmann, *loc. cit.;* Hartl and Hayer-Hartl, loc. cit.); Horwich et al. (Chem. Rev. 106 (2006), 1917-1930)). They function together with factors of the GroES/Cpn10 family, single-ring assemblies of seven ~10 kDa subunits, which bind transiently to the ends of the chaperonin cylinder. ATP-dependent GroES association results in the encapsulation of a single molecule of unfolded protein inside the GroEL cavity for folding to occur unimpaired by aggregation. Whereas bacterial GroEL and the mitochondrial chaperonin are homo-oligomeric, the chloroplast chaperonin is composed of homologous α and β subunits. Moreover, plant chloroplasts have two types of co-chaperone, Cpn10 and Cpn20, the latter consisting of a tandem repeat of Cpn10 units (reviewed in Hill and Hemmingsen (Cell Stress Chaperones 6 (2001), 190-200)). These features may represent an adaptation to chloroplast-specific substrate proteins.

The folding and assembly of form II Rubisco from R. *rubrum* has been reconstituted with bacterial GroEL/GroES (Goloubinoff et al. (Nature 342 (1989), 884-889)). In this reaction Rubisco subunits fold inside the chaperonin cage, followed by dimerization upon release into solution (Brinker et al. (Cell 107 (2001), 223-233)). Expression of cyanobacterial form I Rubisco of Synechococcus sp. PCC6301 (also known as *Anacystis nidulans)* in E. coli was reported to result in the chaperonin-dependent production of enzymatically active holoenzyme (Goloubinoff et al. (Nature 337 (1989), 44-47)). As shown in cyanobacteria, assembly of the RbcL₈S₈ complex is thought to involve the formation of RbcL₈ core particles, followed by the docking of unassembled small subunits (Andrews and Lorimer (J. Biol. Chem. 260 (1985), 4632-4536); Paul et al. (Biochem. 30 (1991), 10019-10026)). Recently it has been reported that the product of the *rbc*X gene, present in the intergenic space between the *rbcL* and *rbcS* genes in several cyanobacteria (Larimer and Soper (Gene 126 (1993), 85-92); Rudi et al. (J. Bacteriol. 180 (1998), 3453-3461)), can enhance the production of enzymatically active Rubisco upon coexpression with *rbcL* and *rbcS* in *E. coli* (Li and Tabita (J. Bacteriol. 179 (1997), 3793-3796); Onizuka et al. (Plant & Cell Physiol. 45 (2004), 1390-1395)). Partial inactivation of *RbcX* in Synechococcus sp. PCC7002 resulted in a substantial reduction in Rubisco solubility and activity (Li and Tabita, *loc. cit;* Onizuka et al., *loc cit.).* However, the *RbcX* gene was reported to be non-essential in Synechococcus sp. PCC7942 (Emlyn-Jones et al. (Plant Cell Physiol. 47 (2006), 1630-1640)).

As mentioned above form I Rubisco is the enzyme in plants that is responsible for CO₂ fixation and thus for plant growth. The catalytic efficiency and selectivity of Rubisco for CO₂ versus O₂ is limited and its improvement is generally regarded as a means to increase plant growth and yield. Attempts to improve the catalytic properties of form I plant Rubisco by protein engineering or the transfer of superior homologs from red algae (non-green algae) into plants have been unsuccessful so far. One problem encountered in these attempts is that plant Rubisco cannot be expressed recombinantly in active form in bacteria. In particular, the manipulation of plant, in particular higher-plant, Rubisco in heterologous hosts, such as *Escherichia coli,* is blocked by unknown requirements for folding and/or assembly that are not satisfied in the bacterial host and result in the production of aggregated, nonfunctional protein (see, e.g., Andrews and Whitney (Arch. Biochem. Biophys. 414 (2003), 159-169)).
Similarly, it is not possible so far to achieve a proper assembly of heterologous Rubisco in transgenic plants, e.g. of the superior form I Rubisco of red algae, upon expression in plants such as tobacco. Although, for example, the rbcLS operon encoding the large and small subunits of form I Rubisco from the rhodophyte *Galdieria sulfuraria* and the diatom *Phaeodactylum tricornutum* were transcriptionally and translationally active when transferred into the tobacco plastome, the foreign subunits were recovered exclusively in the insoluble fraction of leaf extracts and were apparently not able to assemble into the L₈S₈ Rubisco complex to any extent (see Andrews and Whitney (2003), *loc. cit.).* This indicates that one or more processes associated with the folding and/or assembly of the red-type Rubiscos is completely blocked in chloroplasts. Thus, attempts to provide plant Rubisco molecules with superior and improved properties, e.g., by introducing mutations and screening for improved activity, are severely hampered by the problems in expressing plant Rubisco molecules, in particular those of higher plants and red algae, in heterologous host, such as bacteria, which would allow large throughput screening. Moreover, the improvement of plants by introducing superior Rubiscos, e.g. from red algae or mutagenized forms of heterologous Rubisco, is impossible to put into practice at present because of the above described problems of proper assembly of such Rubiscos in higher plants.

Thus, there is a need in providing means and methods which allow expression of active and properly assembled Rubisco molecules, in particular form I Rubisco molecules, in heterologous hosts such as bacteria, and in heterologous organisms, such as transgenic plants.

This need is addressed by the embodiments as characterized in the claims. The present invention is based on the identification of a protein, i.e. RbcX, as a factor that interacts directly with large Rubisco subunits and is required for their efficient assembly into L₈ core particles which then associate with small Rubisco subunits to form the active enzyme. The RbcX protein is recycled in this process. It could be shown that RbcX interacts specifically with the C-terminal peptide sequence of RbcL, i.e. the large subunit of Rubisco. Moreover, the results obtained show that RbcL and RbcX sequences have co-evolved and that generally only homologous pairs of RbcL and RbcX are efficient. The results also show that heterologous pairs of RbcL and RbcX may even be inhibitory.
Moreover, the C-terminal recognition peptide for RbcX in red-algae RbcL has been found to deviate from that in cyanobacteria and in higher plants which suggests that red-algae RbcX is necessary for successful expression of red-algae Rubisco in plants.
Thus, by identifying RbcX as the factor necessary for proper assembly of RbcL and by realizing that only homologous pairs of RbcL and RbcX work properly together and allow assembly of RbcL, the present invention now allows to provide efficient means and methods for expressing active plant Rubisco in heterologous hosts, such as bacteria, in order to establish screening assays for identifying Rubisco variants with improved properties, and for producing transgenic plants expressing a heterologous Rubisco by simultaneously introducing the corresponding RbcX protein which ensures proper assembly of RbcL.

Accordingly, in a first aspect, the present invention relates to a method for expressing a form I Rubisco of plant origin in a prokaryotic host cell comprising:
(i) expressing in the prokaryotic host cell a nucleic acid sequence encoding the large subunit of a plant form I Rubisco (RbcL) and a nucleic acid sequence encoding the small subunit of a plant form I Rubisco (RbcS); and
(ii) expressing in the prokaryotic host cell a nucleic acid sequence encoding a plant RbcX protein;
wherein the RbcX protein is derived from the same plant species from which the RbcL subunit is derived.

It has been found that the RbcX protein which has previously only been described to occur in some cyanobacteria, plays a crucial role in the proper assembly of the L₈ core complex of form I Rubisco by directly interacting with RbcL and by acting as a chaperon. Moreover, it has been shown that proteins related to the RbcX previously described to occur in cyanobacteria also occur in plants. Finally, evidence provided by the appended Examples shows that RbcX can only efficiently promote assembly of the L₈ core complex if the RbcL subunit is derived from the same species as the RbcX protein, i.e. if RbcL and RbcX are homologous with respect to each other, and that the use of a heterologous RbcX/RbcL pair may actually be inhibitory to the whole assembly process. Thus, the present invention provides crucial information for allowing the successful expression of assembled plant (L₈S₈) Rubisco protein in heterologous host cells.

The term "Rubisco" in the context of the present invention refers to an enzyme also referred to as D-ribulose-1,5-biphosphate carboxylase/oxygenase (EC 4.1.1.39). This enzyme provides for net synthesis of carbohydrate from atmospheric carbon dioxide by catalyzing the carboxylation of ribulose-P₂ (ribulose-P₂+CO₂ → 2 D-3-phosphoglycerate), i.e. the initial step of the reductive pentose phosphate pathway.
The term "form I Rubisco" relates to the Rubisco found in plants (and also in cyanobacteria and some proteobacteria) which is characterized in that it is a hexadecamer comprised of eight large subunits (referred to in the following as "RbcL"; but sometimes also designated "LSU" or simply "L") and eight small subunits (referred to in the following as "RbcS"; but sometimes also designated "SSU" or simply "S"). The hexadecameric complex of eight large and eight subunits is generally referred to as L₈S₈. RbcL has a molecular weight of between 50 and 55 kDa and RbcS has a molecular weight of between 12 and 18 kDa, depending on the origin. The skilled person is aware of means and methods for determining whether a given Rubisco belongs to form I, form II or form III. In particular, it has become standard in taxonomic approaches for classifying an organism to determine the sequence of any possibly occurring Rubisco subunit genes and to verify whether they belong to form I, II or III. Accordingly, in the meantime a multitude of nucleotide sequences encoding form I, II or III Rubiscos is available as well as corresponding amino acid sequences and it has turned out that the conservation of the sequences encoding form I Rubisco is rather high and lies at about at least 70%. This means that any known form I Rubisco sequence has at least about 70% sequence identity to any other known form I Rubisco sequence, preferably at least 75% and more preferably at least 80%.

On the other hand there is only a low degree of sequence identity between sequences encoding form I Rubisco and those encoding form II Rubisco. Here the sequence identity which can be found lies only in the range of about 45% to 50%. Similarly, sequences encoding form II Rubisco subunits show generally a high degree of sequence identity when compared to each other, e.g. at least about 70%, preferably at least about 80%, but there is only a low degree of sequence identity between form I and form III or form II and form III sequences (about 30%).

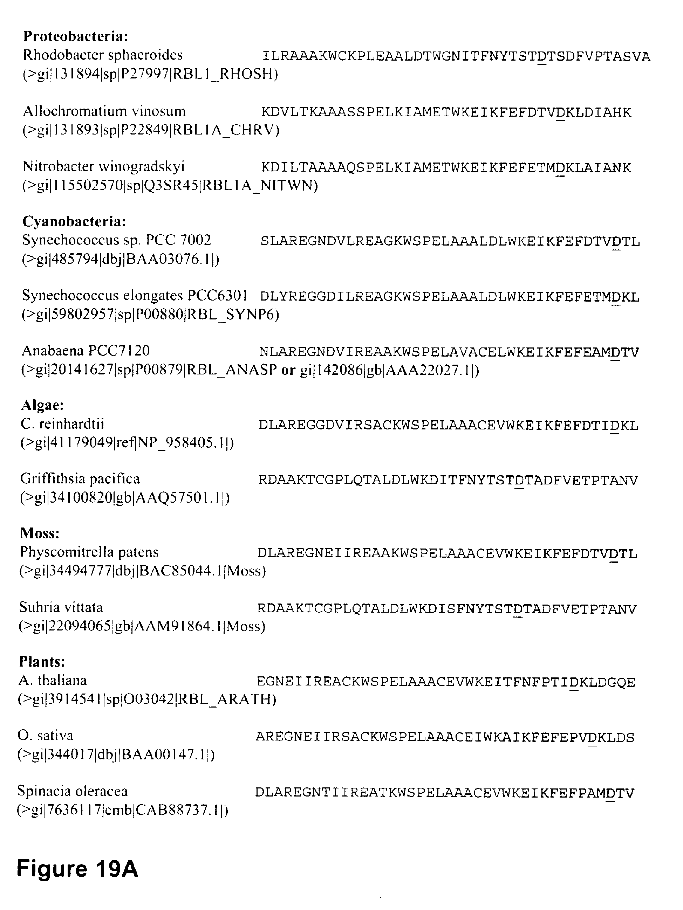

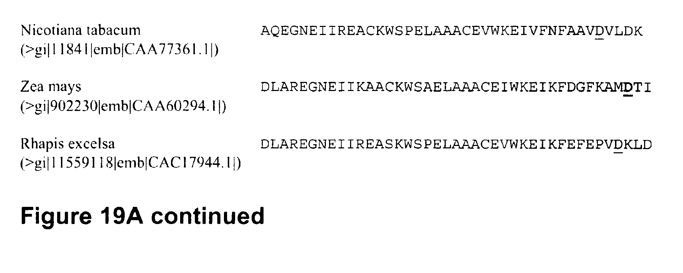

Furthermore, form I Rubiscos have been found to show some striking sequence similarity in the C-terminus of their RbcL subunits, in particular a conserved sequence of 12 (or in exceptional cases 13) amino acids wich has been characterized by the present inventors to be involved in the binding of the RbcX protein. Figure 19A shows some examples of C-termini of RbcL subunits from different sources (i.e. proteobacteria, cyanobacteria, green algae, red algae and higher plants) in which the conserved 12 (or 13) amino acid sequence is highlighted).

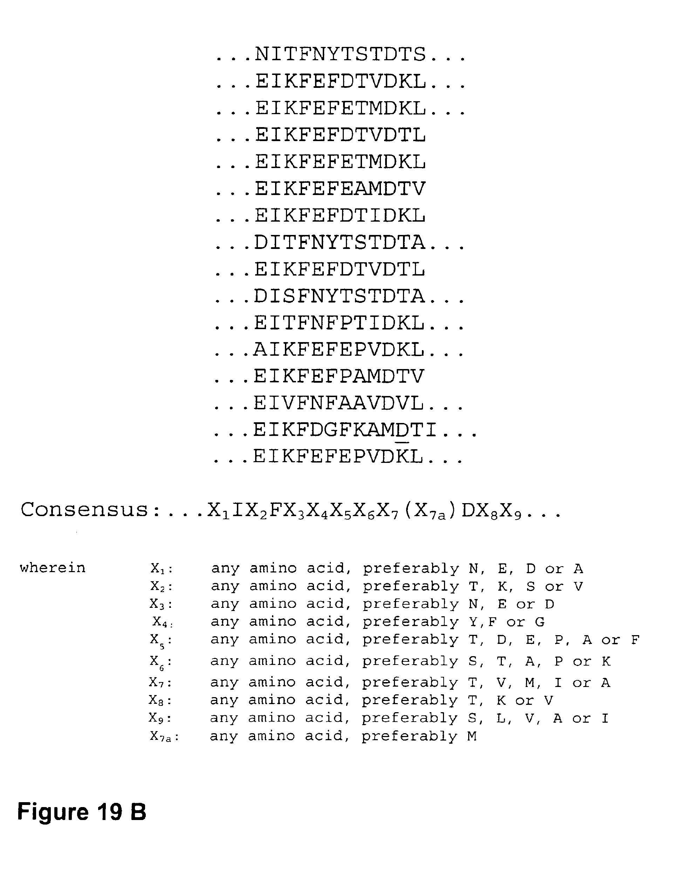

Thus, the term "form I" preferably refers to a subunit which contains in its C-terminal part, preferably within the last 30, more preferably within the last 25 amino acid residues, an amino acid sequence which resembles one of the highlighted 13 or 12 amino acid long regions shown in Figure 19B. More preferably the term "form I" refers to a subunit which contains in its C-terminal part a sequence which matches the consensus

X₁ IX₂FX₃X₄X₅X₆X₇DX₈X₉

or

X₁IX₂FX₃X₄X₅X₆X₇X₇ₐDX₈X₉

wherein X₁ can be any amino acid and can vary between species and is preferably N, E, D or A, X₂ can be any amino acid and can vary between species and is preferably T, K, S or V, X₃ can be any amino acid and can vary between species and is preferably N, E or D, X₄ can be any amino acid and can vary between species and is preferably Y, F or G, X₅ can be any amino acid and can vary between species and preferably is T, D, E, P, A or I, X₆ can be any amino acid and can vary between species and preferably is S, T, A, P or K, X₇ can be any amino acid and can vary between species and preferably is T, V, M, I or A, X₈ can be any amino acid and can vary between species and preferably is T, K or V, X₉ can be any amino acid and can vary between species and preferably is S, L, V, A or I, and X₇ₐ can be any amino acid and can vary between species and preferably is M.

According to the present invention the Rubisco to be used in the above described method is a plant Rubisco form I. Based on the already known sequences encoding form I Rubiscos, it is known that plant form I Rubiscos show a high degree of sequence identity, preferably of at least about ≥ 90%. Thus, based on the already known sequences and this high degree of conservation plant form I Rubiscos can be distinguished from form I Rubiscos from other sources.

The term "of plant origin" means that the form I Rubisco is from a plant organism. This encompasses, e.g. form I Rubisco molecules which can naturally be found in a plant organism, but also form I Rubisco molecules which are derived from such a naturally occurring Rubisco, e.g. by mutation, or modifications such as, e.g., deletions, additions, rearrangements, replacements or substitutions. Such derivatives cover in particular sequences which still share a high degree of sequence identity to a naturally occurring plant form I Rubisco, namely at least 70%, preferably at least 75%, more preferably at least 80%, even more preferably at least 90%, particularly preferred at least 91 % 92%, 93%, 94%, 95%, 96%, 97%, 98% and most preferred at least 99%. Thus, also plant form I Rubiscos are covered which have been modified, e.g. mutated, for example by classical and well-known methods for introducing directed or random mutations into sequences. Such methods are described, e.g., in Sambrook and Russell (2001), Molecular Cloning: A Laboratory Manual, CSH Press, Cold Spring Harbor, NY, USA) and include, for instance, deletions, point mutations, additions, substitutions, rearrangements, etc.

In the context of the present invention, the term "plant" means any eukaryotic organism which is classified as a eukaryotic plant organism according to the systematic classification of living organisms. According to this classification plants encompass slime fungi (Acrasiomycota, Myxomycota, Plasmodiophoromycota), fungi (Oomycota, Eumycota, Deuteromycetes), lichen (Lichenes), eukaryotic algae (Euglenophyta, Cryptophyta, Dinophyta, Haptophyta, Heterokontophyta (also called Chrysophyta), Rhodophyta (red algae), Chlorophyta (green algae)), moss (Bryophyta), fern (Pteridophyta) and higher plants. The term "higher plant" in the context of the present invention means a plant which belongs to the Spermatophyta. Spermatophyta encompass Gymnospermae (Coniferophytina and Cycadophytina) and Angiospermae (dicotyledonous or monocotyledonous plants). In a preferred embodiment the term "plant" refers to a plant organism which can be classified as an alga or as a higher plant. In an even more preferred embodiment a plant organism which can be classified as an alga is an alga belonging to the Rhodophyta (red algae). Preferred are red algae belonging to the taxonomic groups of Bangiophyceae, Cyanidiales and Cyanidiaceae. Alternatively, preferred are red algae belonging to the taxonomic groups of Florideophyceae, Ceramiales and Ceramiaceae and even more preferably an alga of the genus Griffithsia or Galdieria, most preferred of the species *Griffithsia monilis* or Galdieria sulfuraria.

The term "prokaryotic host cell" means any prokaryotic cell, preferably it refers to prokaryotic cells already known to be suitable for expressing heterologous proteins, in particular plant proteins. In a preferred embodiment the prokaryotic host cell is a bacterial cell. In general, any bacterial cell could be used, preferably bacterial cells are used in the method according to the invention which are already known to be suitable for expressing heterologous proteins, in particular plant proteins. Gram-positive as well as gram-negative bacteria can be used. In a preferred embodiment the host cell is a gram-negative bacterium. Examples are *Escherichia coli* or *Acinetobacter.* However, also gram-positive bacteria can be used, e.g. bacteria belonging to the *Steptomycetaceae,* such as *Streptomyces.*

The RbcL and RbcS subunits of the form I Rubisco expressed in the method according to the invention can be any possible plant form 1 RbcL and RbcS subunits. DNA sequences encoding such RbcL or RbcS subunits have been extensively described in the literature. As already described above, a multitude (actually hundreds) of plant RbcL and RbcS sequences is available in the literature and in the data-bases since they are frequently determined and used for taxonomic purposes.

Generally, the RbcL and RbcS subunits expressed in the method according to the invention will be derived from the same plant species, i.e. they are homologous with respect to each other. However, it is also conceivable that the RbcL and RbcS subunits expressed in the method according to the invention originate from different plant species, i.e. are heterologous with respect to each other. If the RbcL and RbcS subunits are heterologous to each other, it is preferred that they are derived from plants which are taxonomically closely related. Closely related preferably means that the plants belong to the same class, order, family, genus or prefereably species.

In one preferred embodiment of the method according to the invention the RbcL and RbcS subunits are of algae origin and, even more preferably, of red-algae (non-green algae) origin, most preferably of the taxonomic groups mentioned herein above.

In another preferred embodiment the RbcL and RbcS subunits are from a higher plant. Preferred are RbcL or RbcS subunits of plants of agricultural interest, in particular plants suitable for a high production of biomass, e.g. grasses, trees, etc. Agriculturally interesting plants are in general plants cultivated by mankind for human needs, such as fruit and vegetable, cereals, tobacco, plants for producing natural fibers, such as cotton, plants produced as animal feedstuff, etc.

Apart from already known sequences encoding plant form I RbcL or RbcS, it is of course also possible to use in the method according to the invention newly identified plant RbcL or RbcS sequences or modified versions/derivatives of the newly identified or known sequences. New RbcL/RbcS sequences can be identified by methods known to the person skilled in the art, e.g. heterologous screening of cDNA or genomic/plastomic DNA libraries by using as a probe known RbcL or RbcS sequences, PCR using as primers sequences covering conserved regions of the RbcL or RbcS sequences, etc., carrying out homology searches in data bases containing plant DNA or protein sequences. Modified versions or derivatives of the RbcL or RbcS sequences can be provided by methods known to the person skilled in the art such as directed or random mutation, truncation, insertions, deletions, additions, substitutions, etc. or by processes involving directed evolution.

The term "expressing" means that the nucleic acid sequences encoding the RbcL and RbcS subunit, respectively, are transcribed and translated into protein in the prokaryotic host cell. It is well-known to the person skilled in the art what measures to take to obtain transcription and translation of a heterologous protein in a given prokaryotic host cell. In particular, generally the nucleic acid sequence to be expressed is equipped with regulatory elements ensuring transcription and translation in the given host, i.e. elements which are recognized by the transcription and translation machinery of the chosen host. Such elements may include, e.g. promoters, terminators, enhancers, targeting signals, ribosome binding sites, etc. and are known in the art.
An overview of different expression systems is for instance contained in Methods in Enzymology 153 (1987), 385-516, in Bitter et al. (Methods in Enzymology 153 (1987), 516-544) and in Sawers et al. (Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440). Expression vectors have been widely described in the literature. Normally, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E. coli)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-ß-D-thiogalactopyranoside) (deBoer et al., Proc. Natl. Acad. Sci. USA 80 (1983), 21-25). Termination signals for transcription are also described in the literature.
The transformation of host cells with nucleic acid molecules can be carried out by standard methods, as for instance described in Sambrook and Russell (2001), Molecular Cloning: A Laboratory Manual, CSH Press, Cold Spring Harbor, NY, USA. The host cell is cultured in nutrient media meeting the requirements of the particular host cell used, in particular in respect of the pH value, temperature, salt concentration, aeration, antibiotics, vitamins, trace elements etc. The expressed polypeptide can be recovered and purified from recombinant cell cultures by methods including whole protein extraction, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Finally, high performance liquid chromatography (HPLC) can be employed for final purification steps.

The term "RbcX" protein refers in a first embodiment to a protein with the following characteristics:

In a first embodiment an RbcX protein generally has a length of between about 120 amino acids and about 220 amino acids, generally of about 130 to 210 amino acids. Cyanobacterial RbcX proteins have a length of about between 120 and 160 amino acids, preferably of about 120 to about 150 amino acids, most preferably of about 130 to 140 amino acids. Plant RbcX proteins are generally slightly longer than cyanobacterial RbcX proteins because they may contain a transit peptide. Namely, plant RbcX proteins normally have a length of about more than 100 to about less than 250 amino acids, preferably a length between 160 and 210 amino acids, even more preferably a length between 170 and 205 amino acids. Their molecular weight is between about 12 kDa and 18 kDa, preferably between 14 kDa and 17 kDa, more preferably between about 15 and 16 kDa. RbcX proteins have, for example, already been described for cyanobacteria, such as Microcystis, Planktothrix, Tychonema, Nostoc, Synechococcus, e.g. Synechococcus sp. PCC7002, Synechococcus sp. PCC7942, Synechoccus sp. PCC6301, and for Anabaena, such as Anabaena sp. CA. DNA sequences encoding the RbcX proteins as well as the corresponding amino acid sequences are publicly available and corresponding data base accession numbers are e. g., disclosed in Rudi et al. (J. Bacteriol. 180 (1998), 3453-3461). Although RbcX sequences show a considerable degree of variability, RbcX proteins show some structural characteristics namely the occurrence of characteristic amino acid residues in a characteristic spacing.

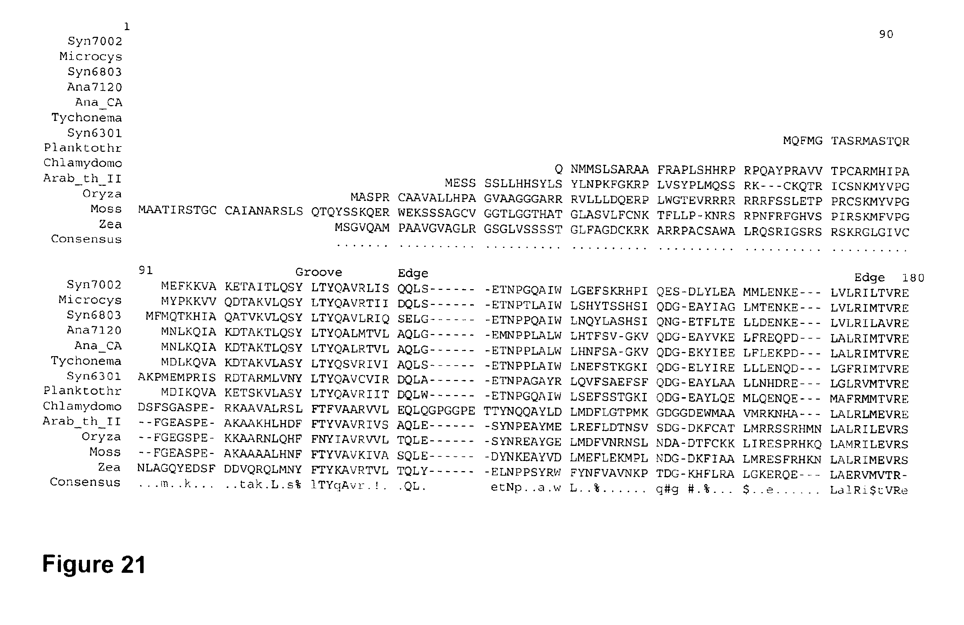

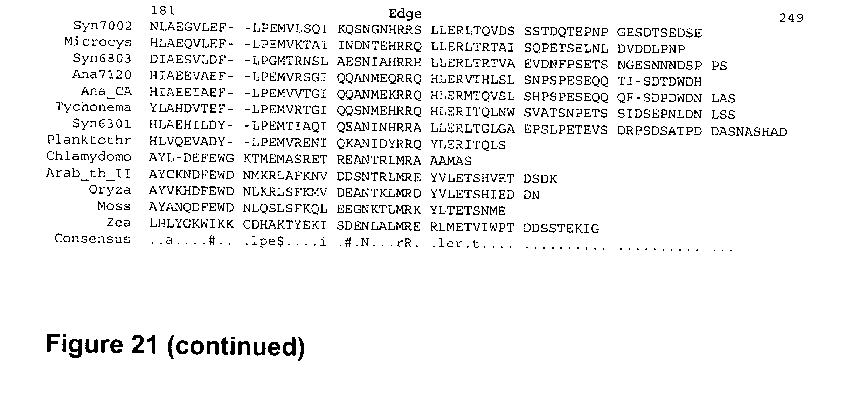

Namely, the present inventors found that some areas of the RbcX protein are particularly relevant for RbcX function and are also rather highly conserved among RbcX proteins from different species. A comparison between the RbcX sequences from different sources is shown in Figure 21. From this comparison it is evident that four areas are conserved among RbcX proteins, namely an area of about 4 amino acids corresponding to amino acids 17 to 20 of the RbcX protein of Synechococcus sp. PCC7002 (in the following Syn7002), an area of about 6 amino acids corresponding to amino acids 70 to 75 of the RbcX protein of Syn7002 and an area of about two amino acids corresponding to amino acids 102 and 103 of the RbcX protein of Syn 7002.

In a preferred embodiment of the first embodiment an RbcX protein is characterized as showing at least one of the followng amino acid residues:
1) a T at the position coresponding to position 19 of the RbcX protein of Syn7002;
2) a Y at the position corresponding to position 20 of the RbcX protein of Syn 7002;
3) a Q at the position corresponding to position 29 of the RbcX protein of Syn7002;
4) an L at the position corresponding to position 30 of the RbcX potein of Syn7002;
5) an R at the position corresponding to position 70 of the RbcX protein of Syn 7002;
6) an R at the position corresponding to position 75 of the RbcX protein of Syn 7002;
7) an R at the position coresponding to position 103 of the RbcX protein of Syn 7002.

More preferably, in the first embodiment an RbcX protein is characterized in that it contains at least two, at least three, at least four, at least five, at least six and most preferred all seven of the above mentioned amino acid residues. In a particuarly preferred embodiment, an RbcX protein is characterized in that it contains at least the amino acid residues mentioned above under (2), (4), (5), (6) and (7).
As is evident from Figure 21, there are some further conserved residues, namely at the position coresponding to amino acid positions 14, 34 and 98 of the RbcX protein of Syn7002. Thus, in another preferred embodiment, an RbcX protein also contains an L at the position corresponding to amino acid residue 14 of the RbcX protein of Syn7002, or an N at the position corresponding to amino acid 34 of the RbcX protein of Syn7002 or an N at the position corresponding to position 98 of the RbcX protein of Syn7002 or any possible combination of these residues.
The term "corresponding to position X of the RbcX protein of Syn7002" refers to an amino acid position which would correspond to said position X of the RbcX protein of Syn7002 when the respective amino acid sequence is aligned with the RbcX protein sequence of Syn7002, preferably in a manner which leads to the best match. Corresponding methods are well-known to the person skilled in the art and include, e.g., a multiple sequence alignment with hierarchical clustering which can be performed with MultAlin (Corpet (Nucl. Acids. Res. 16 (1988), 10881-10890); http://bioinfo.genopole-toulouse.prd.fr/multalin.htlm).
Preferably, in the context of the present invention a protein is understood to be a RbcX protein if it shows an amino acid sequence of at least 25%, more preferably at least 30%, even more preferably at least 40%, particularly preferred at least 60% sequence identity to a known cyanobacterial RbcX protein sequence. In a preferred embodiment the known cyanobacterial RbcX amino acid sequence is of Synechococcus, preferably of Synechococcus sp. PCC7002, sp. PCC7942, sp.

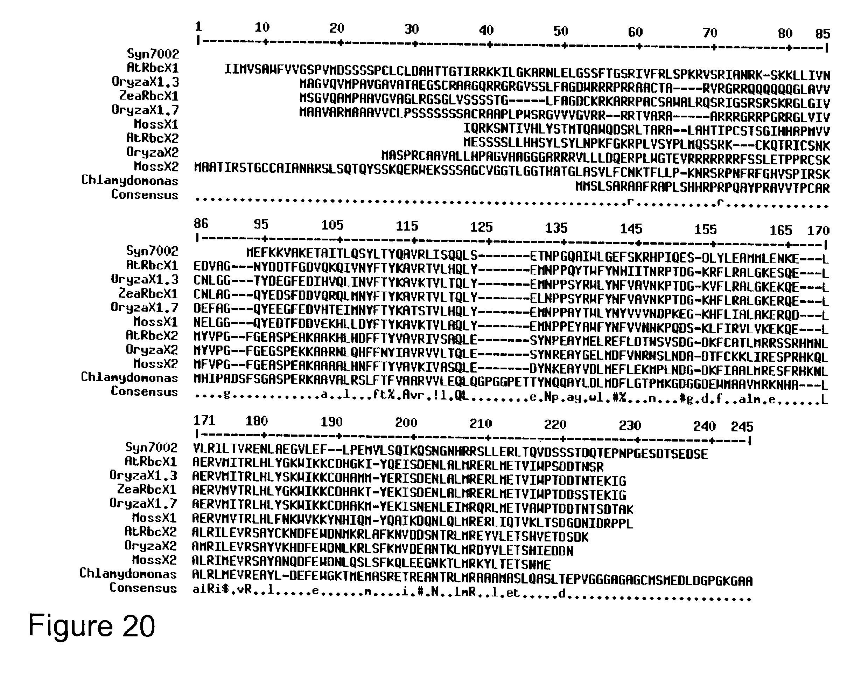

PCC6301 or from Anabaena, preferably Anabaena sp. CA and most preferably of Synechococcus sp. PCC7002 (see, e.g., Figures 20 and 21).

The term "plant RbcX" protein refers to a RbcX protein which originates from a plant organism. Generally, a plant RbcX protein has an amino acid sequence which shows about 25% to 40% sequence identity to a cyanobacterial RbcX amino acid sequence, preferably to the RbcX amino acid sequence of Synechococcus sp. PCC7002.

Plant RbcX proteins are preferably characterized by a length of between 160 and 210 amino acids, more preferably of between about 170 and 205 amino acids. Even more preferably, a plant RbcX protein is characterized by the fact that it is encoded by the nuclear genome and that it contains a transit peptide when naturally expressed in the plant cell.
Examples for some plant RbcX proteins are provided in Figure 20. Namely, shown are the RbcX amino acid sequences of Chlamydomonas reinhardtii, Physcomitrella patens (moss), Arabidopsis thaliana, Zea mays and Oryza sativa. The corresponding nucleotide sequences are shown in SEQ ID NOs: 1, 3, 5, 7, 9, 11, 13, 15 and 17. A plant RbcX protein preferably shows a sequence identity to another plant RbcX protein of about at least 40%, preferably of about at least 50%, even more preferably of at least about 60%. Preferably, the plant RbcX protein sequence used as a reference sequence in this regard is that of A. thaliana shown in SEQ ID NOs: 2 or 4. DNA sequences encoding plant RbcX proteins can be identified by the skilled person by methods well-known in the art. It is, e.g, possible to use the herein described plant RbcX sequences of *Chlamydomonas reinhardtii, Physcomitrella patens, Arabidopsis thaliana, Zea mays* or *Oryza sativa* in computer assisted methods in order to search in databases for related sequences. For this purpose, any suitable algorithm can be used. Examples are the NCBI Blast programs, e.g. "blastp" for protein searches and "blastn" for nucleotide sequence searches. Sequences which show on the nucleotide level a sequence identity of more than 40% to the plant RbcX sequence used as reference sequence are selected as possible candidates. These sequences are then further analyzed for structural characteristics, e.g. the length, the occurrence of a transit peptide and the above described characteristic amino acid residues and their spacing.

Furthermore, plant RbcX sequences can also be identified and isolated by well-known techniques for cloning genes. One possibility is the use of the plant RbcX nucleotide sequences as disclosed herein for screening approaches, e.g. heterologous screening of genomic, plastomic or cDNA libraries of other plant species. Another possibility is the design of degenerate oligonucleotide probes based on conserved sequence regions. A further possibility is the isolation of the respective RbcX protein in order to obtain partial amino acid sequence data. The isolation of the RbcX protein can, e.g., be achieved by pulling it down with the help of a RbcL subunit or a fusion protein containing the C-terminus which can, e.g., be obtained by recombinant expression. Since the C-terminus of RbcL binds RbcX, RbcX proteins can be isolated in this manner. Amino acid sequence data can then be obtained from the isolated RbcX protein and these can be used to clone the corresponding nucleotide sequence by approaches well-known to the person skilled in the art.

In a second embodiment the term "RbcX" protein also refers to proteins which have been found to occur in red algae and which are characterized by the following features:

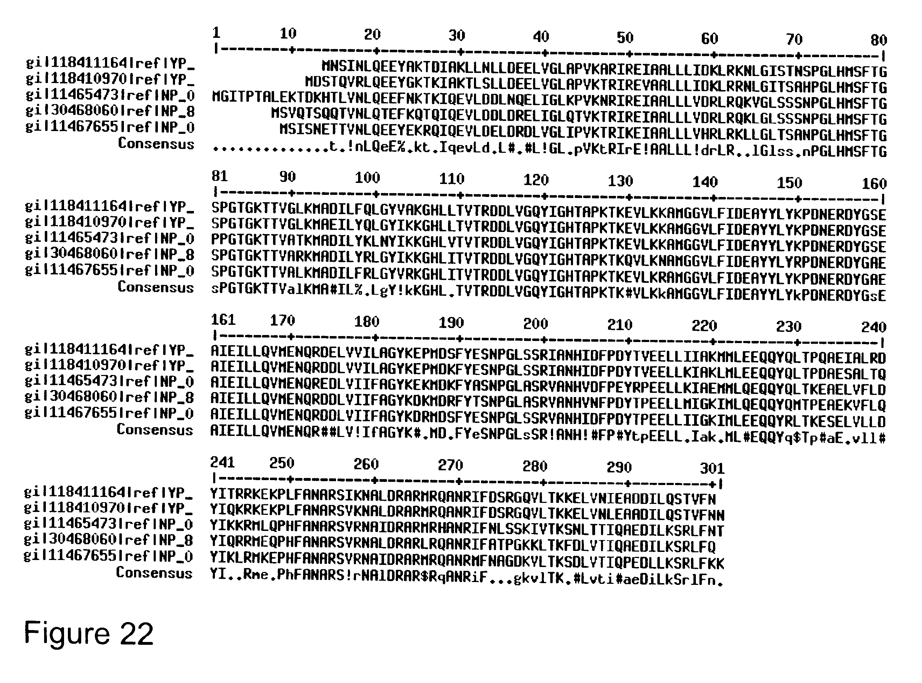

They are encoded by an open reading frame which is located in the same operon as the red algae RbcL and RbcS subunit and the open reading frame generally is located downstream of the open reading frame encoding RbcS. The encoded protein has a molecular weight of about between 25 kDa and 35 kDa, preferably of about 30 kDa. Preferably, the protein is characterized by having an ATP-binding site. In a preferred embodiment such an RbcX protein is of red algae origin. Examples of corresponding RbcX proteins are shown in Figure 22. The shown amino acid sequences are available at the NCBI database under accession numbers 118411164, 118410970, 11465473, 30468060 and 11467655, respectively. As is evident from Figure 22, these RbcX proteins show a considerable degree of sequence identity among each other. Thus, in another preferred embodiment, a protein is understood to be a RbcX protein if it shows at least 50% sequence identity, preferably at least 60%, even more preferably at least 70% and particularly preferred at least 80% sequence identity to an amino acid sequence shown in Figure 22, preferably to the sequence in the 1^{st} line (accession number 118411164).

The above-described expression method for an assembled plant Rubisco protein now allows to establish a screening platform in order to select Rubisco proteins with improved properties.

Accordingly, the present invention also relates to a method for selecting a form I Rubisco of plant origin for desired properties comprising the steps of:
(a) subjecting a nucleic acid sequence encoding the large subunit of a plant form I Rubisco (RbcL) and/or a nucleic acid sequence encoding the small subunit of a plant form I Rubisco (RbcS) and/or a nucleic acid sequence encoding a plant RbcX protein to a process which introduces (a) mutation(s) into the nucleic acid sequence;
(b) expressing in a prokaryotic host cell a nucleic acid sequence encoding a plant form I RbcL, a nucleic acid sequence encoding a plant form I RbcS and a nucleic acid sequence encoding a plant RbcX protein, wherein at least one of said sequences has been subjected to a process of step (a) which introduces (a) mutation(s) and wherein the RbcX protein is derived from the same plant species from which the RbcL subunit is derived;
(c) analyzing the properties of the Rubisco protein obtained according to step (b); and
(d) selecting a nucleic acid sequence encoding a Rubisco protein with the desired properties.

The term "method for selecting" means that the method aims at screening at least one, but preferably more, Rubisco molecules for a desired property and selecting among the screened molecules a molecule which shows the desired property or an improvement in said property.
A process which introduces one or more mutations into a nucleic acid sequence encoding RbcL, RbcS or RbcX can be any known process suitable to introduce such mutations. Included are methods for introducing directed mutations, such as site-directed mutagenesis, controlled deletions or truncations etc., as well as methods introducing random mutations. Such mutations include all possible sequence modifications, e.g. deletions, additions, substitutions, inversions, rearrangements, replacement of certain domains or regions from RbcL, RbcS or RbcX molecules, e.g. by corresponding domains of RbcL, RbcS or RbcX molecules from other species, etc.

It is possible to mutate only one of the three proteins and to assess the effect of the mutation on the desired property. However, it is also possible to mutate the sequences coding for two or even all three proteins and to assess whether the desired property is achieved and/or improved.
Since it has been found that obviously RbcL and RbcX proteins have co-evolved during evolution, it is preferred to mutate both, the sequences encoding RbcL and the sequences encoding RbcX. However, it is also possible to only mutate the sequences encoding RbcL or only those encoding RbcX or only those encoding RbcS. Similarly, it is possible to mutate the sequences encoding RbcL and the sequences encoding RbcS or the sequences encoding RbcS and the sequences encoding RbcX.

The expression of the nucleotides sequences according to step (b) of the method may be achieved according to methods well-known in the art and as described herein-above.

The analyzing in step (c) of the method depends on exactly which property of the Rubisco molecule is of interest and can, accordingly, be carried out by methods known to the person skilled in the art. One property of interest of Rubisco is its property to fixate CO₂. In particular, in principle an enzyme perfectly adapted for speed and specificity would convert its substrate as fast as it diffused into its active site and never mistake nonsubstrate for substrate. A "perfect" Rubisco would have:
(1) a k^{c}_{cat}/K_{c} quotient (k^{c}_{cat} = CO₂ saturated carboxylase activity; K_{c}=Michaelis constant for CO₂) of at least 10⁸M⁻¹s⁻¹,
(2) a K_{c} higher than the CO₂ concentration in the chloroplast stroma (commonly approximately 8 µM, which is a little less than that of air-equilibrated water due to diffusional barriers) if both k^{c}_{cat}/K_{c} and carboxylation rate have been maximized during evolution, and
(3) an infinitive specificity for CO₂ relative to O₂ (S_{c/o}).

Naturally occurring Rubiscos fulfill criterion 2 but fail the other two miserably which affects the efficiency of photosynthetic CO₂ assimilation negatively (Andrews and Whitney (Arch. Biochem. Biophys. 414 (2003), 159-169). Thus, properties of interest of Rubiscos are, e.g., its k^{c}_{cat} value, the k^{c}_{cat}/K_{c} quotient and its specificity for CO₂.

Methods for determining these properties are well-known to the person skilled in the art, are described in the appended Examples and are extensively described in the literature. A further possible property to select for is the thermal stability of the Rubisco, in particular a higher thermal stability. In one aspect this may mean that the Rubisco is more stable at higher temperature. However, in another aspect, it may be of interest to select for Rubiscos which have a broader temperature range in which they are stable.
Another possible property of interest of Rubisco is its efficient and correct assembly into the L₈S₈ hexadecameric complex. Methods to assess whether the expressed RbcL and RbcS subunits properly assemble into the L₈S₈ complex are known to the person skilled in the art and are described in the literature. One approach to assess whether the subunits correctly assemble is, e.g., whether they occur in the insoluble or in the soluble protein fraction after harvesting the host cells. Not correctly assembled subunits aggregate and occur in the insoluble protein fraction. Correctly assembled subunits are present in the soluble fraction of cell proteins. The presence of the subunits in the different fractions can, e.g., be detected by immunological methods such as Western-Blot or immunoprecipitation. Whether the subunits are assembled can be verified, e.g., by native PAGE followed, e.g., by immunoblotting with subunit specific antibodies.

The step of selecting a nucleic acid sequence which encodes a Rubisco protein with the desired property (step (d)) generally involves an evaluation of the results obtained in the analyzing step (c) in comparison with an already achieved value for the desired property or in comparison to a value which should at least be achieved. "Selecting" may then mean that a nucleotide sequence is chosen for further use if it encodes a Rubisco showing a value for the property of interest which is higher than the already previously achieved value or showing the value which comes closest to the value which should be achieved. Such a selected sequence may then either be further used, e.g., for introduction into plants (see further below) or it may also be used again as a starting material for the method for selecting a Rubisco according to the present invention in order to possibly achieve a further improvement of the property of interest.

In a preferred embodiment of the above described methods according to the invention (i.e. method for expressing a form I Rubisco and method for selecting a form I Rubisco, respectively) the host cell also (over)expresses chaperonin molecules which are known to support the proper folding of proteins in the prokaryotic host cell. It has been shown that in *E*. *coli* the overexpression of the chaperonin 60 (GroEL) heat-shock protein (generally together with the chaperonin 10 (or chaperonin 20) GroES) facilitates assembly of cyanobacterial Rubisco in *E. coli.* Thus, although the RbcX protein expressed according to the methods of the invention in the host cell supports the assembly of the Rubisco molecule, a further enhancement may be achieved by the simultaneous overexpression of prokaryotic chaperonins, preferably those naturally expressed in the chosen host cell. Thus, in a preferred embodiment, the methods also include the (over)expression of nucleotide sequences encoding a chaperonin of prokaryotic origin. In one preferred embodiment said chaperonin is homologous to the chosen host cell. In another preferred embodiment the chaperonin is the E. *coli* chaperonin GroEL/GroES. The overexpression of this chaperonin in prokaryotic host cells has been described in the literature (see, e.g., Larimer and Soper (Gene 126 (1993), 85-92)) and is also described in the appended Examples.

The present invention furthermore relates to a method for selecting a form I Rubisco with desired properties comprising the steps of:
(a) subjecting a nucleic acid sequence encoding the large subunit of a form I Rubisco (RbcL) to a process by which (a) mutation(s) is/are introduced into the C-terminal sequence of the RbcL subunit;
(b) expressing the mutated sequence obtained in step (a) in a host cell simultaneously with a nucleic acid sequence encoding a small subunit of a form I Rubisco (RbcS) and together with a nucleic acid sequence encoding an RcbX protein which is derived from the same species as said RbcL;
(c) analyzing the properties of the Rubisco protein obtained according to step (b); and
(d) selecting a nucleic acid sequence encoding a Rubisco protein with the desired properties.

The present inventors found out that the RbcX directly interacts with the RbcL subunit via the C-terminus of the RbcL subunit and promotes assembly of the RbcL subunit into the L₈ core complex of the Rubisco. Although there have been some reports in the prior art that the RbcX protein of cyanobacteria may have a chaperon-like function in E. coli, recent reports cast doubts on this and suspect that the effects observed when expressing RbcX of cyanobacteria in E. *coli* may be due to an indirect stimulation of the activity of one or more components of the E. *coli* chaperon network. Namely, it is suspected that RbcX may interact with the 5' regulatory sequences of the rbc mRNA (encoding RbcL and RbcS) to facilitate its translational processing (see Emlyn-Jones et al. (Plant Cell Physiol. 47 (2006), 1630-1640)).
However, the data provided in the appended Examples show that this conclusion drawn in the literature is wrong and that the RbcX protein directly interacts with the RbcL subunit. Namely, the RbcX protein forms a homodimer with two RbcL binding regions that functionally cooperate. A central peptide binding cleft recognizes specifically an exposed C-terminal peptide of unassembled RbcL, shielding its hydrophobic side chains. This interaction promotes the formation of RbcL₈ core complexes. RbcS can then displace RbcX once RbcL₈ core complexes have formed.

The C-terminal sequence of RbcL is known to be involved in the enzymatic activity of the Rubisco (Duff et al. (J. Mol. Biol. 298 (2000), 903-916)). Thus, modifications aiming at increasing the enzymatic activity, i.e. CO₂ assimilation, may be placed in this region. However, as RbcX interacts also with this C-terminal region and thus this region is also required for efficient RbcL₈ assembly, any screening method effecting mutations in the C-terminal sequence may interfere with RbcX binding.
The above described screening method according to the present invention takes this interaction into consideration and requires the simultaneous expression of RbcX. By this it can be made sure that only mutations in the C-terminal sequence will be selected which do not interfere with the interaction of RbcL with RbcX.

With respect to the terms "selecting", "form I Rubisco", "large subunit", "small subunit", "RbcX protein", "property", "process introducing mutations", "expressing", "analyzing" and "selecting" the same applies as has already been set forth above in connection with the other methods according to the invention.
The term "host cell" as used in the context of this method means any suitable host cell which allows expression of the RbcL, RbcS and RbcX sequences. It may be a eukaryotic or prokaryotic cell, e.g. a plant, animal, fungal or bacterial cell. Preferably, the host cell is a fungal or bacterial cell, more preferably it is a bacterial cell and most preferably *Escherichia coli.*
The form I Rubisco and the RbcL, RbcS and RbcX subunits can be of any possible origin, i.e. plant, cyanobacterial or proteobacterial origin. Generally, the RbcL and RbcS subunits are homologous to each other, i.e. derived from the same species. However, they may also be heterologous to each other as already described above. The RbcX subunit is preferably homologous with respect to the RbcL subunit. Moreover, the RbcL and RbcX proteins are preferably of plant origin or proteobacterial origin, most preferably of plant origin.

The term "C-terminal sequence" relates to the C-terminal part of RbcL, in particular to the C-terminal 40 amino acids, more preferably to the C-terminal 30 amino acids or even more preferably to the C-terminal 25 amino acids. The present inventors have found that the form I RbcL subunits contain in their C-terminal part a conserved sequence motif which has been shown to be involved in the interaction with RbcX. This sequence motif is about 12 amino acids (in exceptional cases 13 amino acids) in length and shows some degree of conservation between all possible sources of form I RbcL subunit, i.e. from proteobacteria, cyanobacteria, red algae, green algae and higher plants, as is evident from Figure 19. In a preferred embodiment the 12 (or 13) amino acid sequence motif matches the consensus sequence as indicated in Figure 19B.
In a preferred embodiment a mutation is introduced into the above-described conserved 12 (or 13) amino acid sequence motif of the C-terminus of the RbcL subunit.

In another preferred embodiment of the above-described screening method according to the invention the nucleic acid sequence encoding an RbcX protein mentioned in step (b) has also been subjected to a process for introducing a mutation(s) before expressing it together with RbcL and RbcS in the host cell. This allows to "co-evolve" RbcL and RbcX in the screening method. Namely, mutations effected in the C-terminal sequence of RbcL which may lead to improved enzymatic properties but which interfere with the interaction with RbcX thereby hampering assembly of RbcL₈ may be compensated by simultaneous mutations in RbcX restoring interaction with RbcL.

In another preferred embodiment also the sequence encoding RbcS mentioned in step (b) has been subjected to a process for introducing (a) mutation(s) before expressing it together with RbcL and RbcX in the host cell.

In a further preferred embodiment the mutation effected according to step (a) in the C-terminal sequence of RbcL is a replacement of the C-terminal sequence by a C-terminal sequence of a RbcL subunit of a different organism, genus or species. In this case, the RbcX protein expressed in step (b) is preferably homologous to the RbcL subunit of which the new C-terminal sequence is derived.

The present invention furthermore relates to a method for producing a modified Rubisco RbcL subunit characterized in that the C-terminal sequence of a RbcL subunit of one species is modified so as to have the C-terminal sequence of a RbcL subunit of a different species thereby resulting in a chimeric RbcL subunit in which the C-terminal sequence is heterologous to the remainder of the molecule.
As has been described above, it has been found by the present inventors that RbcX directly interacts with the C-terminal sequence of RbcL and is necessary for the assembly of the RbcL₈ core complex. Thus, in order to obtain effective assembly of a Rubisco in a given host cell, such as for example a transgenic plant, it is necessary to make sure that together with the RbcL subunit a "matching" RbcX protein is expressed. Generally, this "matching" RbcX protein will be the RbcX protein naturally expressed in the same species, i.e. a homologous RbcX protein. However, the findings of the present invention now also allow to produce RbcL subunits known to match a given RbcX protein. Namely, since it has been found that the RbcX protein interacts with the C-terminal sequence of RbcL, it is now possible to transfer the C-terminal sequence of a given RbcL subunit to a RbcL subunit of a different origin, e.g. an organism of a completely different type or of a different genus or species. This new chimeric RbcL subunit will then interact with the RbcX protein of the organism from which the C-terminus originates. Thus, by this approach it is now possible to express successfully a given RbcL in a heterologous environment even if the matching RbcX protein may not be available. Thus, it is now, for example, conceivable to replace in a RbcL subunit derived from red-algae the C-terminal sequence by that of the RbcL of a higher plant, e.g., tobacco. This chimeric red-algae/tobacco RbcL could then be expressed in tobacco. Since the tobacco plant would provide the RbcX protein recognizing the tobacco C-terminal sequence in the chimeric red-algae/tobacco RbcL subunit, such a subunit can efficiently be assembled in tobacco into RbcL₈ core complexes. The RbcL subunit in which the C-terminal sequence is replaced and the RbcL subunit from which the new RbcL sequence is taken can be of any possible origin. In a preferred embodiment the C-terminal sequence used to be transferred to a RbcL subunit is of a form I RbcL subunit. In one embodiment the RbcL subunit in which the C-terminal sequence is replaced and the RbcL subunit from which the new C-terminal sequence is taken are both RbcL subunits of form I Rubisco molecules.
In an alternative embodiment the RbcL subunit in which the C-terminal sequence is modified is a RbcL subunit of a form II Rubisco. As explained above, form II Rubisco is a simpler form found in some photosynthetic bacteria (e.g. *Rhodospririllum rubrum,* purple non-sulfur bacteria) and dinoflagellates which is a dimer of RbcL subunits. According to the present invention it is now possible to equip a form II RbcL subunit with a C-terminal sequence of a form I RbcL subunit. This will allow the form II RbcL subunit thus modified to interact with the RbcX protein which interacts with the form I RbcL C-terminal sequence transferred to the form II RbcL subunit. The thus modified form II RbcL subunit may acquire improved properties when expressed in the presence of the corresponding RbcX protein recognizing the transferred RbcL C-terminal sequence. In particular, such engineered versions of form II Rubisco may form active L₈ complexes in a RbcX-dependent manner which can be analyzed for interesting properties.
In general, any form II RbcL can be used in this context. Sequences encoding form II Rubisco are described in the literature and are available in databases.

The present invention also relates to a modified form I RbcL subunit resulting from the replacement of its C-terminal sequence by a C-terminal sequence of a form I RbcL from a different species. Such a modified form I RbcL is a chimeric RbcL which comprises a C-terminal sequence of a form I RbcL from one species and in which the rest of the molecule is heterologous with respect to this C-terminal sequence, i.e. it is derived from a RbcL subunit which is derived from a different species than that from which the C-terminal sequence stems. In a preferred embodiment the C-terminal sequence is derived from a RbcL of a higher plant and the remainder of the molecule is derived from a RbcL subunit of algae, preferably red algae (non-green algae).

In a further aspect the present invention relates to a modified form II RbcL resulting from the modification of its C-terminus so as to have a C-terminal sequence of a form I RbcL. Such a modified form II RbcL is a chimeric RbcL which comprises a C-terminal sequence of a form I RbcL and in which the remainder of the molecule is derived from a form II RbcL.

As described above, the present inventors have found that Rbx proteins directly interact with RbcL and are involved in the correct assembly of the L₈ core complex.
Moreover, the experimental results show that heterologous pairs of RbcL and RbcX are most efficient, that heterologous pairs of RbcL and RbcX are not efficient and that the use of a heterologous RbcX protein may even be inhibitory. Thus, the present invention provides important information for successfully expressing an active heterologous Rubisco in plant cells which has so far been hampered by the inability to obtain properly assembled L₈S₈ complexes. Namely, the present invention teaches to use for expression of a heterologous RbcL in a plant cell a RbcX protein which is homologous with respect to the RbcL subunit, i.e. is derived from the same species. Moreover, it may be advantageous to suppress the expression of a RbcX protein endogenously occurring in the plant cell to be transformed in order to suppress its possible inhibitory effect on the formation of the heterologous L₈ complex.
Therefore, the present invention in another aspect also relates to a method for producing a transgenic plant cell comprising the step of (i) transforming a plant cell with a nucleic acid molecule encoding a form I RbcL subunit and with a nucleic acid molecule encoding a RbcX protein, wherein the RbcL subunit and the RbcX protein are derived from the same species.

The term "plant cell" relates to any possible plant cell. In this context, the term "plant" is used as defined herein-above. The term "transgenic" means that the plant cell is genetically modified so as to comprise a nucleic acid molecule which naturally does not occur in said plant cell. This may mean that the nucleic acid molecule is heterologous with respect to the plant cell, i.e. the whole or parts of the nucleic acid molecule do not naturally occur in said plant cell, or it may mean that the nucleic acid molecule occurs in the transgenic plant in a genomic context in which it naturally does not occur in a corresponding wild-type cell. Preferably, the term "transgenic" means that the nucleic acid molecule is stably integrated into the genome of said plant cell, most preferably into the plastome, i.e. into the genetic information contained in the plastids. In an alternative embodiment the nucleic acid is stably integrated into the nuclear genome.
The terms "RbcL subunit" and "form I" are used in this context as defined herein-above. These terms cover any naturally occurring form I RbcL subunit as well as any possible variants or mutants thereof. Such variants and mutants also have been described herein-above in the context of the methods for expressing or screening a plant form I Rubisco in a (prokaryotic) host cell or of the methods for producing a modified Rubisco.
The form I RbcL subunit may be of any origin, e.g. cyanobacterial, proteobacterial or plant origin. In a preferred embodiment, the RbcL subunit is of plant origin and in an even more preferred embodiment of algae origin, most preferred redalgae *(Rhodophyta)* origin. Particularly preferred are red algae as mentioned above in connection with the methods according to the invention, most preferred red-algae of the genus *Griffithsia* or of the genus *Galdieria,* in particular *Griffithsia monilis* or *Galdieria sulfuraria.*
The term "RbcX protein" covers any possible RbcX protein, e.g. a RbcX protein of plant or cyanobacterial origin. The term "plant RbcX" refers to RbcX proteins as defined herein above in connection with the methods according to the invention. RbcX proteins from cyanobacteria have already been described in the literature and include those mentioned hereinabove.

According to the method according to the invention the RbcX protein is derived from the same species as the RbcL subunit. The phrase "the RbcL subunit and the RbcX protein are derived from the same species" means that at least the C-terminal part of the RbcL subunit and the RbcX protein are derived from the same species. Thus, it also covers the embodiments in which the C-terminal part of the RbcL subunit stems from the same species as the RbcX protein while the remainder of the RbcL subunit may be derived from a different species. In a preferred embodiment the RbcL subunit and the RbcX protein stem from the same species and are naturally capable of interacting with each other. In another preferred embodiment, the RbcX protein is derived from a red-alga (Rhodophyte) and the RbcL subunit contains at least the C-terminal part of the RbcL subunit derived from said red alga. More preferably, the RbcL subunit is the complete RbcL subunit derived from said red alga.

Methods for transforming plant cells with nucleic acid molecules are widely described in the literature. These include, for example, the transformation of plant cells or tissues with T-DNA using *Agrobacterium tumefaciens* or *Agrobacterium rhizogenes,* the fusion of protoplasts, direct gene transfer (see, e.g., EP-A 164 575), injection, electroporation, vacuum infiltration, biolistic methods like particle bombardment, pollen-mediated transformation, plant RNA virus-mediated transformation, liposome-mediated transformation, transformation using wounded or enzyme-degraded immature embryos, or wounded or enzyme-degraded embryogenic callus and other methods known in the art. The vectors used in the method of the invention may contain further functional elements, for example "left border"- and "right border"-sequences of the T-DNA of *Agrobacterium* which allow stable integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example co-transformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., WO97/08331; Bayley, Plant Mol. Biol. 18 (1992), 353-361); Lloyd, Mol. Gen. Genet. 242 (1994), 653-657; Maeser, Mol. Gen. Genet. 230 (1991), 170-176; Onouchi, Nucl. Acids Res. 19 (1991), 6373-6378). Methods for the preparation of appropriate vectors are described by, e.g., Sambrook and Russell (2001), Molecular Cloning: A Laboratory Manual, CSH Press, Cold Spring Harbor, NY, USA. Suitable strains of *Agrobacterium tumefaciens* and vectors as well as transformation of *Agrobacteria* and appropriate growth and selection media are well known to those skilled in the art and are described in the prior art (GV3101 (pMK90RK), Koncz, Mol. Gen. Genet. 204 (1986), 383-396; C58C1 (pGV 3850kan), Deblaere, Nucl. Acid Res. 13 (1985), 4777; Bevan, Nucleic. Acid Res. 12(1984), 8711; Koncz, Proc. Natl. Acad. Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287). Although the use of *Agrobacterium tumefaciens* is preferred in the method of the invention, other *Agrobacterium* strains, such as *Agrobacterium rhizogenes,* may be used, for example if a phenotype conferred by said strain is desired.
Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer To Plants. Springer Verlag, Berlin, NY (1995).
The transformation of most dicotyledonous plants is possible with the methods described above. But also for the transformation of monocotyledonous plants several successful transformation techniques have been developed. These include the transformation using biolistic methods as, e.g., described above as well as protoplast transformation, electroporation of partially permeabilized cells, introduction of DNA using glass fibers, etc. Also, the transformation of monocotyledonous plants by means of Agrobacterium-based vectors has been described (Chan et al., Plant Mol. Biol. 22 (1993), 491-506; Hiei et al., Plant J. 6 (1994) 271-282; Deng et al, Science in China 33 (1990), 28-34; Wilmink et al, Plant Cell Reports 11 (1992), 76-80; May et al., Bio/Technology 13 (1995), 486-492; Conner and Dormisse, Int. J. Plant Sci. 153 (1992), 550-555; Ritchie et al. Transgenic Res. 2 (1993), 252-265). An alternative system for transforming monocotyledonous plants is the transformation by the biolistic approach (Wan and Lemaux, Plant Physiol. 104 (1994), 37-48; Vasil et al., Bio/Technology 11 (1993), 1553-1558; Ritala et al., Plant Mol. Biol. 24 (1994) 317-325; Spencer et al., Theor. Appl. Genet. 79 (1990), 625-631). The transformation of maize in particular has been repeatedly described in the literature (see for instance WO 95/06128, EP 0 513 849, EP 0 465 875, EP 29 24 35; Fromm et al, Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726). The successful transformation of other types of cereals has also been described for instance of barley (Wan and Lemaux, supra; Ritala et al., supra, Krens et al., Nature 296 (1982), 72-74), wheat (Nehra et al., Plant J. 5 (1994), 285-297) and rice. Moreover, methods for plastid transformation are well-known to the person skilled in the art and have been described in detail in the literature (see, e.g., Andrews and Whitney (Arch. Biochem. Biophys. 414 (2003), 159-169)). In particular, the surgical precision of homologous recombination mechanism enables plastom manipulations (Maliga (Curr. Opin. Plant Biol. 5 (2002, 164-172)). Both, site-specific mutagenesis (Whitney et al. (Plant Physiol. 121 (1999), 579-588)) and total replacement of plastid-encoded genes are routine (Kanevski et al. (Plant Physiol. 119 (1999), 133-141); Whitney and Andrews (Proc. Natl. Acad. Sci. USA 98 (2001), 14738-14743)).
Also methods for the transformation of algae are known in the art and have been described, for example, in Fuhrmann (Protist 153 (2002), 357-364), Schroda (Curr. Genet. 49 (2006), 69-84) and Blowers et al. (Plant Cell 1 (1989), 123-132).

The resulting transformed plant cell can then be used to regenerate a transformed plant in a manner known by a skilled person.

The nucleic acid molecule encoding the RbcL subunit and the nucleic acid molecule encoding the RbcX protein are designed in a way that they allow the expression of the RbcL subunit and of the RbcX protein in the transgenic plant cell produced according to the method of the invention or in at least one part, e.g. organ tissue, cell type, of the transgenic plant produced according to a method of the invention.

Thus, the nucleic acid molecule introduced into the transgenic plant can in principle be expressed in all or substantially all cells of the plant. However, it is also possible that it is only expressed in certain parts, organs, cell types, tissues etc. Moreover, it is possible that expression of the nucleic acid molecule only takes place upon induction, e.g. at a certain developmental stage. In a preferred embodiment, the nucleic acid molecule is expressed in those parts of the plant that are photosynthetically active.
In order to be expressed, the nucleic acid molecule that encodes RbcL and/or RbcX and that is introduced into a plant cell is preferably operatively linked to one or more expression control sequences, e.g. a promoter, active in this plant cell. The promoter may be homologous or heterologous with regard to its origin and/or with regard to the gene to be expressed. Suitable promoters are for instance the promoter of the 35S RNA of the Cauliflower Mosaic Virus (see for instance US-A 5,352,605) and the ubiquitin-promoter (see for instance US-A 5,614,399) which lend themselves to constitutive expression, the patatin gene promoter B33 (Rocha-Sosa et al., EMBO J. 8 (1989), 23-29) which lends itself to a tuber-specific expression in potatoes or a promoter ensuring expression in photosynthetically active tissues only, for instance the ST-LS1 promoter (Stockhaus et al., Proc. Natl. Acad. Sci. USA 84 (1987), 7943-7947; Stockhaus et al., EMBO, J. 8 (1989) 2445-2451), the Ca/b-promoter (see for instance US-A-5,656,496, US-A-5,639,952, Bansal et al., Proc. Natl. Acad. Sci. USA 89 (1992), 3654-3658) and the Rubisco SSU promoter (see for instance US-A-5,034,322; US-A-4,962,028) or the glutelin promoter from wheat which lends itself to endosperm-specific expression (HMW promoter) (Anderson, Theoretical and Applied Genetics 96, (1998), 568-576, Thomas, Plant Cell 2 (12), (1990), 1171-1180), the glutelin promoter from rice (Takaiwa, Plant Mol. Biol. 30(6) (1996), 1207-1221, Yoshihara, FEBS Lett. 383 (1996), 213-218, Yoshihara, Plant and Cell Physiology 37 (1996), 107-111), the shrunken promoter from maize (Maas, EMBO J. 8 (11) (1990), 3447-3452, Werr, Mol. Gen. Genet. 202(3) (1986), 471-475, Werr, Mol. Gen. Genet. 212(2), (1988), 342-350), the USP promoter, the phaseolin promoter (Sengupta-Gopalan, Proc. Natl. Acad. Sci. USA 82 (1985), 3320-3324, Bustos, Plant Cell 1 (9) (1989), 839-853) or promoters of zein genes from maize (Pedersen et al., Cell 29 (1982), 1015-1026; Quatroccio et al., Plant Mol. Biol. 15 (1990), 81-93). However, promoters which are only activated at a point in time determined by external influences can also be used (see for instance WO 93/07279). In this connection, promoters of heat shock proteins which permit simple induction may be of particular interest. Likewise, artificial and/or chemically inducible promoters may be used in this context. Moreover, seed-specific promoters such as the USP promoter from Vicia faba which ensures a seed-specific expression in Vicia faba and other plants may be used (Fiedler et al., Plant Mol. Biol. 22 (1993), 669-679; Bäumlein et al., Mol. Gen. Genet. 225 (1991), 459-467). Moreover, fruit-specific promoters, such as described in WO 91/01373 may be used too. In one embodiment, promoters which ensure constitutive expression are preferred. However, in another preferred embodiment, the nucleic acid molecule may be operatively linked to a promoter which is active in photosynthetically active cells.
Moreover, the nucleic acid molecule may contain a termination sequence which serves to terminate transcription correctly and to add a poly-A-tail to the transcript which is believed to have a function in the stabilization of the transcripts. Such elements are described in the literature (see for instance Gielen et al., EMBO J. 8 (1989), 23-29) and can be replaced at will.
Furthermore, if needed, polypeptide expression can in principle be targeted to any sub-localization of plant cells (e.g. cytosol, plastids, vacuole, mitochondria) or the plant (e.g. apoplast). In order to achieve the localization in a particular compartment, the coding region to be expressed may be linked to DNA sequences encoding a signal sequence (also called "transit peptide") ensuring localization in the respective compartment. It is evident that these DNA sequences are to be arranged in the same reading frame as the coding region to be expressed. Preferably, signal sequences directing expression into the plastids, in particular the chloroplasts, are used in connection with the present invention.
In order to ensure the location in the plastids it is conceivable to use one of the following transit peptides: of the plastidic Ferredoxin: NADP+ oxidoreductase (FNR) of spinach which is enclosed in Jansen et al. (Current Genetics 13 (1988), 517-522). In particular, the sequence ranging from nucleotides -171 to 165 of the cDNA sequence disclosed therein can be used which comprises the 5' non-translated region as well as the sequence encoding the transit peptide. Another example is the transit peptide of the waxy protein of maize including the first 34 amino acid residues of the mature waxy protein (Klösgen et al., Mol. Gen. Genet. 217 (1989), 155-161). It is also possible to use this transit peptide without the first 34 amino acids of the mature protein. Furthermore, the signal peptides of the ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764), of the NADP malat dehydrogenase (Gallardo et al., Planta 197 (1995), 324-332), of the glutathione reductase (Creissen et al., Plant J. 8 (1995), 167-175) or of the R1 protein (Lorberth et al. Nature Biotechnology 16, (1998), 473-477) can be used.
In order to ensure the localization in the mitochondria, it is for example conceivable to use the transit peptide described by Braun (EMBO J. 11, (1992), 3219-3227).

The RbcL subunit and the RbcX protein may be encoded by two different nucleic acid molecules and, thus, the transgenic cells may be transformed with two separate nucleic acid molecules. However, it is also possible that the sequences encoding RbcX and RbcL are placed on the same nucleic acid molecule.

In a preferred embodiment of the above-described method according to the invention for producing a transgenic plant cell the plant cell is also transformed with a nucleic acid molecule encoding a RbcS subunit. Preferably, this RbcS subunit is homologous with respect to the RbcL subunit, i.e. it is derived from the same species, or it is at least known to be able to properly assemble with the RbcL subunit so as to give a functional form I Rubisco.

In a preferred embodiment the method for producing a transgenic plant cell also comprises the step of introducing into the plant cell a nucleic acid molecule which leads to an inhibition of the expression of an RbcX protein naturally occurring in said plant cell.
As already mentioned above, it has been found that for successfully expressing a heterologous Rubisco in plants it is necessary to also express the corresponding RbcX protein. Moreover, it has been observed that in such a case where matching heterologous RbcL and RbcX proteins are expressed in a host cell, the expression of the endogenous RbcX protein may interfere with the proper assembly of the heterologous Rubisco. Thus, it may be advantageous to inhibit in the transgenic plant cell the expression of the naturally occurring RbcX gene, i.e. the endogenous RbcX gene. The term "inhibition" means that there is a suppression, i.e. a reduction, of the expression of the RbcX protein in comparison to a corresponding non-modified wildtype cell. "Reduction" means a reduction by at least 10%, preferably at least 20%, 30%, 40%, 50%, 60%, 70% or 80%, more preferably at least 90% and most preferably of 100%. Thus, it is most preferred that expression of the endogenous RbcX protein is completely abolished. A reduction of gene expression can be measured according to well known methods. A reduction may e.g. be a reduction on the transcription level, i.e. it means that less mRNA is produced. This can be measured by determining the amount of corresponding mRNA by known methods, such as Northern Blot. A reduction may alternatively be a reduction on the protein level. This can be determined by measuring the amount of the produced RbcX protein by known methods, such a Western Blot, immunoprecipitation and the like. Methods for achieving a suppression of gene expression in a cell are well known to the person skilled in the art and are abundantly described in the literature.

It is, for example, possible to introduce into the plant cells a nucleic acid molecule which codes for an "inhibitor" of the endogenous RbcX protein wherein "inhibitor" means that it is a molecule which is capable to prevent the generation of or is capable to reduce an increased amount of or the activity of the endogenous RbcX protein in the transgenic plant.
For example, an "inhibitor" as referred to herein may be a compound that prevents the splicing of the maturing mRNA of the RbcX protein, that prevents the expression of the endogenous RbcX gene, that reduces the RbcX mRNA or that prevents the generation of (i.e. the translation of) or that reduces or inhibits the activity of the endogenous RbcX protein.
An inhibitor of the endogenous RbcX protein may be one selected from the group consisting of:
(a) a binding molecule specifically recognizing nucleotide sequences of the endogenous RbcX gene or RbcX polypeptides encoded by said nucleotide sequences
(b) a nucleic acid molecule which specifically introduces an insertion of a heterologous sequence or a mutation into the endogenous RbcX gene via in vivo mutagenesis; and
(c) a nucleic acid molecule specifically reducing the expression of mRNA of the endogenous RbcX gene.

Generally, the binding molecule may be selected from the group consisting of antibodies, affybodies, trinectins, anticalins, aptamers, antisense nucleotide sequences (like antisense DNAs or antisense RNAs), PNAs, and the like.
Based on prior art literature, the person skilled in the art is familiar with obtaining specific binding molecules that may be useful in the context of the present invention. These molecules are directed and bind specifically to or specifically label the above-described target molecules. Non-limiting examples of suitable binding molecules may be selected from aptamers (Gold, Ann. Rev. Biochem. 64 (1995), 763-797)), aptazymes, RNAi, shRNA, RNAzymes, ribozymes (see e.g., EP-B1 0 291 533, EP-A1 0 321 201, EP-B1 0 360 257), antisense DNA, antisense oligonucleotides, antisense RNA, siRNA, antibodies (Harlow and Lane "Antibodies, A Laboratory Manual", CSH Press, Cold Spring Harbor, 1988), affibodies (Hansson, Immunotechnology 4 (1999), 237-252; Henning, Hum Gene Ther. 13 (2000), 1427-1439), lectins, trinectins (Phylos Inc., Lexington, Massachusetts, USA; Xu, Chem. Biol. 9 (2002), 933), anticalins (EPB1 1 017 814) and the like.
The term "specifically recognizing" is meant to refer to the high affinity antibodies or other binding molecules known in the prior art typically have for the target molecule against which they were prepared.
Advantageously, the term "specifically recognizing" refers to a specificity of the binding molecule that allows a distinction between the above described target molecules, i.e. the endogenous RbcX gene and encoded protein, and molecules not targeted by the binding molecule(s), i.e. the heterologous RbcX nucleotide sequence introduced into the transgenic plant cell and the corresponding RbcX protein, in the sense that the binding molecule does not show a significant cross-reactivity with the latter molecules. The person skilled in the art is able to prepare such distinctive binding molecules, for example by selecting mainly or exclusively nucleotide sequences or amino acid sequences which only occur in the endogenous RbcX gene/protein as a target for the binding molecule to be prepared.
In a preferred embodiment a binding molecule comprises or is a nucleotide sequence.

A preferred binding molecule in the context of the present invention may be one selected from the group consisting of:
(a) an antisense nucleotide sequence, for example an antisense oligonucleotide, specifically binding to mRNA (or hnRNA) encoding the endogenous RbcX protein;
(b) an antibody specifically binding to mRNA encoding the endogenous RbcX protein or specifically binding to the endogenous RbcX protein;
(c) a siRNA specifically binding to mRNA encoding the endogenous RbcX protein;
(d) an aptamer specifically binding to mRNA encoding the endogenous RbcX protein or specifically binding to the endogenous RbcX protein; and
(e) a ribozyme specifically recognizing mRNA encoding the endogenous RbcX protein.

Another possible embodiment of an inhibitor in the context of the present invention is an antisense oligonucleotide suitable for "exon skipping".

The meaning of "exon skipping" is known in the art and this term is used correspondingly in the context of the present invention. For example, the technology of "exon shipping" is described in Gebski (2003, Human Molecular Genetics, 12, 15, 1801-1811).
Without being bound by theory, "exon skipping" takes advantage of (an) oligonucleotide(s) that hybridize(s)/bind(s) to the primary transcript of a gene (also referred to herein as pre-mRNA or hnRNA) in a manner that a certain exon of a gene, is not spliced into the resulting mRNA, for example, but without being bound by theory, because of an interference of the hybridized/bound oligonucleotide(s) with the splicosome assembly.
Based on his common general knowledge and the teaching of the present application, the skilled person is readily in the position to generate antisense oligonucleotides "suitable" for skipping of certain exon. For example, but not limiting, "suitable" in this context means that the antisense oligonucleotide hybridizes/binds to such part of the hnRNA that spans a splicing site of an exon.

As mentioned above, one particular binding molecule in the context of the present invention is envisaged to be an antisense nucleotide sequence, i.e. a nucleotide sequence complementary to those target molecules defined herein being nucleotide sequences.
As used herein, the term "antisense nucleotide sequence corresponding to a nucleotide sequence" means that the antisense nucleotide sequence provides the same sequence information as the nucleotide sequence to which it "corresponds", but that this sequence information is implied in that strand of a nucleotide sequence which is complement of the sense strand of said nucleotide sequence.

Particularly, the target molecules of the antisense nucleotide sequence may be transcripts of the endogenous RbcX gene (e.g. mRNA or hnRNA). Since these transcripts may include nucleotide sequences corresponding to (an) intron sequence(s) (particularly when the transcript is an hnRNA), it is also envisaged herein that the antisense nucleotide sequence in the context of the present invention may, at least partially, be antisense with respect to (an) intron sequence(s) of the endogenous RbcX gene.
The antisense nucleotide sequences as provided and described herein may have a length of at least 15, preferably of more than 50, more preferably of more than 100, even more preferably of more than 200 and most preferably of more than 500 nucleotides. However, antisense nucleotide sequences that usually are employed in the art are shorter than 5000 nucleotides or even shorter than 2500 nucleotides, and so the antisense nucleotide sequences as provided herein are intended to be.
In general, the antisense technology to reduce the amount of a desired target molecule (e.g. an mRNA) by means of an antisense effect is known in the art (see also: Antisense oligonucleotides: from design to therapeutic application. Chan, Clin Exp Pharmacol Physiol. 2006 May-Jun;33(5-6):533-40).
For this purpose, usually a nucleotide sequence (or a part thereof) corresponding to the target nucleotide sequence is linked in antisense orientation with a promoter ensuring the transcription and possibly with a termination signal ensuring the termination of the transcription as well as the polyadenylation of the transcript.

The siRNA technology as also intended to be employed in the context of the present invention is also well known in the art. Based on his common general knowledge and the teaching provided herein, the skilled person is readily in the position to find out siRNAs suitable to bind the corresponding target molecule(s), and hence, suitable to reduce products of the endogenous RbcX gene.

As mentioned above, one particular binding molecule in the context of the present invention is an antibody specific for/specifically binding the target molecules as defined herein, particularly the endogenous RbcX mRNA or to the endogenous RbcX protein.
The antibody useful in the context of the present invention can be, for example, polyclonal or monoclonal. The term "antibody" also comprises derivatives or fragments thereof which still retain the binding specificity.
The target molecules according to the invention, its fragments or other derivatives thereof, or cells expressing them, can be used as an immunogen to produce antibodies thereto. The present invention in particular also includes chimeric, single chain, and humanized antibodies, as well as Fab fragments, or the product of an Fab expression library. Various procedures known in the art may be used for the production of such antibodies and fragments.
In the context of the present invention, the term "antibody" relates to full immunoglobulin molecules as well as to parts of such immunoglobulin molecules substantially retaining binding specificity. Furthermore, the term relates, as discussed above, to modified and/or altered antibody molecules, like chimeric and humanized antibodies. The term also relates to recombinantly or synthetically generated/synthesized antibodies. The term also relates to intact antibodies as well as to antibody fragments thereof, like, separated light and heavy chains, Fab, Fab/c, Fv, Fab', F(ab')₂. The term "antibody" also comprises bifunctional antibodies, trifunctional antibodies and antibody constructs, like single chain Fvs (scFv) or antibody-fusion proteins.
Techniques for the production of antibodies are well known in the art and described, e.g. in Harlow and Lane "Antibodies, A Laboratory Manual", CSH Press, Cold Spring Harbor, 1988. Antibodies directed against a polypeptide according to the present invention can be obtained, e.g., by direct injection of the target molecule into an animal or by administering the target molecule to an animal, preferably a non-human animal. The antibody so obtained will then bind the target molecule itself. In this manner, even a fragment of the target molecule can be used to generate antibodies binding the whole target molecule, as long as said binding is "specific" as defined above.
Particularly preferred in the context of the present invention are monoclonal antibodies. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Various procedures are known in the art and may be used for the production of such antibodies and/or fragments. Thus, the antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the respective RNA or polypeptide.
Surface plasmon resonance as employed in the BIAcore system can be used to increase the efficiency of phage antibodies which bind to an epitope of the polypeptide (Schier, Human Antibodies Hybridomas 7 (1996), 97-105; Malmborg, J. Immunol. Methods 183 (1995), 7-13). Accordingly, also phage antibodies can be used in the context of this invention.

In accordance with the present invention, the term "aptamer" means nucleic acid molecules that can specifically bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold (1995), Ann. Rev. Biochem 64 , 763-797).

In a preferred embodiment, the antibody or aptamer as provided and described herein is envisaged to specifically bind to a nucleotide sequence of the endogenous RbcX gene or to the endogenous RbcX polypeptide.
It is clear that for the purpose of antibody/aptamer production, peptides can be employed that represent (at least partially) an epitope of the amino acid sequence encoded by the endogenous RbcX gene and, optionally, that can further be modified according to the corresponding general practice well known in the art. The skilled person is readily in the position deduce which modification(s) of a peptide representing an epitope of the amino acid sequence encoded by the endogenous RbcX gene are suitable in order to generate the antibodies/aptamers provided and described in the context of the present invention.

Another particular binding molecule provided and described herein is a ribozyme, particularly such a ribozyme that specifically recognizes and cleaves the target nucleotide sequences as defined herein. The ribozyme technology is also well known in the art. Generally, ribozymes are known to be catalytically active RNA molecules capable of cleaving RNA molecules and specific target sequences. By means of recombinant DNA techniques it is possible to alter the specificity of ribozymes. There are various classes of ribozymes. For practical applications aiming at the specific cleavage of the transcript of a certain gene, use is preferably made of representatives of two different groups of ribozymes. The first group is made up of ribozymes which belong to the group I intron ribozyme type. The second group consists of ribozymes which, as a characteristic structural feature, exhibit the socalled "hammerhead" motif. The specific recognition of the target RNA molecule may be modified by altering the sequences flanking this motif. By base pairing with sequences in the target molecule these sequences determine the position at which the catalytic reaction and therefore the cleavage of the target molecule takes place. Since the sequence requirements for an efficient cleavage are low, it is in principle possible to develop specific ribozymes for practically each desired RNA molecule.
In order to produce nucleotide sequences encoding a ribozyme which specifically cleaves the target nucleotide sequence(s) as defined herein, for example, a DNA sequence encoding a catalytic domain of a ribozyme is bilaterally linked with DNA sequences which are homologous to sequences of the target nucleotide sequence(s). Sequences encoding the catalytic domain may for example be the catalytic domains of the satellite DNA of the SCMo virus (Davies, Virology 177 (1990), 216-224) or that of the satellite DNA or the TobR virus (Steinecke, EMBO J. 11 (1992), 1525-1530; Haseloff, Nature 334 (1988), 585-591). The DNA sequences flanking the catalytic domain are preferably derived from the above-described target nucleotide sequences. The general principle of the expression of ribozymes and the method is described, for example, in EP-B1 0 321 201.

In one embodiment, the inhibitor of the endogenous RbcX gene is a nucleic acid molecule that leads to a reduction of the target molecules via in vivo mutagenesis. Thereby, an insertion of a heterologous sequence or a mutation into nucleotide sequences comprising of the endogenous RbcX gene leads to an inhibition of the expression of the gene. Generally, methods of "in vivo mutagenesis" (also known as "chimeroplasty") are known in the art. In such methods, a hybrid RNA/DNA oligonucleotide (chimeroplast) is introduced into cells (WO 95/15972; Kren, Hepatology 25 (21997), 1462-1468; Cole-Stauss, Science 273 (1996), 1386-1389). A part of the DNA component of the RNA/DNA oligonucleotide is homologous to a nucleotide sequence occurring endogenously in the cell and encoding a corresponding protein, but displays a mutation or comprises a heterologous part which lies within the homologous region. Due to base pairing of the regions of the RNA/DNA oligonucleotide which are homologous to the endogenous sequence with these sequences, followed by homologous recombination, the mutation or the heterologous part contained in the DNA component of the oligonucleotide can be introduced into the cell genome. This leads to a reduction of the activity, i.e. expression, of the gene, into which the heterologous part or the mutation has been introduced.

In view of the above, it is clear that the nucleic acid molecule causing in vivo mutagenesis comprises a heterologous sequence or a sequence carrying a mutation flanked by parts of the nucleotide sequence of the endogenous RbcX gene.

In a further embodiment of the invention, the inhibitor is a nucleic acid molecule that leads to a reduction of the target molecules by a cosuppression effect. "Cosuppression effect" means that the synthesis of a nucleotide sequence, particularly of an RNA, in a living cell reduces the expression of a gene being homologous to said nucleotide sequence. The general principle of cosuppression and corresponding methods are well known to the person skilled in the art and are described, for example, in Pal-Bhadra (Cell 90, 1997), 479-490) and Birchler (Nature Genetics 21 (1999), 148-149).
In a particular embodiment, the nucleic acid molecule causing a cosuppression effect comprises a nucleotide sequence of the endogenous RbcX gene.

The present invention also relates to a method for producing a transgenic plant comprising the step (i) of the above-described method for producing a transgenic plant cell and furthermore the step of (ii) regenerating from the cell obtained according to step (i) plants.

The method according to the invention for producing a transgenic plant optionally also comprises further the step of producing progeny from the plant produced according to an above-described method.

The present invention also relates to transgenic plant cells comprising a nucleic acid molecule encoding a heterologous form I RbcL subunit and a nucleic acid molecule encoding a RbcX protein, wherein the RbcL subunit and the RbcX protein are derived from the same species as well as to transgenic plant cells obtainable by the above-described methods.
The term "heterologous" means that the sequence encoding RbcL does naturally not occur in a corresponding non-transformed cell. In particular, the sequence encoding RbcL is derived from a different species than that plant cell.

The present invention also relates to transgenic plants comprising the above-described transgenic plant cells of the present invention or obtainable by the above-described method. The transgenic plant cells of the present invention may be cells of any possible plant organism. The term "plant" has the meaning as described hereinabove in connection with the methods according to the invention. Namely, this term inlcudes slime fungi, fungi, lichen, eukaryotic algae, moss, fern and higher plants. In a preferred embodiment the term plant refers to eukaryotic algae or higher plants. The findings of the present invention are in particular useful for preparing plants which express a Rubisco with an increased capacity to fixate CO₂. Such plants may be useful in approaches to reduce the CO₂ level of the atmosphere, thereby supporting attempts to prevent the changes in climate due to global warming. Plants which are especially interesting in this regard are, e.g., algae which contribute considerably to the biomass in the oceans, lakes and rivers but also fast growing trees. Plants with an improved capacity to fixate CO₂ are of course also interesting in agriculture. Thus, the term "plant" in a preferred embodiment relates to a useful plant, in particular a plant of agricultural interest, such as cereals (e.g., corn, wheat, rye, barley, rice, sorghum, oats etc.), vegetables (e.g., cucumber, tomato, melon, cabbage, potato, eggplant, zucchini, pumpkin, leek, etc.) or fruit (e.g. apple, pear, plum, banana, berries, etc.). Of particular interest are plants which can be cultivated in order to obtain biological substances of interest for producing energy, e.g. oil producing plants such as rapeseed or oil palm, sugar producing plants, e.g. sugar beet, sugar cane etc., or plants which produce high amounts of storage carbohydrates, such as starch, e.g. cereal plants.

### FIGURE LEGENDS

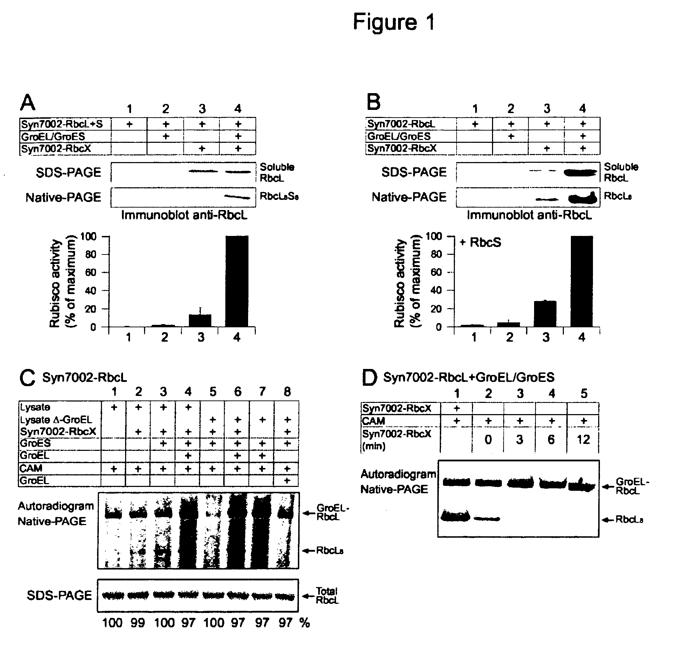

**Figure 1****. Requirement of the Chaperonin System and RbcX for Rubisco Assembly.**
(A and B) Syn7002-RbcL and RbcS (A) or Syn7002-RbcL (B) were expressed in E. coli with or without coexpression of Syn7002-RbcX and GroEL/GroES as indicated. RbcL in soluble cell lysates was analyzed by SDS-PAGE and RbcL₈S₈ or RbcL₈ complexes by Native-PAGE and immunoblotting. Carboxylation activity was measured directly in soluble cell lysates (A) or upon addition of purified RbcS (B). Activities measured upon coexpression with GroEL/GroES and RbcX are set to 100%.
(C) Syn7002-RbcL was translated in E. coli lysate in vitro in the presence of ³⁵S-Met (1.5 h, 30°C). When indicated, GroEL/GroES (0.5 µM/1.0 µM) and Syn7002-RbcX (40 µM) were added to normal lysate (lanes 1-4) or GroEL-depleted lysate (Δ-GroEL, lanes 5-8). In lane 8, GroEL was added after stopping translation with CAM. Assembled RbcL₈ and total RbcL protein were analyzed by Native-PAGE and SDS-PAGE, respectively, followed by autoradiography.
(D) Syn7002-RbcL was translated in the presence of GroEL/GroES as in (C). RbcX was either present during translation (lane 1), was added together with CAM (lane 2) or after CAM addition (lanes 3-5) and assembled RbcL₈ analyzed by Native-PAGE.

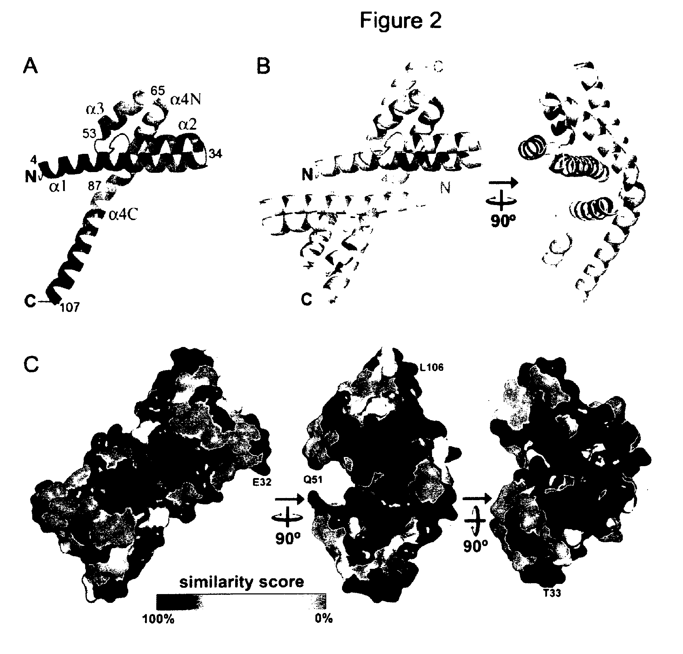

**Figure 2****. Crystal Structure of Syn7002-RbcX.**
**(A)** Ribbon representation of the RbcX monomer. The peptide backbone is depicted from N- to C-terminus using a color gradient from blue to red. Secondary structure elements, selected residue numbers and chain termini are indicated.
**(B)** Structure of the RbcX dimer. Protomers are shown in yellow and blue.
**(C)** Surface conservation in RbcX. The similarity score from an alignment of 151 sequences of cyanobacterial RbcX in the PFAM database was plotted onto the accessible surface of the RbcX dimer. Sequence conservation is indicated by a color gradient, indicating highly conserved residues in magenta and variable regions in cyan. The positions of conserved surface residues are indicated.

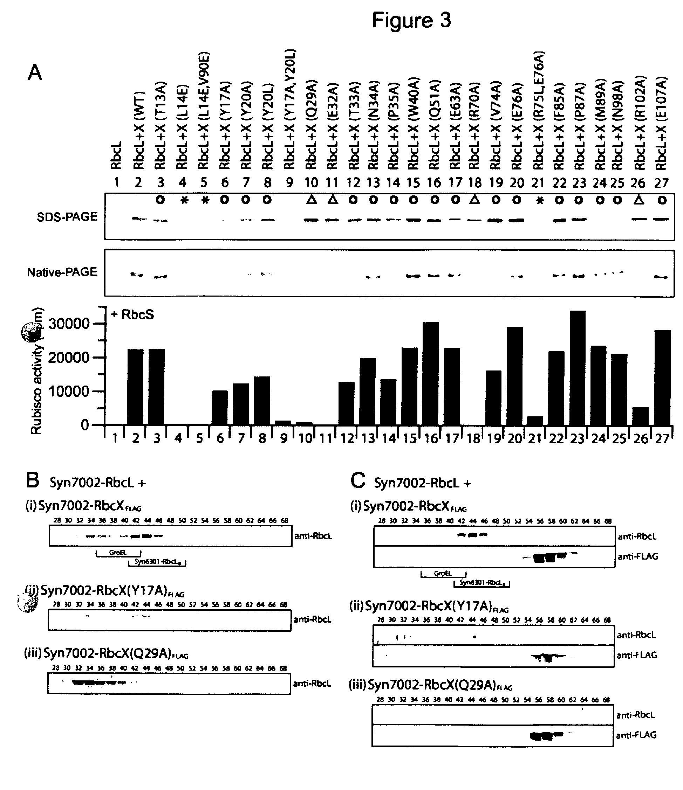

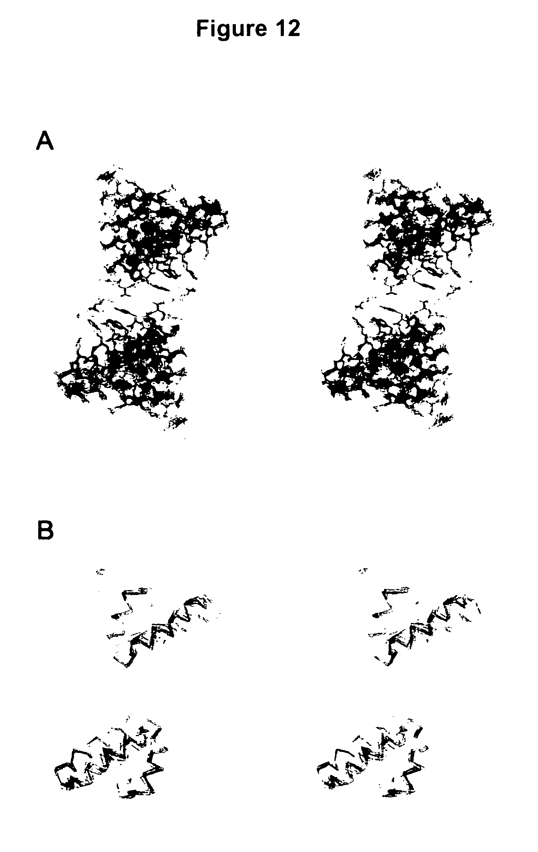

**Figure 3****. Functional Analysis of Syn7002-RbcX Mutants.**
**(A)** Soluble RbcL (SDS-PAGE), formation of RbcL₈ (Native-PAGE) and carboxylation activity were analyzed as in Figure 1B upon coexpression of Syn7002-RbcL in E. coli with wildtype and mutant RbcX proteins as indicated. (o), RbcX mutants supporting production of soluble and assembled RbcL; (*), RbcX mutants exhibiting poor solubility (see Figure 12); (Δ), RbcX mutants supporting formation of soluble RbcL but little or no assembly of RbcL₈. and formation of active enzyme upon RbcS addition.
**(B)** Gel filtration analysis of soluble lysates prepared from E. coli cells coexpressing Syn7002-RbcL with (i) RbcX_{FLAG}, (ii) RbcX(Y17A) FLAG or (iii) RbcX(Q29A) FLAG. Fractions were analyzed by SDS-PAGE and immunoblotted for RbcL. Elution of GroEL (-800 kDa) and Syn6301-RbcL₈ (-400 kDa) is indicated.
**(C)** Co-immunoprecipitation of RbcL with anti-FLAG antibody from cell lysates in (B), followed by native elution with FLAG peptide and gel filtration analysis as above. Fractions from gel filtration were immunoblotted for RbcL and RbcX_{FLAG}.

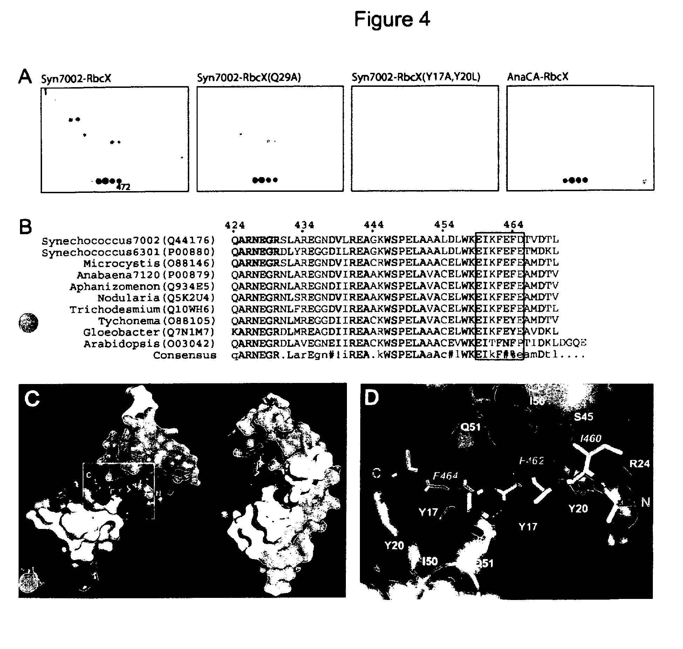

**Figure 4****. Binding of C-terminal RbcL Peptide to RbcX.**
**(A)** A cellulose membrane containing an array of overlapping dodecamer peptides covering the sequence of Syn7002-RbcL was probed with the RbcX proteins indicated. Peptide-bound RbcX was visualized by chemiluminescent immunodetection with anti-RbcX antibody.
**(B)** Alignment using MultAlin (Corpet (Nucl. Acids Res. 16 (1988), 10881-10890)) of C-terminal amino acid sequences of RbcL from the cyanobacterial and higher plant species indicated (Swiss-Prot accession numbers in brackets). High consensus level (≥ 90 %) is depicted in red and low consensus level (≥ 50 %) in blue.
**(C)** Structure of the complex of peptide EIKFEFD bound to RbcX dimer. The peptide is shown in ball-and-stick representation; RbcX is represented as a molecular surface with protomers colored white and blue, respectively. N- and C-termini of the peptide are indicated.
**(D)** Magnification of boxed area in (C) presenting a view of the refined peptide bound to RbcX. Molecular interactions between peptide and RbcX are highlighted. Dashed lines represent hydrogen bonds. Residues of the RbcX monomers participating in peptide binding are displayed in ball-and-stick representation below the transparent surface of the molecule and are numbered in white and yellow, respectively. The important hydrophobic residues in the bound peptide are also labeled.

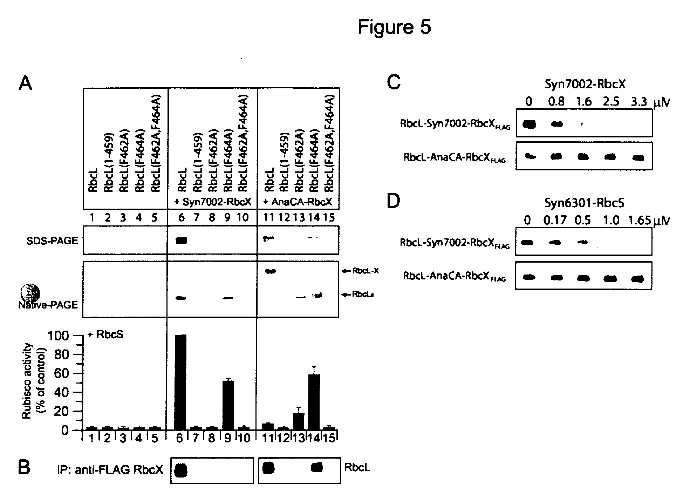

Figure 5. Effects of Modulating RbcX Binding Strength on RbcL Assembly.
**(A)** Syn7002-RbcL or C-terminal mutants indicated were expressed in E. coli either alone or with Syn7002-RbcX or AnaCA-RbcX. Soluble fractions of cell lysates were analyzed for RbcL (SDS-PAGE), RbcL₈ (Native-PAGE) and carboxylation activity as in Figure 1B.
**(B)** Soluble lysates from coexpression of Syn7002-RbcL wildtype or mutants with Syn7002-RbcX_{FLAG} or AnaCA-RbcX_{FLAG} were subjected to immunoprecipitation with anti-FLAG affinity beads. Co-immunoprecipitated RbcL was detected by immunoblotting with RbcL antibody.
**(C and D)** Dynamic nature of the RbcL₈-RbcX interaction. Complexes of Syn7002-RbcL/Syn7002-RbcX_{FLAG} or Syn7002-RbcL/AnaCA-RbcX_{FLAG} produced by coexpression in E. coli were immunoprecipitated from soluble lysate as in (B). The immunobeads were then incubated with the indicated concentrations of purified non-tagged Syn7002-RbcX (C) or RbcS (D) for 15 min at room temperature. Bound RbcL was eluted and analyzed by SDS-PAGE and immunoblotting.

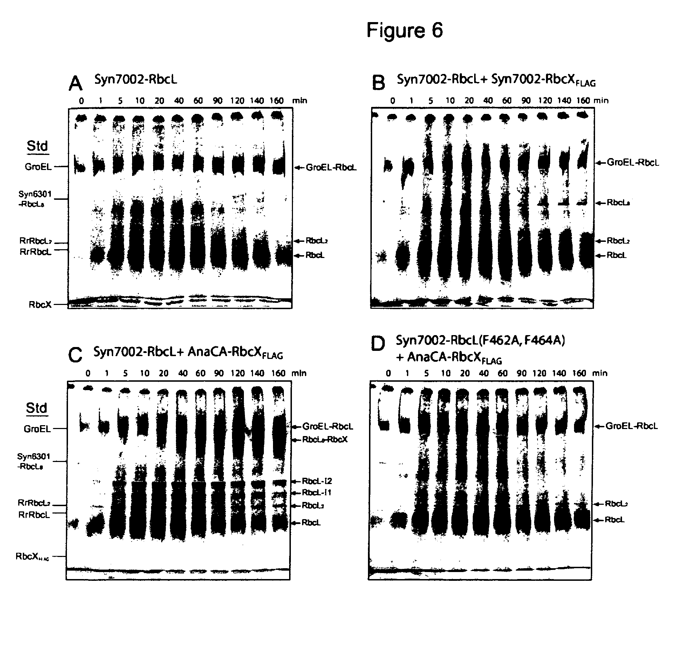

**Figure 6****. Pulse-chase Analysis of RbcL Assembly upon In Vitro Translation.**
Pulse-chase translation of Syn7002-RbcL was performed as described in Experimental Procedures in the absence of RbcX **(A),** presence of Syn7002-RbcX_{FLAG} **(B),** or AnaCA-RbCX_{FLAG} **(C).** Syn7002-RbcL(F462A, F464A) was translated in the presence of AnaCA-RbcX_{FLAG} **(D).** Reactions were stopped at the chase times indicated analyzed by discontinuous Bis-Tris Native-PAGE and autoradiography. Positions of protein standards are indicated.

**Figure 7****. Working Model of RbcX Function in Cyanobacterial Rubisco Assembly.**
RbcX acts on folded RbcL subunits subsequent to their GroEL/GroES-mediated folding. Recognition of RbcX requires the exposed C-terminal RbcL peptide (see also Figure 18).

**Figure 8****. Coexpression of RbcL with RbcX and GroEL/GroES**
**(A)** Fractionation properties on Native-Page of the proteins indicated, analyzed by Coomassie staining (left panel) and immunoblotting with anti-RbcL (right panel).
**(B)** Effect of coexpression of Syn7002-RbcX on the assembly and activity of Syn6301-RbcL in E. coli overexpressing GroEL/GroES. Soluble cell lysate was analyzed for RbcL and carboxylation activity as in Figure 1B.

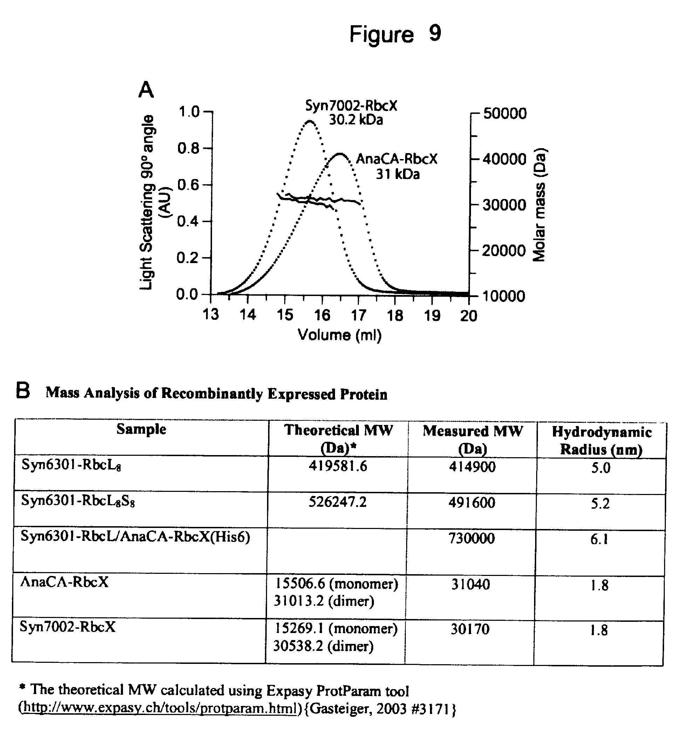

**Figure 9****. Mass Analysis of Recombinantly Expressed Proteins**
**(A)** Example of FFF-MALS data showing the analysis of AnaCA-RbcX (31040 Da) and Syn7002-RbcX (30170 Da). Horizontal lines across the peaks indicate molar mass and homogeneity of the sample.
**(B)** Molar mass and the hydrodynamic radius of purified proteins was analyzed by multi-angle light scattering (FFF-MALS, see Material and Methods).

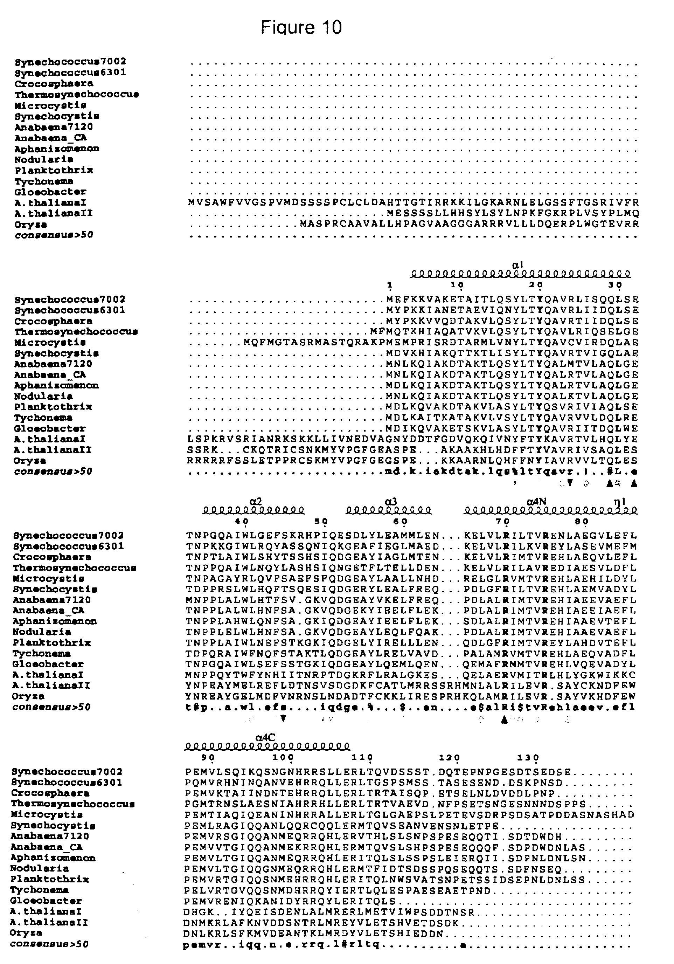

**Figure 10****. Amino Acid Sequence Alignment of Selected RbcX Homologs.**
Amino acid sequences of selected RbcX homologs were aligned using the MultAlin server. Residues indicated in red are invariable and residues indicated in blue conserved in more than 50 % of the sequences. A consensus sequence according to these criteria using the standard symbols of the MultAlin analysis program (Corpet (Nuc. Acids Res. 16 (1988), 10881-10890)) is given in the bottom row of the alignment. The secondary structure elements and the residue numbering for RbcX of *Synechococcus* sp. PCC7002 are indicated above the sequences. Residues of the central groove of RbcX contacting the phenylalanines and isoleucines in the C-terminal recognition motif of RbcL are indicated by yellow and green inverted triangles, respectively. Red triangles denote residues at the corner surfaces of RbcX implicated by mutagenesis in binding to an uncharacterized surface of RbcL. Open blue circles denote structural residues involved in dimerization and filled blue circles indicate residues forming the hydrophobic core of the four-helix bundle. SwissProt accession numbers of the sequences shown are as follows: *Synechococcus* sp. PCC7002 (Q44177), *Synechococcus* sp. PCC6301 (Q5MZ09), *Crocosphaera watsonii* (Q4C1R0), *Thermosynechococcus elongatus* (Q8DIS6), *Microcystis aeruginosa* NIVA-CYA 143 (Q9R3Q3), *Synechocystis* sp. PCC 6803 (Q55670), *Anabaena* sp. PCC7120 (Q44307), *Anabaena* sp. CA (Q44212), *Aphanizomenon gracile* (Q933W0), *Nodularia harveyana* BECID29 (Q5K2U3), *Planktothrix agardhii* NIVA-CYA 127 (Q61833), *Tychonema bourrellyi* (086944), *Gloeobacter violaceus,* Q7NIM6, *A. thaliana* gene I (NP_568382), *A. thaliana* gene II (NP_567263), and *Oryza sativa* (japonica cultivar-group) (Q84M38).

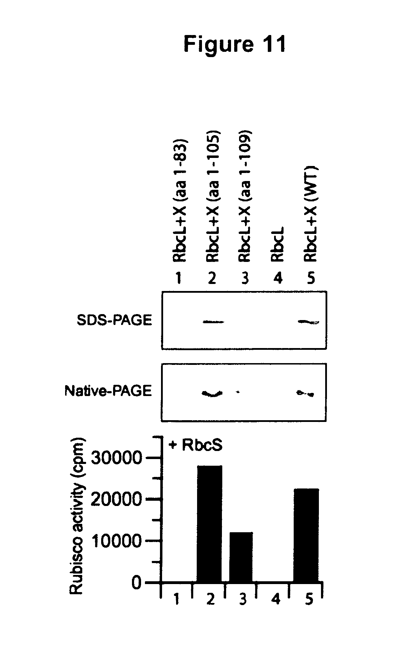

**Figure 11****. C-terminal Truncation Mutants of Syn7002-RbcX.**
Effects of coexpression of C-terminal truncated Syn7002-RbcX on solubility and assembly of Syn7002-RbcL in *E. coli*. Soluble lysates were analyzed for RbcL and carboxylation activity as in Figure1B. Syn7002-RbcL expressed in the absence or presence of wildtype Syn7002-RbcX served as control.

**Figure 12****. Conformational Ensemble of RbcX Structures From Synechococcus sp. PCC7002.**
**(A)** Stereo representation of a conformation ensemble generated by superposition of 18 individual conformations observed in the crystal lattices. Covalent bonds are shown as sticks. Carbon, nitrogen, oxygen, and sulfur atoms are indicated by black, blue, red and yellow color, respectively.
**(B)** Stereo representation of the superposed peptide backbones of RbcX dimers. Helix 2 in the foreground appears to be the most mobile element in the structure.

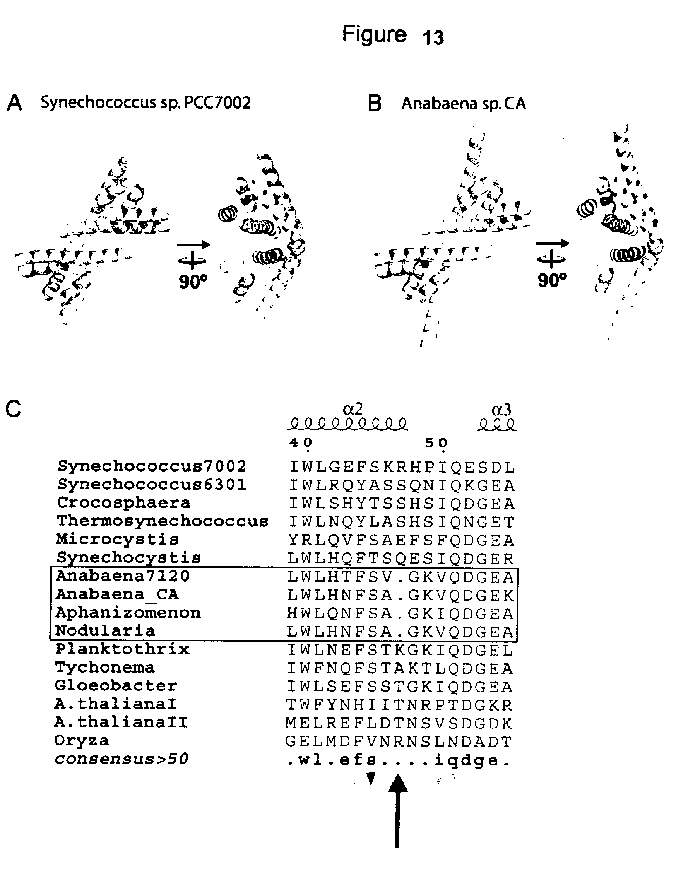

**Figure 13****. Comparison of Crystal Structures of Syn7002-RbcX and AnaCA-RbcX**
**(A, B)** Panels A and B show the structures of RbcX from *Synechococcus* sp. PCC7002 and *Anabaena sp.* CA in the same views as in Figure 2B. A residual mean square deviation of 1.09 Å for 190 Cα atoms was calculated, indicating that the structures are virtually identical in the common region.
**(C)** Sequence alignment of the loop region between α-helices 2 and 3 (residues 46 to 52) in RbcX, which forms important contacts to the C-terminal RbcL peptide in the complex with RbcX from *Synechococcus* sp. PCC7002 (excerpt from Figure 9). In RbcX sequences from the order Nostocales (boxed), including AnaCA-RbcX, this loop is shorter by one residue (position indicated by arrow) and contains a lysine at position 49, thus introducing a positive charge in the proximity of the binding groove. This may explain the increase in affinity of AnaCA-RbcX for the negatively charged C-terminus of RbcL. Residues contacting the phenylalanine and isoleucine residues in the C-terminal recognition motif are indicated by yellow and green triangles, respectively.

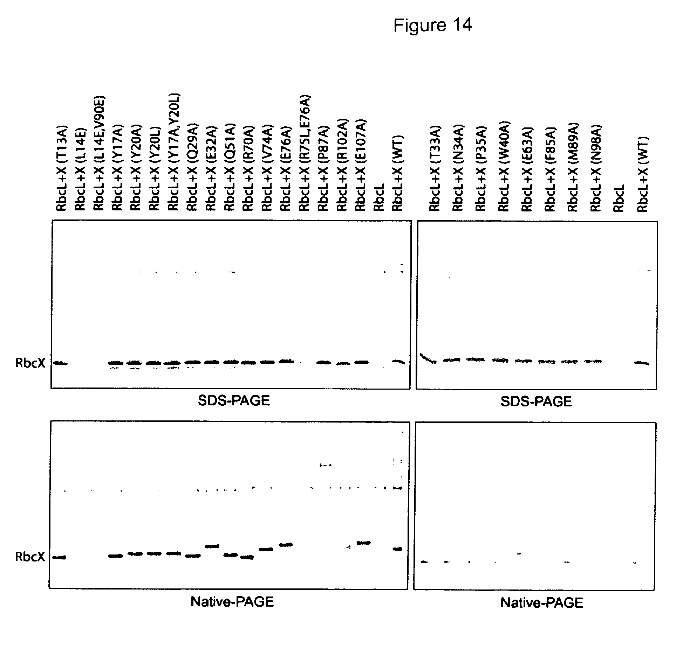

**Figure 14****. Analysis of Syn7002-RbcX Mutants.**
Syn7002-RbcX mutant proteins were coexpressed with Syn7002-RbcL in *E. coli* (see Figure 3A). Soluble lysates were analyzed by SDS-PAGE and Native-PAGE followed by Coomassie staining.

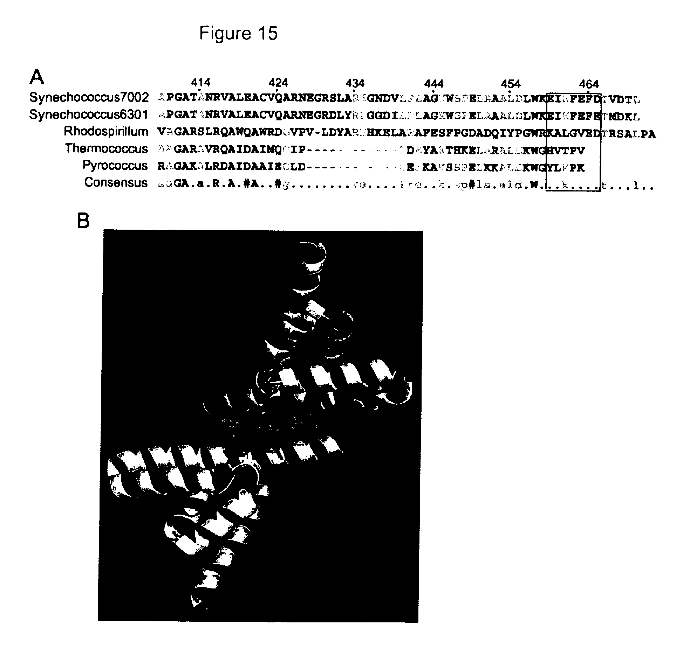

**Figure 15****. Sequence of C-terminal Peptide of RbcL and its Structure in Complex with RbcX.**
**(A)** Comparison of C-terminal RbcL sequences. Alignment using MultAlin (Corpet (Nucl. Acid Res. 16 (1988), 10881-10890)) of the RbcL sequences of the cyanobacterial species *Synechococcus* sp. PCC7002 (Q44176), *Synechococcus* sp. PCC6301 (P00880), the proteobacterium *Rhodospirillum rubrum* (Rhodospirillales) (Q2RRP5), the archaeal species Thermococcus kodakaraensis (093627) and Pyrococcus abyssi (Q9UZD7) showing only the region of residues 409-470 (residue numbers based on Syn7002-RbcL). High consensus level (≥ 90 %) is depicted in red, low consensus level (≥ 50 %) in blue and # indicates residue E, D or Q.
**(B)** Unbiased difference density for peptide EIKFEFD in the complex structure with Syn7002-RbcX. The green meshwork indicates the Fo-Fc difference density for the peptide after 10 cycles of refinement using the wildtype RbcX as model structure at 2.5 σ. The RbcX dimer is shown in ribbon representation. In the crystal structure, clear difference density for bound peptide was observed for only one of three RbcX dimers. One of the other dimers has a considerably more open structure; while the third dimer contains some spurious density at the approximate position of the phenyl rings of the peptide, but steric hindrance with a crystal contact presumably precluded stable binding in a fully extended conformation.

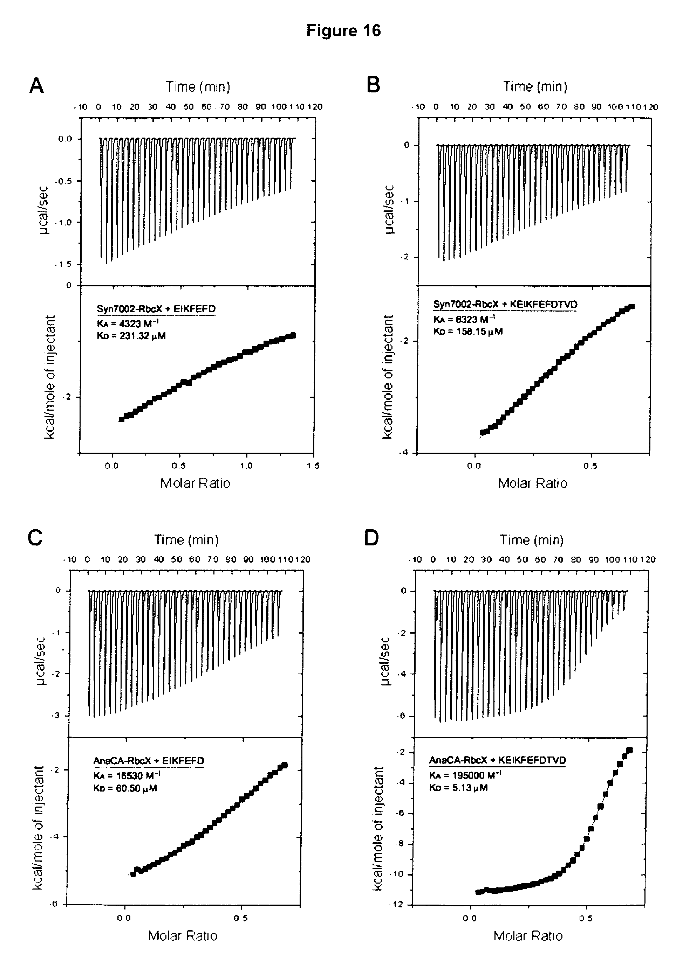

**Figure 16****. Isothermal Titration Calorimetry of the RbcX-peptide Interaction**
Titration of Syn7002-RbcX with Syn7002-RbcL peptide 1, EIKFEFD **(A),** or peptide 2, KEIKFEFDTVD **(B).** Titration of AnaCA-RbcX with Syn7002-RbcL peptide 1 **(C),** or peptide 2 **(D)**. Titration was performed as described in Materials and Methods.

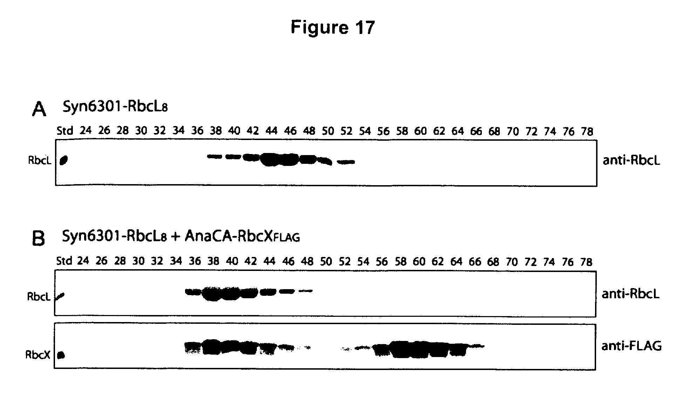

**Figure 17****. Analytical Gel-Filtration of Syn6301-RbcL₈ and Syn6301-RbcL₈/AnaCA-RbcX_{FLAG} complex.**
**(A)** 2 µM of Syn6301-RbcL₈ was analyzed on a Superdex 200 gel filtration column equilibrated in 50 mM Tris-HCl pH 8.0, 50 mM NaCl, 5 mM MgCl₂. 250 µl fractions were analyzed by 17.5 % SDS-PAGE and immunoblotting with anti-RbcL.
**(B)** 2 µM of Syn6301-RbcL₈ and 40 µM of AnaCA-RbcX_{FLAG} were incubated for 60 min at 4°C and separated by gel filtration as above, followed by immunoblotting with anti-RbcL and anti-FLAG as indicated.

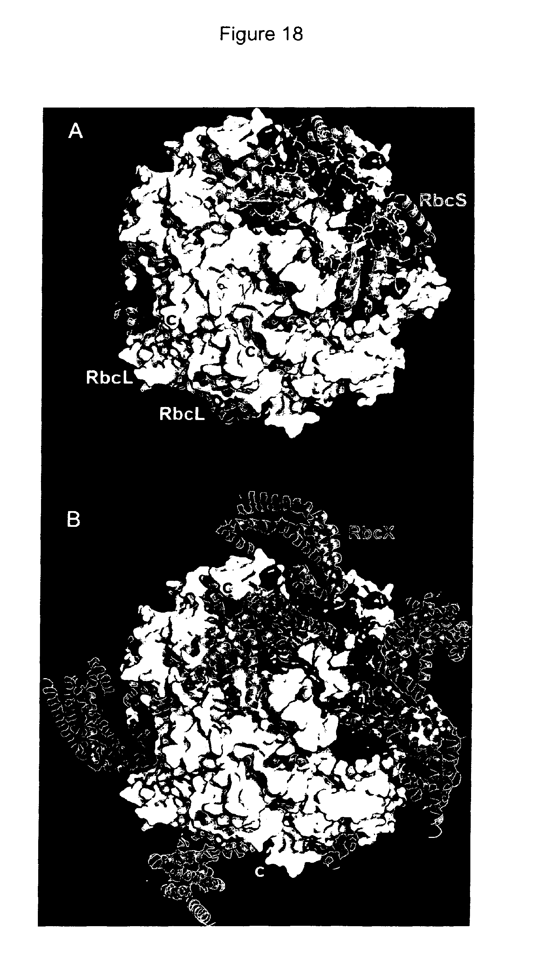

**Figure 18****. Crystal Structure of Syn6301-RbcL₈S₈ and Model of the RbcL₈-RbcX Complex.**
**(A)** Molecular representation of the Rubisco complex of Synechococcus sp. PCC6301 bound to CABP (pdb code, 1rbl) (Newman et al. (Acta Cryst. D Biol. Crystallogr. 49 (1993), 548-560)). The RbcL subunits are shown in surface representation, with the exception of the C-terminal peptides comprising residues 460-475, which is depicted as blue coils. This conformation is found only in complex with the enzymatic substrate. Subunits comprising RbcL dimers are colored white and pale blue; residues interacting with RbcS are shown in red. The small subunits themselves are shown as green ribbons.
**(B)** The lower panel is a hypothetical model of RbcX binding to the assembled RbcL₈ core complex using the same representation as in (A). The model is based on the structures of the Syn6301-RbcL₈S₈ and the RbcX-peptide complex. One edge of RbcX fills the gap between adjacent RbcL dimers, masking most of the surface covered by the globular domain of RbcS in the holoenzyme complex. In this arrangement, the C-termini of RbcL (shown in red) kink horizontally immediately N-terminal to the recognition motif, leaving the secondary structure composition of RbcL intact.

**Figure 19****. Comparison of C-termini of RbcL subunits from different sources.**
**(A)** Shown are the C-terminal sequences of the RbcL subunits of Rubisco from different organisms as indicated on the left-hand side. The conserved motiv of 12 amino acids is highlighted in each sequence. The underlined "D" is the aspartic acid residue involved in anchoring the C-terminus in the closed position (Duff et al. (J. Biol. Chem. 298 (2000), 903-916)).
**(B)** Shown are only the conserved sequences which are always 12 amino acid residues long with the only exception of Zea mays where the spacing between the conserved "I"and the conserved "D" is 6 instead of 5. Also indicated is a proposal for a consensus sequence.

**Figure 20****. Amino acid sequences of plant RbcX proteins.**
Shown is an alignment of the RbcX protein sequence of Synechococcus sp. PCC7002 and the amino acid sequences of RbcX proteins from Arabidopsis thaliana, Oryza sativa, Zea mays, Physcomitrella patens (moss), and Chlamydomonas reinhardtii.

**Figure 21****.** Alignment of amino acid sequences of RbcX proteins from different sources. The plant RbcX proteins are shown with the transit peptides at the N-terminus.

**Figure 22****. Alignment of amino acid sequences of red algae RbcX proteins.**

The following non-limiting Examples serve to further illustrate the invention.

### EXAMPLES

### Materials and Methods

### Plasmids and Proteins

Plasmid constructs for expression of Rubisco and RbcX wildtype and mutant proteins from cyanobacterial species Synechococcus sp. PCC7002, Synechococcus sp. PCC6301 and Anabaena sp. CA, as well as the expression and purification of chaperone and Rubisco proteins are described in the following.

### Plasmids

Syn7002-*rbcL*, Syn7002-*rbcS* and Syn7002-ORF134 (encoding RbcX) were amplified by PCR from genomic DNA of *Synechococcus sp.* PCC7002 [PR-6] (ATCC Nr. 27264) (Brahamsha (Appl. & Environ. Microbiol. 62 (1996), 1747-1751)) and cloned between Ndel/BamHl restriction sites into Novagen plasmids (Darmstadt, Germany) to generate the following constructs: Syn7002-*rbcL*-pET11a, Syn7002-*rbcS*-pET11a, Syn7002-*rbcX*-pET11a, Syn7002-*rbcX*_{*His*6}-pET28b and Syn7002-*rbcX-*pET30b. The coexpression plasmids Syn7002-*rbcLX*-CoEx-pET11a/28b/30b and Syn7002-*rbcLS*-CoEx-pET11a were generated by extracting the *rbcL* expression cassette from Syn7002-*rbcL*-pET11a with Sphl/Nhel and introducing it between the Sphl and Xbal sites of Syn7002-*rbcX*-pET11a/28b_{His6} (N-terminal)/30b or Syn7002-*rbcS-*pET11a. For FLAG-tagged constructs, Syn7002-*rbcX*_{FLAG}-pET30b, Syn7002-*rbcLX_{FLAG}*-CoEx-pET30b (for expression of both wildtype or mutant RbcX) and Syn7002-*rbcS_{FLAG}*-pET11a, the FLAG-tag sequence (MDYKDDDDKAG) was introduced in front of the start codon for *rbcX* or *rbcS.* The C-terminally truncated Syn7002-*rbcX* or Syn7002-*rbcL* constructs were cloned using modified reverse primers. Point mutations in *rbcX* or *rbcL* were introduced by whole plasmid PCR with mutation-site specific primers (Weiner et al. (Gene 151 (1994), 119-123).
The plasmids pUC-*rbcLS* of species *Synechococcus sp.* PCC6301 (Smith and Tabita (J. Mol. Biol. 331 (2003), 557-569) and pLS32 of species Anabaena sp. CA (Li and Tabita (J. Bacteriol. 179 (1997), 3793-3796) were a kind gift from F. R. Tabita and were used as templates to PCR the *rbcL, rbcS*, *rbcLS,* and *rbcX* genes. These were cloned into pET-vectors (Novagen) between the Ndel and BamHl-sites, resulting in Syn6301-rbcL-pET11a, Syn6301-*rbcS*-pET11a, Syn6301-rbcLS-pET11a, AnaCA-*rbcX*-pET11a as well as AnaCA-rbcX-pET30b. Plasmids Syn6301-*rbcL*-pET11a with C-terminal deletion or mutations, AnaCA-*rbcX_{FLAG}-*pET30b as well as Syn7002-*rbcL*/AnaCA-*rbcX_{FLAG}*-CoEx-pET30b were cloned as described above.
Plasmid pBAD33ES/EL encoding the arabinose inducible *groEL*/*ES* operon of E. coli and plasmid pG-KJE8 (CamR), containing the operons for both E. coli *groEL*/*ES* and *dnaK*/*dnaJ*/*grpE* inducible by tetracycline and arabinose respectively were as described (Ewalt et al. (Cell 90 (1997), 491-500); Nishihara et al. (Appl. Environ. Microbiol. 66 (2000), 884-889)).

### Proteins

The E. coli proteins GroEL, GroES, GroEL(D87K) and Rubisco from Rhodospirillum rubrum (RrRbcL and mutant RrRbcL(K168E)) were purified as described previously (Brinker et al. (Cell 107 (2001), 223-233); Hayer-Hartl et al. (Embo J. 15 (1996), 6111-6121); Kerner et al. (Cell 122 (2005), 209-220)).

### RbcX

E. coli BL21(DE3) cells, transformed with respective RbcX plasmids, were grown at 37°C and induced with 1 mM isopropyl-β-D-galactopyranoside (IPTG) according to standard procedures. Harvested cells were incubated for 1 h in lysis buffer (50 mM Tris-HCl pH 8.0, 20 mM NaCl, 1.0 mM EDTA, 1.0 mM DTT, 0.5 mg/ml lysozyme, 10 U/ml Benzonase, Complete protease inhibitor cocktail) at 4°C and disrupted by ultrasonication. After removal of cell debris by centrifugation, the supernatant was applied to a SourceQ column (Amersham Biosciences), equilibrated with 50 mM Tris-HCl pH 8.0, 0.5 mM DTT, and eluted with a linear salt gradient from 0 to 1 M NaCl. Fractions containing RbcX were dialyzed against 20 mM imidazole pH 6.4, 20 mM NaCl, 0.5 mM DTT, applied to an equilibrated MonoQ column (Amersham Biosciences), and eluted with a linear salt gradient from 0 to 0.7 M NaCl. RbcX containing fractions were dialyzed against buffer A (50 mM Tris-HCl pH 8.0, 10 mM MgCl₂)/50 mM NaCl, and then applied to a Heparin Sepharose column (Amersham Biosciences). The RbcX protein, eluted in the flow through, was concentrated and subjected to Superdex 200 gel filtration chromatography in buffer A/50 mM NaCl. Fractions containing RbcX were concentrated using Amicon Ultra MWCO 10 kDa (Millipore) and supplemented with 10 % glycerol, flash-frozen in liquid nitrogen and stored at -80°C. All purification steps were performed at 4°C and protein concentration was determined spectrophotometrically at 280 nm.

### RbcS

RbcS was purified by a modified protocol (Somerville et al. (The cloning and expression in Escherichia coli of RuBP Carboxylase/Oxygenase large subunit genes; Academic Press, Inc. (1986)). E. coli BL21(DE3) cells, transformed with Syn6301-*rbcS*-pET11a or Syn7002-*rbcS_{FLAG}*-pET11a, were grown at 37°C and induced with 1 mM IPTG (3.5 h). The majority of RbcS was found in inclusion bodies. The cells were incubated for 1 h at 4°C in lysis buffer/0.25 M sucrose/5 % (v/v) Triton X-100. After freeze-thawing, ultrasonication and centrifugation, the pellet was resuspended and washed in 40 mM Tris-HCl pH 8.0, 0.25 M sucrose, 10 mM EDTA, 5 % (v/v) Triton X-100, 2 M urea. Washing of the pellet was repeated with 40 mM Tris-HCl pH 8.0, 0.25 M sucrose, 10 mM EDTA. Pellets were then finally dissolved in denaturation buffer (40 mM Tris-HCl pH 7.5, 6 M guanidine-HCl, 1 mM EDTA, 5 mM DTT). The denatured RbcS protein was refolded by dialysis against 50 mM Tris-HCl pH 8.0, 1 mM EDTA. The recovery of refolded protein was -65 to 85 %. Protein concentration was determined spectrophotometrically at 280 nm (19060 M⁻¹, monomer Syn6301-RbcS and 24410 M-¹, monomer Syn7002-RbcS_{FLAG}).

### Syn6301-RbcL₈

E. coli BL21(DE3) cells, transformed with Syn6301-*rbcL*-pET11a and pG-KJEB, were grown to mid-log phase at 30°C. Expression of DnaK/DnaJ/GrpE was induced with 0.4 % (w/v) arabinose=for 2 h, before cells were resuspended in fresh medium (w/o inducer) and expression of Syn6301-RbcL was induced with 1 mM IPTG for -3 h. Note that while GroEL/GroES overexpression enhanced production of Syn6301-RbcL₈S₈ (Goloubinoff et al. (Nature 337 (1989), 44-47)), purification of RbcL₈ was facilitated by the absence of high GroEL levels. Overexpression of DnaK/DnaJ/GrpE was found to maintain newly-synthesized RbcL soluble for subsequent interaction with GroEL at wildtype levels (Langer et al. (Nature 356 (1992), 683-689). Cells were lysed (see RbcX) and the lysate subjected to ion-exchange chromatography on a SourceQ column, equilibrated with buffer A/50 mM NaHCO₃/1 mM EDTA/0.5 mM DTT. Proteins were eluted with a linear NaCl gradient from 0 to 1 M. Fractions were analyzed for Syn6301-RbcL₈ by SDS- and Native-PAGE, followed by immunoblotting, and by carboxylation activity assay (after supplementation with Syn6301-RbcS). Fractions with highest activity and most enriched in Syn6301-RbcL₈ were pooled and dialyzed against 20 mM imidazole pH 6.5, 50 mM NaHCO₃, 10 mM MgCl₂, resulting in a white precipitate, which was pelleted and dissolved in 50 mM Tris-HCl pH 8.0.
After filtration through a 0.2 µm membrane and dialysis against buffer A/50 mM NaHCO₃, the protein solution was applied onto a MonoQ column and eluted with a linear salt gradient up to 0.7 M NaCl. The Syn6301-RbcL₈ containing fractions were concentrated and applied to a Superdex 200 gel filtration column in buffer A/50 mM NaHCO₃. Fractions containing Syn6301-RbcL₈ were pooled, adjusted to 10 % (v/v) glycerol and concentrated (Amicon MWCO 100 kDa). Complex concentration was determined spectrophotometrically at 280 nm using the extinction coefficient 553040 M⁻¹ (complex). Light scattering and Native-PAGE confirmed that the purified protein was assembled Syn6301-RbcL₈ complex, which showed carboxylation activity upon addition of RbcS.

### Syn6301-RbcL₈S₈

E. coli BL21(DE3) cells were transformed with Syn6301-*rbcLS*-pET11a and pBAD33ES/EL and grown to mid-log phase at 30°C, followed by induction of GroEL/GroES with 0.4 % (v/v) arabinose. After 1.5 h, cells were shifted to fresh medium containing 1 mM IPTG to induce expression of Syn6301-RbcLS for 3 h. Soluble lysate was fractionated on a DE52-column with a linear gradient of 20-1000 mM NaCl in 50 mM Tris-HCl pH 7.5, 20 mM NaCl, 1 mM DTT. Fractions were analyzed for Syn6301-RbcLS by SDS-PAGE and immunoblotting. The Syn6301-RbcLS pool was supplemented with (NH₄)₂SO₄ (20 % saturation) and applied to a Phenyl-Sepharose column (Amersham Biosciences), equilibrated with 50 mM Tris-HCl pH 7.5, 20 % saturation (NH₄)₂SO₄, 0.5 mM DTT. Elution was performed with a linear gradient of (NH₄)₂SO₄ from 20 % to 0 % saturation. Fractions containing Syn6301-RbcL₈S₈ were dialyzed against 20 mM imidazole pH 6.2. After ultracentrifugation, the supernatant was applied to a MonoQ ion-exchange chromatography column equilibrated with the same buffer and the protein eluted with a linear salt gradient from 0 to 0.7 M NaCl. Fractions containing Syn6301-RbcL₈S₈ were concentrated and applied to a Superose 6 gel filtration column (Amersham Biosciences) equilibrated in 20 mM Tris-HCl pH 7.5, 50 mM NaCl, 5 % (v/v) glycerol.

Complex concentration was determined spectrophotometrically at 280 nm using an extinction coefficient of 705520 M⁻¹ (complex). Purified Syn6301-RbcL₈S₈ was characterized by light scattering, Native- PAGE and carboxylation activity assay.

### Complex of Syn6301-RbcL and AnaCA-RbcX_{His6}

*E. coli* BL21(DE3) cells, transformed with pG-KJEB, Syn6301-*rbcL*-pET11a and AnaCA-*rbc*X*_{His6}*-pET28b, were grown to mid-log phase at 30°C. Expression of DnaK/DnaJ/GrpE and GroEL/GroES was induced with 0.4 % (w/v) arabinose and 20 ng/ml tetracycline for 2 h, before cells were resuspended in fresh medium (without inducer) and expression of Syn6301-RbcL and AnaCa-RbCX_{His6} was induced with 1 mM IPTG for - 3 h. Cells were lysed in lysis buffer/500 mM NaCl/10 mM imidazole by freeze-thawing and ultrasonicated. The soluble lysate was applied to a Ni-NTA-agarose (Sigma) column, equilibrated with 20 mM Tris-HCl pH 7.5, 500 mM NaCl, 10 mM imidazole. After successive washing steps with 10 mM, 50 mM and 100 mM imidazole the complex of Syn6301-RbcL/AnaCA-RbcX_{His6} and free dimers of AnaCA-RbcX_{His6} were eluted with 250 mM imidazole. This protein pool was dialyzed against 20 mM Tris-HCl pH 9.0, 50 mM NaHCO₃ 10 mM MgCl₂ and applied to a MonoQ ion-exchange chromatography column equilibrated with the same buffer and developed with a linear salt gradient from 0 to 0.7 M NaCl. Fractions containing the complex were concentrated (MWCO 10 kDa) and applied to a Superdex 200 gel filtration column equilibrated with 20 mM Tris-HCl pH 9. The initially eluting peak contained Syn6301-RbcL/AnaCA-RbcX_{His6} complex as verified by Native-PAGE.

### Antisera and Antibodies

Antisera against purified Syn7002-RbcX and E. coli GroEL were produced in rabbits. Antibody against a conserved peptide of RbcL was purchased from AgriSera (Vännäs, Sweden) and mouse monoclonal anti-FLAG M2 antibody as well as secondary HRP-conjugated antibodies were from Sigma-Aldrich (Steinheim, Germany).

### Carboxylation Assay

Carboxylation activity of Rubisco was determined by diluting aliquots of E. coli lysate or in vitro translation reactions into buffer containing 100 mM Tris-HCl pH 7.5, 10 mM KCI, 2 mM Mg(OAc)₂, 1 mM DTT and 2 µM BSA. Samples containing only RbcL were supplemented with ~7 µM purified Syn7002-RbcS or Syn6301-RbcS and assembly allowed to proceed for 5 min at room temperature. Thereafter, a ¹⁴C-Mix was added to give final concentrations of 60 mM NaHCO₃ 0.5 µCi NaH¹⁴CO₃ and 10 mM MgCl₂ and incubation continued for a further 5 min. Carboxylation was initiated by the addition of 2.5 mM ribulose-1,5-bisphosphate (RuBP) and reactions stopped by addition of acetic acid (3 N) after 30 min. The amount of carbon fixed was quantified as previously described (Goloubinoff et al. (Nature 337 (1989), 44-47)).

### Field Flow Fractionation - Multiangle Light Scattering (FFF-MALS)

Protein complexes (80 µg) were analyzed by Field Flow Fractionation (FFF) using a 490 nm spacer and 30 kDa MWCO membrane (Wyatt Technology, Santa Barbara, CA) with elution and cross-flow of 1 ml/min (Roessner and Kulicke (J. Chromatography A. 687 (1994), 249-258)). The FFF was online with DAWN EOS multi-angle light scattering (Wyatt Technology, 690 nm laser), variable wavelength UV absorbance set at 280 nm (Agilent 1100 series) and Optilab DSP refractive index (Wyatt Technology, 690 nm) detectors (Wyatt (Anal. Chim. Acta 272 (1993), 1-40)). Masses were calculated using the ASTRA software (Wyatt Technology) with a value set to for dn/dc for protein of 0.185 ml/g.

### Crystallography and Structure Analysis

### Incorporation of SeMet and Proteolysis of Syn7002-RbcX

SeMet incorporation into Syn7002-RbcX followed an established protocol (Van Duyne et al. (J. Mol. Biol. 229 (1993), 105-124)). For purification of (SeMet)-Syn7002-RbcX, all buffers contained 1 mM DTT. (SeMet)-Syn7002-RbcX(ΔC25) was produced by incubating 1 mg/ml (SeMet)-Syn7002-RbcX with 25 µg/ml subtilisin for 2 h in ice-cold 20 mM HEPES-NaOH, pH 7.5, 50 mM NaCl. Protease action was terminated by the addition of 10 mM PMSF. Truncated Syn7002-RbcX was identified as Syn7002-RbcX(ΔC25) by Edman degradation and ESI-MS.

### Crystallization

Prior to crystallization, RbcX wildtype and mutant proteins were transferred into 10 mM Tris-HCl, pH 7.5. Crystals were grown using the hanging drop vapor diffusion method at 20°C by mixing 1 µl protein sample at -28 mg/ml and 1 µl reservoir solution. Initial rod-shaped crystals were obtained overnight from Crystal Screen I conditions 7 and 29 (Hampton Research, USA). The final optimized crystallization solution contained 0.1 M HEPES-NaOH pH 7.5 and 1.3-1.55 M sodium acetate. Substantially higher concentrations of sodium acetate (1.5-3.0 M) were required for crystallization of RbcX mutant proteins. For cryo-protection, the crystals were transferred stepwise into mother liquor containing 30 % (v/v) glycerol.

### Syn7002-RbcX/Syn7002-RbcL peptide

Crystals of Syn7002-RbcX were incubated for 1 h in buffer (0.1 M HEPES-NaOH pH 7.5, 1.4 M sodium acetate) containing 1 mM Syn7002-RbcL peptide, EIKFEFD. For cryo-protection, the crystals were transferred stepwise into mother liquor containing 1 mM peptide and 20 % (v/v) glycerol.
For co-crystallization trials, a mixture of Syn7002-RbcX (in 10 mM Tris-HCl pH 7.5) and Syn7002-RbcL peptide (EIKFEFD (in H₂O) at a molar ratio of 1:1.5 (467 µM RbcX-dimer, 700.5 µM peptide) was used and crystallization performed as above. Well-diffracting RbcX crystals of space group *I222* were obtained from PEG/Ion Screen condition 14 (0.2 M potassium thiocyanate, 20 % (w/v) polyethylene glycol 3350) (Hampton Research, USA). These crystals apparently contained no bound peptide.

### (SeMet)-Syn7002-RbcX(ΔC25)

(SeMet)-Syn7002-RbcX(ΔC25) was used at 2.8 mg/ml in crystallization trials. Small rhombic crystals grew overnight in Crystal Screen I condition 22 (Hampton Research, USA). Prior to cryo-cooling, crystals were incubated in cryo-protectant buffer for 10 min (0.085 M Tris-HCl pH 8.5, 0.17 M sodium acetate, 25.5 % (w/v) PEG 4000, 15 % (v/v) glycerol).

### AnaCA-RbcX

Crystals of AnaCA-RbcX_{His6} were produced in crystallization trials of the complex of Syn6301-RbcL/AnaCA-RbcX_{His6} using a Syn6301-RbcL/AnaCA-RbcX_{His6} protein sample at 8.3 mg/ml. Rod-shaped crystals of AnaCA-RbcX_{His6} were obtained together with a massive precipitate presumably containing mainly Syn6301-RbcL after several days at 20°C from Crystal Screen I condition 11 (Hampton Research, USA). Crystals were cryo-protected by stepwise transfer into mother liquor containing 35 % (v/v) glycerol.

### Structure Determination

Diffraction data were collected at beamlines BM14 and ID14-4 at the European Synchrotron Radiation Facility (ESRF) in Grenoble, France, and at beamline X10SA-PX-II at the Swiss Synchrotron Light Source (SLS) in Villigen, Switzerland. Diffraction data were integrated with Mosflm (Leslie (Joint CCP4 and ESF-EACBM Newsletter 26 (1992))) and Scala (Evans (Joint CCP4 and ESF-EACBM Newsletter 33 (1997), 22-24)).
The structure of Syn7002-RbcX was solved by SIRAS using crystals incubated with 0.2 mM K₂(PtCl₄) in mother liquor for 22 h. Six Platinum sites were found by Patterson methods using Solve (Terwilliger and Berendzen (Acta. Crystallogr. D. Biol. Crystallogr. 55 (Pt4) (1999), 849-861)). Density modification was carried out with Resolve (Terwilliger (Acta. Cystallogr. D. Biol. Crystallogr. 56 (Pt8) (2000), 965-972)). The resulting map was readily interpretable, and continuous backbone density for all six RbcX chains between residues 4 and 109 could be traced with O (Jones et al. (Acta Crystallogr. A 47 (1991), 110-119)). Subsequent iterative model improvement and refinement were performed with O and Refmac5 (Murshudov et al. (Acta Crystallogr. D Biol. Crystallogr. 53 (1997), 240-255)).

The structure of SeMet-labeled Syn7002-RbcX(ΔC25) was solved initially by molecular replacement (MR) using a dimer of RbcX as a search model employing the program Molrep (Vagin and Isupov (Acta. Crystallogr. D Biol. Crystallogr. 57 (2001), 1451-1456)). The MR-model phases were used to calculate a map using the anomalous differences from the Se K-edge peak dataset, in which 29 Se-sites were located. SHARP was used for refinement of the Se positions, yielding 8 additional Se-sites, and for phase calculation (de la Fortelle and Bricogne (Methods Enzymol. 276 (1997), 472-494)). Density modification was performed with Resolve (Terwilliger, *loc. cit.*)*.* The resulting phases were used as restraints during initial model-building and refinement cycles using O and Refmac.
The structure of AnaCA-RbcX was solved by molecular replacement using Molrep with a monomer of Syn7002-RbcX as search model. Model bias was reduced by automated model building with ArpWarp6.1 (Perrakis et al. (Nat. Struct. Biol. 6 (1999), 458-463)). The ArpWarp-model was completed manually using O and refined with Refmac.

The high resolution crystal form of Syn7002-RbcX was solved by molecular replacement using Molrep (Vagin and Isupov, *loc. cit.*)*.* This structure and the RbcX mutant structures were edited with Coot (Emsley and Cowtan (Acta. Crystallogr. Sect D-Biol. Crystallogr. 60 (2004), 2126-2132)) and refined with Refmac.
In all structures, residues facing solvent channels without detectable side chain density were modeled as alanines. All models obey good stereochemistry with no Ramachandran outliers as judged by the program Procheck (Laskowski et al. (J. Appl. Cryst. 26 (1993), 283-291)). Coordinates were aligned with Lsqman (Kleywegt and Jones (CCP4/ESF-EACBM Newsletters on Protein Crystallogr. 31 (1994), 9-14)). The similarity score was calculated with ESPript using the Rissler scoring matrix (Gouet et al. (Bioinformatics 15 (1999), 305-308)). Figures were generated with the programs Molscript *(*Kraulis (J. Appl. Crystallogr. 24 (1991), 946-950)), Bobscript (Esnouf (J. Mol. Graph. Model 15 (1997), 132-134, 112-113)), Raster-3D (Merrit and Bacon (Methods Enzymol. 276 (1997), 505-524)), and Pymol (DeLano, W.L. The PyMOL Molecular Graphics System (2002) on World Wide Web (http://www.pymol.org).

### Peptide Binding Assay

Binding of RbcX_{FLAG} to an array of dodecapeptides covering the sequence of Syn7002-RbcL and isothermal calorimetry were performed essentially as described (Rüdiger et al. (Embo J. 16 (1997), 1501-1507); Wiseman et al. (Anal. Biochem. 179 (1989), 131-137)). In particular, an array of dodecapeptides (N-terminally acetylated) covering the sequence of Syn7002-RbcL with an overlap of 10 amino acid residues were synthesized. Peptides were covalently bound at the C-terminus to a cellulose-PEG-membrane (JPT Peptide Technologies GmbH, Berlin, Germany). The 231 peptide spots were of 0.37 cm x 0.37 cm in size and carried approximately 5 nmol of peptide each. The peptide membrane was equilibrated in TBS buffer (50 mM Tris-HCl, pH 8, 137 mM NaCl, 2.7 mM KCI) containing 1 % (w/v) milk powder for 3 h at room temperature, followed by overnight incubation with 10 µg/ml RbcX in TBS/1 % (w/v) milk powder at room temperature with gentle shaking. The membrane was then washed three times for -5 min each in TBST buffer (20 mM Tris/HCl pH 7.5, 137 mM NaCl, 0.1 % (v/v) Tween 20). Incubation with anti-RbcX antibody was followed by horseradish peroxidase-labeled secondary antibodies in TBST blocking solution. RbcX binding was visualized using chemiluminescence detection substrates (Amersham). The membrane was regenerated according to the manufacturer's instructions.

### Isothermal Titration Calorimetry (ITC)

Calorimetric measurements were performed using a MicroCal VP-ITC MicroCalorimeter (MicroCal, Inc., Northhampton, MA, USA) essentially as described (Wiseman et al. (Anal. Biochem. 179 (1989), 131-137)). The reference cell was filled with water. Syn7002-RbcX (327 µM dimer) or AnaCA-RbcX (323 µM dimer) in buffer A/50 mM NaCl were titrated at 22°C with 2 mM of Syn7002-RbcL-peptides (peptide 1: EIKFEFD, peptide 2: KEIKFEFDTVD) dissolved in buffer A/50 mM NaCl. 36 identical aliquots (8 µl) of peptide solution were injected at intervals of 3 min from a syringe into the sample cell containing either buffer or solution of RbcX (1.345 ml) with stirring and reference power set to 30 µCal/s at 22°C. Data analysis by peak integration and curve fitting by least-squares deconvolution was performed using the instrumental MicroCal Origin software to give the binding constant (K), the enthalpy change (ΔH), the entropy (ΔS) of the reaction and the stoichiometry (N) of binding.

### In Vivo Assembly of Syn7002-RbcL or Syn7002-RbcLS in E. coli

Expression of RbcL, RbcL/X, RbcL/S or RbcL/X/S from the respective pET-vectors was induced by IPTG in E. coli BL21(DE3) cells grown to mid-log phase (1 mM IPTG, -3.5 h at 30°C) with or without prior transient overexpression of GroEL and GroES from a tetracycline-inducible promoter of the plasmid pG-*KJE8* (20 ng/ml tetracycline, 2 h at 30°C). Equivalent amounts of cells were lysed by ultrasonication and fractioned into soluble and insoluble fractions by centrifugation (20 800 x g, 30 min at 4°C). Soluble lysate fractions were analyzed for assembled RbcL by 6 % Native-PAGE, followed by immunoblotting with RbcL-specific antibody (AgriSera). Rubisco carboxylation activity in the soluble lysate fractions was measured essentially as described (Goloubinoff et al. (Nature 337 (1989), 44-47); see above).

### In Vitro Translation of Rubisco

Translations were carried out in the coupled RTS100 HY E. coli transcription/translation system (Roche) from T7-promoter driven plasmids in the presence of ³⁵S-Met or unlabeled methionine (Agashe et al. (Cell 117 (2004), 199-209)). Translation reactions were run for 1.5 h at 30°C unless stated otherwise and stopped with chloramphenicol (CAM; 200 µg/ml, 2 min on ice). Purified proteins were added to the translation lysate at the concentrations indicated in figure legends. Post-translational addition of proteins was performed after CAM addition, followed by transfer of reactions to 30°C for further incubation as indicated. Reactions were separated into soluble and insoluble fractions by centrifugation and analyzed on 12.5 % SDS-PAGE or 6% Native-PAGE, followed by autoradiography.
When indicated, GroEL was immunodepleted from the translation mix by incubation with polyclonal GroEL antibody bound to Protein A sepharose beads (Amersham) by gentle shaking for 45 min at 4°C and removal of beads by centrifugation. Efficient depletion of GroEL was confirmed by immunoblotting with GroEL antibody.
In pulse chase experiments translation of Syn7002-RbcL was carried out in the presence of GroEL/GroES (0.5/1.0 µM) and purified RbcX (40 µM) for 6 min at 30°C before addition of ³⁵S-Met for 6 min. The chase was started by addition of 3 mM unlabeled methionine. Aliquots were collected at the time points indicated in the figure legend. Soluble protein was analyzed by discontinuous Bis-Tris Native-PAGE (Hansen et al. (J. Cell Biol. 145 (1999), 265-277)), followed by autoradiography.

### Immunoprecipitation and gel filtration

RbcX_{FLAG} was immunoprecipitated from soluble cell lysates containing coexpressed RbcL and RbcX with anti-FLAG M2 Affinity Gel Beads (Sigma) by overnight incubation at 4°C. When indicated, bound protein was eluted under native conditions by incubating the beads for 30 min on ice with 100 µl of 3-times FLAG peptide solution (Sigma) (300 µg/ml). Cell lysates or FLAG-eluted immunoprecipitates were analyzed on a Superdex 200 column in 50 mM Tris-HCl pH 8.0, 50 mM NaCl, 5 mM MgCl₂. 250 µl fractions were TCA precipitated and analyzed by immunoblotting.

### EXAMPLE 1

### Sequential Action of Chaperonin and RbcX in Assembly of Syn7002 Rubisco

The chaperone requirement for the folding and assembly of cyanobacterial form I Rubisco from Synechococcus sp. PCC7002 (Syn7002) was assessed upon expression of Rubisco subunits in E. coli cells with normal and elevated chaperonin levels. Amounts of soluble protein were determined upon fractionation of cell extracts and immunoblotting. Formation of RbcL₈S₈ holoenzyme (Figure 1A) or RbcL₈ complexes (Figure 1 B) was monitored by Native-PAGE and Rubisco activity assay.

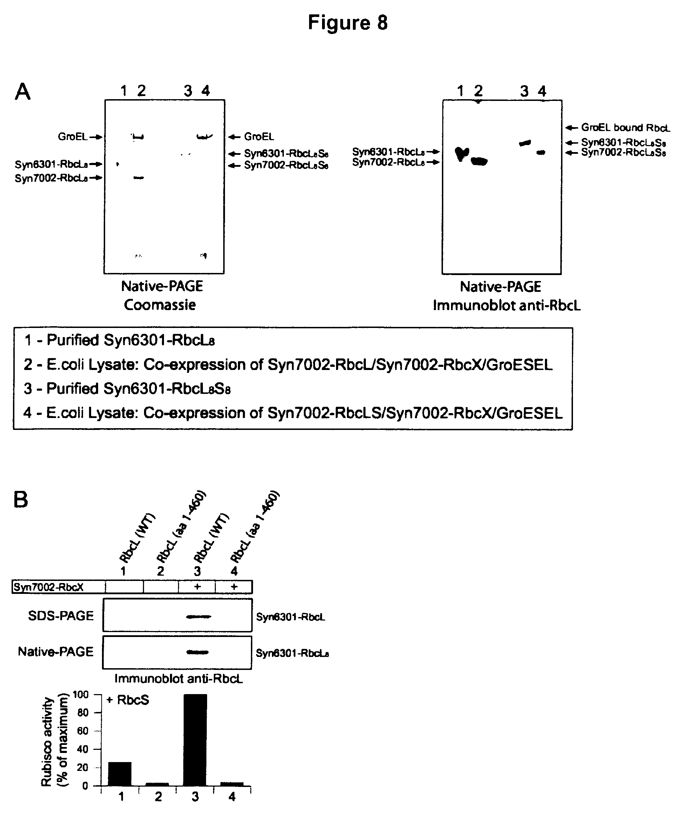

In wildtype cells, expression of Syn7002-RbcL from an IPTG-inducible plasmid did not result in soluble protein, irrespective of whether RbcS was coexpressed (Figure1A and B, lane 1). Transient (~10-fold) overexpression of GroEL/GroES prior to induction of *rbcLS* or *rbcL* also failed to produce significant amounts of soluble RbcL (Figure 1A and B, lane 2). Assembled Rubisco and carboxylation activity were essentially absent (Figure 1A and B, lanes 1-2). In contrast, expression of the homologous Rubisco from *Synechococcus* sp. PCC6301 (Syn6301) resulted in the formation of substantial amounts of RbcL₈S₈ or RbcL₈ complexes which migrated as high molecular weight bands on Native-PAGE (Figure 8A) (Goloubinoff et al. (Nature 37 (1989), 44-47)).
In Syn7002, the RbcL and RbcS subunit genes are located in an operon that also encodes the protein RbcX in the order *rbcL-rbcX-rbcS.* Indeed, the assembly of Syn7002-RbcL₈S₈ proved to be critically dependent on coexpression of RbcLS with RbcX (Figure 1A, lane 3). Importantly, when Syn7002-RbcL was expressed in the absence of RbcS, RbcX was required for the formation of RbcL₈ core complexes competent for assembly to active enzyme with RbcS (Figure 1B, lane 3). Thus, RbcX appears to be required predominantly or exclusively for RbcL₈ formation. Overexpression of GroEL/GroES in the presence of RbcX caused a 3 to 4-fold increase in Syn7002 Rubisco activity, correlating with an increased amount of RbcL₈S₈ or RbcL₈ detected by Native-PAGE (Figure 1A and B, lane 4). In the case of Rubisco from Syn6301, where *rbcX* is encoded outside the *rbcLS* operon, assembly of RbcL₈ was enhanced 4 to 5-fold by RbcX coexpression (Figure 8B).
The positive effect of GroEL/GroES and RbcX for efficient assembly was reproduced upon in vitro synthesis of Syn7002-RbcL in an E. coli translation lysate (Agashe et al. (Cell 117 (2004), 199-209)) (Figure 1C, lanes 1 and 2). Supplementing GroEL/GroES, but not GroES alone, together with RbcX produced mostly soluble RbcL and greatly increased the formation of RbcL₈ core complexes (Figure 1C, lanes 3 and 4). Rubisco activity was detected upon addition of purified RbcS (data not shown). RbcL translation in GroEL-immunodepleted lysate did not produce RbcL₈, even when RbcX was present, demonstrating the requirement of both factors (Figure 1C, lanes 5-7). Importantly, re-addition of GroEL after inhibition of RbcL translation with chloramphenicol (CAM) failed to produce RbcL₈, despite the presence of RbcX during translation (Figure 1C, lane 8). Note that some RbcL was bound to GroEL under these conditions, but this protein was not productive for folding. Thus, RbcX cannot stabilize RbcL for subsequent productive interaction with GroEL, suggesting that GroEL must interact with RbcL immediately upon synthesis. Indeed, while the production of RbcL₈ was most efficient when the lysate was supplemented with both GroEL/GroES and RbcX during translation (Figure 1D, lane 1), addition of RbcX immediately after blocking translation still mediated RbcL₈ formation, albeit at a reduced level (Figure 1D, lane 2). Delayed post-translational addition of RbcX resulted in the rapid loss of RbcL assembly and in the formation of insoluble RbcL (Figure 1D and data not shown). Together these experiments indicate that RbcX has a specific chaperone function in preventing RbcL misassembly downstream of chaperonin.

### EXAMPLE 2

### Crystal Structure of RbcX

To understand the mechanism of RbcX action in Rubisco assembly, a structural analysis of Syn7002-RbcX by X-ray crystallography was undertaken. RbcX was expressed in E. coli as a soluble protein of 15 kDa on SDS-PAGE. Analysis by multiangle light scattering indicated that RbcX forms a dimer of 30.2 kDa (Figure 9). RbcX readily crystallized in space group P4₁2₁2 with three dimers in the asymmetric unit, and the structure was solved by Pt-SIRAS at a resolution of 2.8 Å (crystallographic data are summarized in Table 1). Residues 115 to 134 were not resolved in any of the RbcX peptide chains. Indeed, limited proteolysis of RbcX resulted in the removal of the poorly conserved 25 C-terminal residues (Figure 10), leaving a stable core domain of residues 1-109 (RbcX(ΔC25)) which was active in Rubisco assembly (Figure 11). Also the structure of SeMet-labeled RbcX(ΔC25) containing 6 RbcX dimers per asymmetric unit was solved (Table 1). The average r.m.s. deviation for Cα positions between RbcX dimers in the crystal lattices was 0.623 Å, indicating that the core domain structure is rather rigid (Figure 12).
RbcX consists of four α-helices per monomer (α1-4) that form an unusual helix bundle (Figure 2A). α2 (residues 35-48) turns backwards relative to α1 (residues 4-33) with a steep inter-helical angle of 151° and only one residue (N34) forming the turn. A very similar arrangement is observed at the junction between α3 (residues 52-63) and α4 (residues 65-107) (inter-helix angle 161°). The short helices α2 and α3 are connected by a 5 residue linker. The core of the helical bundle is composed of conserved hydrophobic residues (Figure 10) without authentic coiled-coil side-chain packing. α4 makes a ~60° kink in the vicinity of residue 84 and forms a 35 Å long extension (α4C) pointing away from the helix bundle (Figure 2A). The RbcX dimer has overall dimensions of ~ 64 x 33 x 31 Å (length x height x width). The long α4 helices of the protomers align in an almost anti-parallel fashion, such that the helical bundles are located at opposite ends (Figure 2B). The α1 helices form additional symmetrical contacts and together with the α2 helices delineate a narrow diagonal groove in the arc-shaped complex. The dimer interface is predominantly uncharged and hydrophobic. A polar network around the conserved residue N98, contacting the amide backbone at position L72, and the sidechains of R75 and E76 in the opposing protomer, contributes to dimer stability. The α2-α3 helical hairpins make no direct inter-chain contacts. Helix α2 is the most mobile element of the structure (Figure 12).
Crystallographic analysis of the RbcX from the cyanobacterium Anabaena sp. CA (AnaCA) revealed a structure virtually identical to that of Syn7002-RbcX (r.m.s.d. 1.14 Å over 103 Cα atoms; see Table 1 and Figure 13). Consistent with their high degree of sequence conservation (Figure 10), this suggests that all cyanobacterial RbcX homologs have the same overall architecture. The RbcX homologs identified in plants are more distant, but share the register and the structural residues in the globular helix bundle of cyanobacterial RbcX (Figure 10).

### EXAMPLE 3

### Identification of Protein Interaction Surfaces on RbcX

Two highly conserved regions, representing potential protein-protein interaction sites, were identified on RbcX, the central groove of the dimer and a surface region around the corners of the molecule (Figure 2C). A hydrophobic area comprising the conserved residues Y17, Y20 and 150 of each monomer lines the groove in the center of the molecule (Figure 2C). Y17 packs against T13, which is frequently replaced in cyanobacterial RbcX sequences by an isosteric valine (Figure 10). Central access into the crevice is constricted to a 5.4 Å wide opening by the sidechains of the highly conserved Q51 residues, just wide enough to accommodate a polypeptide chain in an extended conformation. The other conserved region has predominantly polar character and is located at the corners of the RbcX dimer, comprising residues Q29, E32, T33, N34, R70, E76 and R102, R103, L106, E107, R108, respectively (Figure 2C). It is formed by helices 4 of both protomers and by the turn region between the α1 and α2 helices of one subunit, and may serve to interact with a polar protein surface. Because of the two-fold symmetry, this region occurs twice at opposing edges of the dimer.
The possible functional significance of these conserved regions of RbcX was investigated by mutational analysis (Figure 3A). Several single and double mutations predicted to compromise dimer formation failed to produce soluble RbcX and hence did not support the expression of soluble RbcL (Figure 3A, asterisks; Figure 14). All other RbcX mutant proteins were soluble and presumably had native-like dimer structure (Figure 14). A large set of mutations were of little or no effect on RbcL solubility and assembly (Figure 3A, circles). Individual alanine substitution of the central groove residues Y17 and Y20 reduced RbcL solubility and formation of RbcL₈ complexes (Figure 3A, lanes 6 and 7), while mutation of the adjacent residue T13 was without measurable effect (Figure 3A, lane 3). In contrast, the double mutation Y17A/Y20L abolished the formation of soluble RbcL completely (Figure 3A, lane 9). Since the crystal structure of this mutant protein was virtually identical to that of wildtype RbcX (Table 1 and data not shown), this indicates that the central groove of RbcX has an essential function. Alanine substitution of the conserved residue Q51, which forms the upper rim of the central channel, was without detectable effect on RbcX function (Figure 3A, lane 16). Interestingly, several other individual mutations (Q29A, E32A, R70A, R102A) allowed the accumulation of soluble RbcL but did not support assembly to RbcL₈ (Figure 3A, triangles). Notably, all of these mapped to the polar ends of the RbcX dimer, forming symmetrical, contiguous surfaces (Figure 2C).
The soluble RbcL produced upon coexpression with wildtype or mutant RbcX was analyzed by size exclusion chromatography. Coexpression with N-terminally FLAG-tagged wildtype RbcX, which was fully active, resulted mainly in RbcL that fractionated at ~400 kDa, similar to a Syn6301-RbcL₈ standard (Figure 3B(i)). A smaller amount of RbcL fractionated at a high molecular weight in excess of 800 kDa. This protein was not bound to GroEL and apparently represented misassembled, aggregated RbcL. No monomers of RbcL were detected, suggesting a high cooperativity of assembly. The RbcX mutant Y17A, partially impairing the function of the central groove, resulted in a reduced amount of RbcL₈ complex (Figure 3B(ii)). Interestingly, mutation Q29A, disrupting the peripheral polar surface of RbcX, supported exclusively the production of misassembled, high molecular weight RbcL (Figure 3B(iii)), confirming the absence of RbcL₈ complex upon analysis by Native-PAGE (Figure 3A, lane 10). Notably, RbcX did not detectably cofractionate with RbcL₈ (data not shown), suggesting either that the RbcL-RbcX interaction is labile on gel filtration or RbcX binds to RbcL only transiently during RbcL₈ assembly. Co-immunoprecipitation from cell extracts with anti-FLAG antibody supported the former possibility. A substantial amount of RbcL was co-immunoprecipitated with RbcX_{FLAG} but not with non-tagged RbcX (data not shown). Following dissociation from the antibody by incubation with excess FLAG-peptide, the RbcL protein fractionated as the RbcL₈ complex (Figure 3C(i)). Only a small amount of RbcL₈ co-immunoprecipitated with the mutant RbcX(Y17A) and no association was detectable with mutant RbcX(Q29A) (Figure 3C(ii) and (iii)), indicating that these mutations weakened the interaction of RbcX with RbcL, resulting in RbcL misassembly and aggregation.
In summary, these experiments identified two independent, functionally critical regions on RbcX, the central hydrophobic crevice and a peripheral polar surface of the molecule. Whereas the central crevice is essential for the production of soluble RbcL subunits, disruption of the polar surface results in the production of soluble but misassembled RbcL, suggesting a role in proper subunit arrangement.

### EXAMPLE 4

### RbcX Binds the Conserved C-terminal Peptide of RbcL

The structural features of the central groove region of RbcX seemed suitable for binding an extended peptide. To identify such a sequence element(s) in RbcL, an array of 12 amino acid long, acetylated peptides covering the entire sequence of Syn7002-RbcL with a 10 residue overlap (231 peptides in total) were C-terminally attached to a cellulose-PEG membrane. Incubation with Syn7002-RbcX, followed by detection with anti-RbcX antibody, resulted in a strong binding signal with four RbcL peptides sharing the sequence EIKFEFD, close to the C-terminus of RbcL (Figure 4A and B). Other peptides exhibiting weaker binding had no apparent consensus motif. Essentially the same binding pattern was observed with the RbcX polar surface mutant Q29A, while the groove mutant Y17A/Y20L caused the complete loss of RbcX binding to the C-terminal RbcL peptide (Figure 4A). Specific binding to the C-terminal EIKFEFD motif was also detected with RbcX from Anabaena sp. CA (Figure 4A), suggesting that this interaction is a general property of cyanobacterial RbcX homologs. In the Rubisco holoenzyme, the C-terminal sequence of RbcL is located at the surface of the complex and has been implicated in regulating catalysis (Zhu et al. (Photosynth. Res. 57 (1998), 71-79)). It is highly conserved among all form I Rubisco RbcL homologs (Figure 4B), including the proteins from higher plants, but is not found in archaeal and form II Rubisco (Figure 15A), along with the absence of RbcX in these organisms.
To obtain structural information on RbcL peptide binding, crystals of Syn7002-RbcX were soaked with the peptide EIKFEFD. One of the three crystallographically independent dimers in the native crystal form of RbcX exhibited clear difference density for a bound peptide at the central recess (Figure15B and Table 1). The difference density indicates that the peptide is bound asymmetrically despite the dyad symmetry of RbcX, probably as a result of the asymmetric environment in the crystal lattice. The peptide adopts an extended conformation with the side chains F462 and F464 extending into cavities lined by the sidechains of T13, Y17, Y20, 150, and Q51 at the center of the RbcX cleft; the polar sidechains of the peptide face outwards and are mostly disordered (Figure 4C and D). 1460 of the peptide contacts Y20, R24, and S45 of one RbcX chain. In addition, three hydrogen bonds are formed between the peptide backbone and the side chains of Y20, R24, and Q51. The conformations of the two Q51 side chains appear to be mainly governed by van-der-Waals contacts to the phenyl rings of peptide residues F462 and F464 (Figure 4D).
Binding analysis by isothermal titration calorimetry (Figure 16) revealed a relatively low affinity of Syn7002-RbcX for peptide EIKFEFD (K_{D} ∼230 µM), comprising the core binding region. A somewhat higher affinity (K_{D} ∼160 µM) was measured with the longer peptide KEIKFEFDTVD, which includes the conserved residues lysine 458 and aspartate 468. These measurements indicate that the interaction of Syn7002-RbcX with the RbcL C-terminus is highly dynamic. Interestingly, substantially higher affinities for these peptides were measured with AnaCA-RbcX (K_{D} ∼60 µM and 5 µM, respectively). In AnaCA-RbcX, the loop region between helices α2 and α3 is shorter by one residue and contains a lysine at position 49, thus introducing a positive charge in the proximity of the binding groove (Figure 13B and C). The positioning of this lysine may result in an increased affinity for the negatively charged C-terminus of RbcL.
Next, RbcL C-terminal mutants were analyzed to assess the significance of the RbcL C-terminus for assembly. As shown above, wildtype RbcL produced soluble RbcL₈ complex competent for association with RbcS in a manner dependent on Syn7002-RbcX (Figure 5A, lanes 1 and 6). Immunoblotting of the RbcL₈ complex detected on Native-PAGE did not reveal the presence of RbcX (data not shown), consistent with the labile nature of the RbcL₈-RbcX interaction, while immunoprecipitation of RbcX_{FLAG} from cell extracts co-immunoprecipitated a substantial amount of RbcL (Figure 5B). In contrast, expression of RbcL(1-459), lacking the C-terminal recognition sequence for RbcX, did not result in soluble protein, despite the presence of RbcX (Figure 5A, lane 7). Individual or combined mutation of phenylalanines 462 and 464 confirmed the critical nature of these residues. Whereas RbcL(F462A) and RbcL(F462A/F464A) prevented the formation of RbcL₈ (Figure 5A, lane 8 and 10), RbcL(F464A) still allowed the production of a reduced amount of soluble and assembled protein (Figure 5A, lane 9). Thus, F462 appears to be more important in anchoring the RbcL C-terminus to RbcX. Notably, deletion of the C-terminal peptide from Syn6301-RbcL abolished the RbcX-independent assembly of this protein, suggesting a direct role of the C-terminus in RbcL₈ assembly or stability beyond serving as a RbcX docking site (Figure 8B).

### EXAMPLE 5

### Dynamic Interaction with RbcL is Critical for RbcX-Mediated Assembly

The difference in affinities for the RbcL C-terminus observed with Syn7002-RbcX and AnaCA-RbcX (Figure 16) allowed to determine the functional relevance of the dynamic nature of this interaction. Surprisingly, AnaCA-RbcX, while supporting the expression of soluble Syn7002-RbcL, failed to promote the proper formation of assembly-competent RbcL₈ complexes (Figure 5A, lane 11). Instead, a high molecular weight form of RbcL (RbcL-X) was detected which was tightly associated with AnaCA-RbcX by co-immunoprecipitation (Figure 5B and data not shown). A similar high molecular weight complex was observed upon binding of AnaCA-RbcX to preformed Syn6301 RbcL₈ (Figure 17). Based on static light scattering, the mass of this complex was -730 kDa, consistent with 8-9 RbcX dimers bound per RbcL₈ (Figure 9B). Formation of this non-productive complex was not observed with RbcL(1-459) or the RbcL(F462A/F464A) double mutant (Figure 5A, lanes 12 and 15). Interestingly, a substantial amount of assembly-competent RbcL₈ core complex formed upon coexpression of AnaCA-RbcX with the single phenylalanine mutations RbcL(F464A) and RbcL(F462A) (Figure 5A, lanes 13 and 14). Thus, attenuating the affinity of the heterologous AnaCA-RbcX for the RbcL C-terminus restored productive assembly. As revealed by co-immunoprecipitation, RbcL(F464A) interacted more strongly with AnaCA-RbcX than with RbcL(F462A), confirming the dominant role of F462 (Figure 5B).
Next, the exchangeability of RbcX-bound RbcL was analyzed. To this end, RbcL was coexpressed with either FLAG-tagged Syn7002-RbcX or AnaCA-RbcX and the respective complexes immunoprecipitated. Bound RbcL was detected by immunoblotting after incubation with increasing concentrations of non-tagged Syn7002-RbcX. Half-maximal displacement of RbcL was observed with -1 µM of RbcX, indicating that the peripheral surface of RbcX contributes to binding but alone is probably of relatively low affinity, similar to the central peptide cleft. In contrast, RbcL was not displaced from the complex with AnaCA-RbcX_{FLAG} (Figure 5C), consistent with the 30-fold higher affinity of AnaCA-RbcX for the RbcL C-terminus. In an analogous experiment, the displacement reaction was performed with RbcS. RbcL was readily displaced from the complex with Syn7002-RbcX at submicromolar RbcS concentrations but not from the complex with AnaCA-RbcX (Figure 5D), explaining the non-productive nature of the latter. These results support the conclusion that complex formation between RbcL and RbcX must be dynamic in order to support holoenzyme formation. RbcS normally blocks RbcX re-binding, perhaps by stabilizing the RbcL C-terminus in a conformation inaccessible to RbcX or by a steric overlap with the region of RbcL that is recognized by the peripheral RbcX site.

### EXAMPLE 6

### RbcX Acts Early in RbcL Assembly

RbcL₈ assembly is thought to involve the formation of dimers which then associate to tetramers (Hubbs and Roy (J. Biol. Chem. 268 (1993), 13519-13525)). At which stage does RbcX begin to interact in this reaction? To address this question, RbcL was translated in vitro in E. coli lysate supplemented with GroEL/GroES and RbcX_{FLAG}. Translation was first performed for 6 min in the absence of labeled amino acid, to ensure linear RbcL production, followed by a 6 min pulse with ³⁵S-methionine and a chase with excess unlabeled methionine for up to 160 min. Aliquots of the reaction were withdrawn at different times, stopped on ice and then analyzed by a discontinuous Bis-Tris Native-PAGE system in which protein migration correlates well with molecular mass. In the absence of RbcX, GroEL-bound RbcL (-800 kDa) was visible at the beginning of the chase, followed by the appearance of a fuzzy low molecular weight band that migrated similarly to a monomeric mutant form of bacterial Rubisco from R. rubrum (50 kDa) (Figure 6A). This band contained full-length RbcL, as demonstrated by excision and re-analysis by SDS-PAGE (data not shown), and thus represented RbcL monomers. In addition, a sharp RbcL band migrating similarly to dimeric Rubisco from R. rubrum was observed. In the presence of excess Syn7002-RbcX, these forms of RbcL decreased over time and formation of RbcL₈ occurred (Figure 6B). However, no RbcX-bound assembly intermediates were detected, perhaps due to the dynamic nature of the RbcL-RbcX interaction.
Indeed, when the same experiment was performed with AnaCA-RbcX, two slower-migrating forms of RbcL (RbcL-I₁ and RbcL-I₂) in the range of 200-350 kDa were detected in equilibrium with RbcL monomer and dimer. RbcL monomer was more efficiently cleared from GroEL and more quantitatively consumed than in the assembly reaction with Syn7002-RbcX. Immunoprecipitation with anti-FLAG antibody confirmed that AnaCA-RbcX interacted with RbcL early during assembly (data not shown). The appearance of RbcL-I₁ and RbcL-I₂ was followed by the formation of high molecular weight RbcL₈-RbcX complex (-730 kDa) that migrated close to GroEL (Figure 6C). Notably, formation of RbcL-I₁ and RbcL-I₂ was not observed upon translation of RbcL(F462A, F464A) mutant protein (Figure 6D), indicating that recognition of the C-terminal motif initiates the interaction of RbcX with RbcL. Based on these findings, RbcX appears to engage newly-synthesized RbcL subunits immediately after their chaperonin-assisted folding, at the level of monomers or dimers, shifting the equilibrium towards RbcX-bound intermediates and promoting the formation of RbcL₈ core complexes.

### CONCLUSION

### RbcX, a Specific Assembly Chaperone

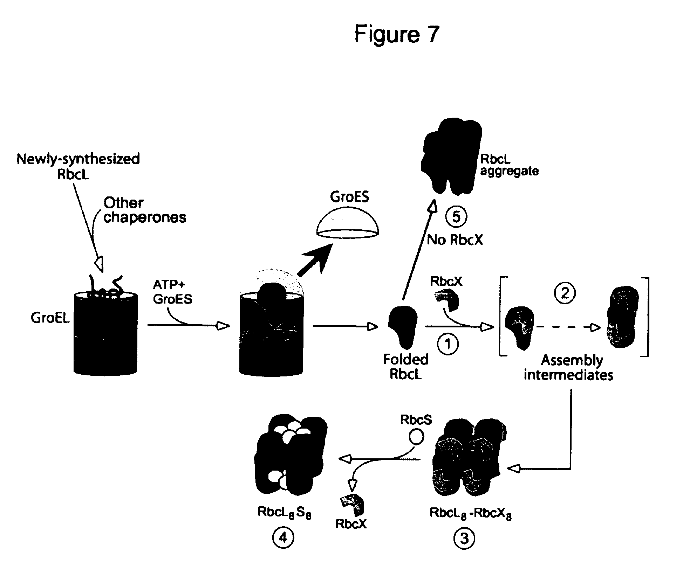

The described structural and mechanistic analysis defines RbcX as a specific assembly chaperone of cyanobacterial Rubisco, an abundant hexadecameric enzyme very similar to Rubisco of higher plants. It has been shown that RbcX interacts with RbcL subunits subsequent to their folding mediated by GroEL/GroES chaperonin (Figure 7, step 1). Folded RbcL monomers may spontaneously form dimers or interact with RbcX immediately upon release from GroEL, resulting in stabilization of assembly intermediates competent for efficient progression to RbcL₈ core particles (Figure 7, steps 2 and 3). Specific recognition of the C-terminal RbcL peptide by a central RbcX binding cleft is critical in this process; it results in shielding of the hydrophobic side chains of this sequence and serves to position peripheral binding surfaces of RbcX in proximity to RbcL. The complex between RbcL and RbcX is highly dynamic, facilitating the eventual displacement of RbcX by RbcS subunits to form the functional holoenzyme (Figure 7, step 4). RbcX is distinct from promiscuous chaperones such as Hsp70 or GroEL in that it recognizes structural features specific to RbcL. While the dependence on RbcX may vary among Rubisco homologs, the conservation of the C-terminal RbcX recognition motif in RbcL sequences suggest a general role of RbcX in ensuring efficient form I Rubisco assembly.

### RbcX Structure and Mechanism

A key feature of the arc-shaped RbcX dimer is a central binding cleft for the conserved C-terminal peptide of RbcL, encompassing the sequence EIKFEFD (residues 459 to 465 in Syn7002-RbcL). The peptide is bound in an extended conformation with the side chains F462 and F464 occupying hydrophobic pockets of the RbcX cleft. In addition, hydrogen bonds between the peptide backbone and RbcX residues lining the groove contribute to specific binding. Although RbcX is all α-helical, this binding mode is generally reminiscent of the interaction of extended hydrophobic peptides with the β-sandwich binding cleft of the general chaperone Hsp70 (Zhu et al. (Science 272 (1996), 1606-1614)). However, bacterial Hsp70, DnaK, failed to bind the C-terminal RbcL peptide (data not shown), consistent with the exclusion of negatively charged amino acid residues by DnaK (Rüdiger et al. (EMBO J. 16 (1997), 1501-1507)).
How does binding of the C-terminal RbcL peptide by RbcX promote Rubisco assembly? Given its high degree of conservation among hexadecameric Rubisco large subunits, this segment is likely to have an important structural and/or functional role. Because disruption of peptide binding abolished the formation of soluble, assembly-competent RbcL protein, RbcX appears to protect this sequence from undergoing aberrant interactions. Interestingly, C-terminal deletion of RbcL or mutation of the phenylalanines critical for RbcX binding also disrupted the RbcX-independent assembly observed with Syn6301-RbcL (Figure 8B and data not shown), indicating a direct role of the C-terminal element in forming or stabilizing the RbcL₈ core complex. Being located on the outer surface of assembled Rubisco, the C-terminal RbcL peptide is assumed to have a regulatory function in catalysis. It is thought to cycle during the enzymatic reaction between a more open conformation and a tightly bound state, stabilizing the lid segment of the active site to enclose the substrate ribulose-bisphosphate (or the transition state analog, carboxyarabinitol bisphosphate (CABP)) (Duff et al. (J. Mol. Biol. 298 (2000), 903-916); Zhu et al. (Photosynth. Res. 57 (1998), 71-79)). Formation of the closed state involves Asp468, immediately adjacent to the RbcX-recognition motif. In the crystal structure of Rubisco from Syn6301 (in complex with CABP) (Figure 18A), the C-terminal RbcL peptide is not covered by RbcS but would nevertheless be inaccessible to RbcX as it is not sufficiently detached from the complex and its hydrophobic side chains are only partially solvent-exposed. Thus, RbcX may interact with the C-terminal sequence until its attachment to the main body of Rubisco occurs.
The central RbcX binding cleft functions in cooperation with polar binding surfaces around the corners of the RbcX dimer. Mutation of the peripheral region in RbcX mutant Q29A impaired RbcL assembly in a manner distinct from that observed upon disabling the central peptide cleft. Soluble but misassembled RbcL of >800 kDa formed under these conditions (Figure 3B), suggesting that the peripheral RbcX surface promotes the proper alignment of RbcL dimers for efficient construction of RbcL₈ complexes, ultimately to be stabilized by RbcS (Curmi et al. (J. Biol. Chem. 267 (1992), 16980-16989)). Binding to the RbcL C-terminus would serve as an anchor to position the peripheral binding surfaces, perhaps allowing RbcX to bridge adjacent RbcL subunits. Assuming the α-helix preceding the C-terminal binding motif in RbcL remains in place, one edge of RbcX would be able to contact the gap between adjacent RbcL dimers, which is occupied by RbcS in the holoenzyme structure (Figure 18). How exactly RbcL interacts with the corner regions of RbcX remains to be defined, but it seems plausible that RbcX and RbcS have overlapping binding regions on RbcL.

### Dynamic Nature of RbcX Function

Binding and release of RbcX from its substrate is independent of ATP-regulated conformational changes. It has been found that the interaction between RbcX and RbcL is dynamic, thereby allowing for efficient final displacement of RbcX by RbcS. While the binding constants of RbcX for the C-terminal RbcL peptide alone are in the 100 µM range, the overall affinity of RbcX for the RbcL₈ core complex appears to be close to 1 µM (Figure 5C). Thus, the polar corner surface of RbcX contributes substantially to overall binding, presumably facilitated by the occurrence of this region on both ends of the symmetrical RbcX dimer. However, the peripheral sites alone fail to maintain a stable interaction with RbcL. Thus, combining two structurally independent, relatively weak interaction sites would provide biologically relevant binding strength coupled with high dynamics, a strategy that may be followed more generally in assisted assembly processes. Importantly, the binding parameters of the RbcX-RbcL interaction (and similarly the RbcS-RbcL interaction) appear to have been optimized for the proteins from the same organism in order to facilitate RbcX recycling. As a consequence, exchangeability of the proteins between organisms is limited, despite their close evolutionary relationship. Combining Syn7002-RbcL with the RbcX from Anabaena sp. CA, which binds the C-terminal RbcL-peptide with ∼30-fold higher affinity, demonstrated the significance of this adaptation. Formation of a stable dead-end complex, most likely RbcL₈-RbcX₈, was observed with AnaCA-RbcX, presumably defining the otherwise transitory end-state of RbcX activity in the assembly pathway prior to entry of RbcS (Figure 7).

### Implications for Rubisco Assembly in Higher Plants

Hexadecameric Form I Rubisco is thought to have evolved from the simpler dimeric Form II as an adaptation to the increased oxygen and lowered carbon dioxide atmosphere. The resulting increase in structural complexity may explain the need for a specific assembly chaperone, particularly when considering the complex role of the C-terminal RbcL segment in assembly and catalysis of Form I Rubisco (Zhu et al., (1998) *loc. cit*.). Indeed, the C-terminal RbcX recognition sequence is conserved in higher plant RbcL, and homologs to RbcX genes are present in plant genomes exhibiting 29% similarity to the cyanobacterial RbcX sequences. It is thus concluded that RbcX has an important role in the assembly process of plant Rubisco, which is faced with the additional difficulty that RbcS subunits must be imported into chloroplasts to assemble with RbcL synthesized within the organelle (Gatenby and Ellis (Ann. Rev. Cell Biol. 6 (1990), 125-149)). The failure of heterologous form I Rubiscos to assemble upon transgenic expression in plants (Andrews and Whitney (Arch. Biochem. Biophys. 414 (2003), 159-169)) may suggest an incompatibility of these enzymes with plant RbcX.

### Table 1: Crystallographic Data Collection, Phasing and Refinement Statistics

Values in parenthesis are for the highest resolution shell. Data statistics are given according to the definitions used in Scala (Evans ((Joint CCP4 and ESF-EACBM Newsletters 33 (1997), 22-24)). The Ramachandran plot distributions were determined with Procheck (Laskowski et al. (J. Appl. Cryst. 26 (1993), 283-291)). An atomic model could not be refined against the RbcX(R70A) dataset because of insufficient experimental parameters limited by the low diffraction power of these crystals. The statistics for rigid body and TLS refinement of the Native I model against this dataset indicated, however, that the crystal packing between wildtype and R70A mutant RbcX was very similar (data not shown).

**TABLE 1 Crystallographic Data Collection, Phasing and Refinement Statistics**

| **Dataset** | **PtC14-2** | **native I** | **SeMet** | **native II** | **peptide cplx.** | **Y17A/Y20L** | **Q29A** | **R70A** | **Anabaena CA** |
|---|---|---|---|---|---|---|---|---|---|
| beamline | ESRF, BM14 | SLS, X10SA | ESRF, ID14-4 | ESRF, ID29 | ESRF, ID29 | ESRF, ID29 | ESRF, ID29 | ESRF, ID29 | SLS, X10SA |
| wavelength (A) | 0.8793 | 0.9789 | 0.9795 | 1.000 | 0.976 | 1.000 | 1.000 | 1.000 | 1.072 |
| space group | *P*4₁2₁2 | *P*4₁2₁2 | *P*1 | 1222 | *P*4₁2₁2 | *P*4₁2₁2 | *P*4₁2₁2 | *P*4₁2₁2 | *P*6₅ |
| cell dimensions, | | | | | | | | | |
| a, b, c (Å); | 93.42, 93.42, 407.75; | 93.33, 93.33, 411.99; | 70.36, 71.10, 113.16; | 63.50, 69.34, 98.79 | 93.21, 93.21, 412.02; | 93.45, 93.45, 411.54, | 93.00, 93.00, 413.72 | 93.54, 93.54, 413.88 | 69.02, 69.02, 113.81, |
| α, β, γ (°) | 90, 90, 90 | 90, 90, 90 | 89.58, 81.10, 80.09 | 90, 90, 90 | 90, 90, 90 | 90, 90, 90 | 90, 90, 90 | 90, 90, 90 | 90, 90, 120 |
| resolution limits (Å)* | 93.25 - 3.15 | 91.29 - 2.8 | 68.52 - 2.7 | 29.24 - 1.9 | 93.25 - 2.95 | 93.45 - 3.4 | 92.85 - 3.1 | 41.00 - 6.1 | 59.77 - 2.4 |
| | (3.32 - 3.15) | (2.95 - 2.8) | (2.85 - 2.7) | (2.0 - 1.9) | (3.11 - 2.95) | (3.58 - 3.4) | (3.27 - 3.1) | (6.43 - 6.1) | (2.53 - 2.4) |
| Rmerge ** | 0.081 (0.621) | 0.080 (0.641) | 0.101 (0.420) | 0.089 (0.575) | 0.080 (0.536) | 0.089 (0.503) | 0.077 (0.485) | 0.107 (0.408) | 0.078 (0.444) |
| I/sigma | 15.9 (3.1) | 26.7 (4.6) | 11.6 (2.7) | 11.5 (2.2) | 11.7 (2.5) | 9.5 (2.4) | 10.6 (2.9) | 6.7 (1.8) | 10.7 (2.0) |
| multiplicity | 7.0 (7.1) | 14.5 (14.8) | 3.9 (3.8) | 4.2 (4.3) | 3.7 (3.8) | 3.4 (3.6) | 3.5 (3.6) | 2.0 (1.9) | 3.6 (2.9) |
| completeness (%) | 100 (100) | 100 (100) | 96.9 (96.3) | 100 (100) | 96.6 (97.6) | 99.6 (99.8) | 99.9 (99.9) | 75.9 (75.9) | 98.1 (88.2) |
| RbcX chains /a.u. | 6 | 6 | 12 | 2 | 6, 1 peptide | 6 | 6 | | 2 |
| solvent content (%) | - | 74.7 | 65.9 | 30.7 | - | - | - | | 50.9 |
| Wilson B-factor (Å²) | - | - | 49.60 | 24.10 | - | - | - | | 41.5 |
| Phasing | | | | | | | | | |
| sites | 6 (Pt) | - | 37 (Se) | - | - | - | - | | - |
| mean FoM | 0.31 (SIRAS) | - | 0.322 (SAD) | - | - | - | - | | - |
| Refinement | | | | | | | | | |
| resolution range | - | 20 - 2.8 | 20 - 2.7 | 20 - 1.9 | 20 - 2.95 | 20 - 3.4 | 20 - 3.1 | | 20 - 2.5 |
| reflections (test set) | - | 43747(2321) | 53770(2881) | 16664(887) | 35788 (1913) | 24596(1319) | 32237 (1726) | | 10128(502) |
| Rwork | - | 0.240 | 0.212 2 | 0.197 | 0.235 | 0.253 | 0.231 | | 0.241 |
| Rfree | - | 0.262 | 0.262 | 0.253 | 0.264 | 0.294 | 0.260 | | 0.298 |
| number of atoms | - | 5105 | 10068 | 1893 | 5232 | 4727 | 4976 | | 1769 |
| r.m.s.d. bonds (Å) | - | 0.011 | 0.020 | 0.013 | 0.012 | 0.010 | 0.010 | | 0.012 |
| r.m.s.d. angles (°) | - | 1.406 | 1.683 | 1.390 | 1.388 | 1.319 | 1.273 | | 1.448 |
| Ramachandran plot *** | | | | | | | | | |
| % most favored region | - | 93.0 | 91.6 | 95.0 | 93.5 | 87.8 | 93.9 | | 93.6 |
| % additionally allowed | - | 6.8 | 8.3 | 5.0 | 6.5 | 11.9 | 6.1 | | 5.9 |

| | | | | | | | | | |
|---|---|---|---|---|---|---|---|---|---|
| * Values in parenthesis for outer shell. ** As defined in Scala. *** As defined in Procheck (Laskowski et al.). | | | | | | | | | |

## Claims

1. A method of expressing a form I Rubisco of plant origin in a prokaryotic host cell comprising:
(i) expressing in the prokaryotic host cell a nucleic acid sequence encoding the large subunit of said form I Rubisco (RbcL) and a nucleic acid sequence encoding the small subunit of a form I Rubisco (RbcS); and
(ii) expressing in the prokaryotic host cell a nucleic acid sequence encoding a plant RbcX protein;
wherein the RbcX protein is derived from the same plant species from which the RbcL subunit is derived.

2. A method for selecting a form I Rubisco of plant origin for desired properties comprising the steps of:
(a) subjecting a nucleic acid sequence encoding the large subunit of a plant form I Rubisco (RbcL) and/or a nucleic acid sequence encoding the small subunit of a plant form I Rubisco (RbcS) and/or a nucleic acid sequence encoding a plant RbcX protein to a process which introduces (a) mutation(s) into the nucleic acid sequence;
(b) expressing in a prokaryotic host cell a nucleic acid sequence encoding a plant form I RbcL, a nucleic acid sequence encoding a plant form I RbcS and a nucleic acid sequence encoding a plant RbcX protein, wherein at least one of said sequences has been subjected to a process of step (a) which introduces (a) mutation(s) and wherein the RbcX protein is derived from the same plant species from which the RbcL subunit is derived;
(c) analyzing the properties of the Rubisco protein obtained according to step (b); and
(d) selecting a nucleic acid sequence encoding a Rubisco protein with the desired properties.

3. A method for selecting a form I Rubisco with desired properties comprising the steps of:
(a) subjecting a nucleic acid sequence encoding the large subunit of a form I Rubisco (RbcL) to a process by which (a) mutation(s) is/are introduced into the C-terminal sequence of the RbcL subunit;
(b) expressing the mutated sequence obtained in step (a) in a host cell simultaneously with a nucleic acid sequence encoding a small subunit of a form I Rubisco (RbcS) and together with a nucleic acid sequence encoding an RcbX protein which is derived from the same species as said RbcL;
(c) analyzing the properties of the Rubisco protein obtained according to step (b); and
(d) selecting a nucleic acid sequence encoding a Rubisco protein with the desired properties.

4. A method for producing a modified Rubisco RbcL subunit **characterized in that** the C-terminal sequence of a RbcL subunit of one species is modified so as to have the C-terminal sequence of a form I RbcL subunit of a different species thereby resulting in a chimeric RbcL subunit in which the C-terminal sequence is heterologous to the remainder of the molecule.

5. The method of claim 4, wherein the RbcL subunit in which the C-terminal sequence is modified is a form I RbcL subunit.

6. The method of claim 4, wherein the RbcL subunit in which the C-terminal sequence is modified is a form II RcbL subunit.

7. A modified form I RbcL subunit resulting from the modification of its C-terminus so as to contain a C-terminal sequence of a form I RbcL from a different species.

8. A modified form II RbcL subunit resulting from the modification of its C-terminus so as to contain a C-terminal sequence of a form I RbcL.

9. A method for producing a transgenic plant cell comprising the step of transforming a plant cell with a nucleic acid molecule encoding a form I RbcL subunit and with a nucleic acid molecule encoding a RbcX protein, wherein the RbcL subunit and the RbcX protein are derived from the same species.

10. A method for producing a transgenic plant comprising the step of regenerating from the cell obtained according to the method of claim 9 a plant.

11. A transgenic plant cell comprising a nucleic acid molecule encoding a RbcL subunit which is heterologous with respect to the plant cell and a nucleic acid molecule encoding a RbcX protein, wherein the RbcL subunit and the RbcX protein are derived from the same species.

12. A transgenic plant comprising a transgenic plant cell of claim 11.


SEQUENCE LISTING

<110> Max-Planck-Gesellschaft zur Förderung der Wissenschaften
e.V.

<120> MEANS AND METHODS FOR PROVIDING RIBULOSE
BISPHOSPHATE-CARBOXYLASE-OXYGENASE WITH IMPROVED PROPERTIES

<130> N1425 EP S3

<160> 18

<170> PatentIn version 3.3

<210> 1
<211> 612
<212> DNA
<213> Arabidopsis thaliana

<220>
<221> CDS
<222> (1)..(612)

<400> 1
atg gtg agt gct tgg ttt gtt gtt ggc tca ccg gtg atg gac tct tcg 48
Met Val Ser Ala Trp Phe Val Val Gly Ser Pro Val Met Asp Ser Ser
1               5                   10                  15

agc agt cca tgt tta tgt ctg gat gct cac acg acg ggt acc atc agg 96
Ser Ser Pro Cys Leu Cys Leu Asp Ala His Thr Thr Gly Thr Ile Arg
            20                  25                  30

agg aag aag atc ctt ggc aaa gca aga aat ctg gaa tta gga agc tct 144
Arg Lys Lys Ile Leu Gly Lys Ala Arg Asn Leu Glu Leu Gly Ser Ser
        35                  40                  45

ttc act ggc tca cgc att gtt ttt agg ctt tct ccg aag cgt gta tcg 192
Phe Thr Gly Ser Arg Ile Val Phe Arg Leu Ser Pro Lys Arg Val Ser
    50                  55                  60

aga att gcg aat cga aag agc aaa aag ctc ttg att gtg aat gag gat 240
Arg Ile Ala Asn Arg Lys Ser Lys Lys Leu Leu Ile Val Asn Glu Asp
65                  70                  75                  80

gtt gct ggt aat tac gac gat acc ttc ggt gat gta caa aag caa att 288
Val Ala Gly Asn Tyr Asp Asp Thr Phe Gly Asp Val Gln Lys Gln Ile
                85                  90                  95

gtt aat tat ttt aca tac aaa gct gtg agg acg gtt ctt cat cag ctg 336
Val Asn Tyr Phe Thr Tyr Lys Ala Val Arg Thr Val Leu His Gln Leu
            100                 105                 110

tac gaa atg aat cct cct caa tac act tgg ttc tat aat cat atc ata 384
Tyr Glu Met Asn Pro Pro Gln Tyr Thr Trp Phe Tyr Asn His Ile Ile
        115                 120                 125

aca aac aga ccg acg gat ggc aaa cgt ttc ctc cgt gcc ctc ggc aag 432
Thr Asn Arg Pro Thr Asp Gly Lys Arg Phe Leu Arg Ala Leu Gly Lys
    130                 135                 140

gag agt cag gag ctt gca gaa aga gtg atg atc acg cgt ctt cac ttg 480
Glu Ser Gln Glu Leu Ala Glu Arg Val Met Ile Thr Arg Leu His Leu

145                 150                 155                 160

tat ggc aaa tgg atc aag aaa tgc gac cat ggg aag ata tac cag gaa       528
Tyr Gly Lys Trp Ile Lys Lys Cys Asp His Gly Lys Ile Tyr Gln Glu
                165                 170                 175

ata tcg gat gaa aac ttg gcg ttg atg cgt gaa cgc ctg atg gag acc       576
Ile Ser Asp Glu Asn Leu Ala Leu Met Arg Glu Arg Leu Met Glu Thr
            180                 185                 190

gtg ata tgg cct tcc gat gac aca aac tca agg taa                       612
Val Ile Trp Pro Ser Asp Asp Thr Asn Ser Arg
        195                 200

<210>  2
<211>  203
<212>  PRT
<213>  Arabidopsis thaliana

<400>  2

Met Val Ser Ala Trp Phe Val Val Gly Ser Pro Val Met Asp Ser Ser
1               5                   10                  15

Ser Ser Pro Cys Leu Cys Leu Asp Ala His Thr Thr Gly Thr Ile Arg
            20                  25                  30

Arg Lys Lys Ile Leu Gly Lys Ala Arg Asn Leu Glu Leu Gly Ser Ser
        35                  40                  45

Phe Thr Gly Ser Arg Ile Val Phe Arg Leu Ser Pro Lys Arg Val Ser
    50                  55                  60

Arg Ile Ala Asn Arg Lys Ser Lys Lys Leu Leu Ile Val Asn Glu Asp
65                  70                  75                  80

Val Ala Gly Asn Tyr Asp Asp Thr Phe Gly Asp Val Gln Lys Gln Ile
                85                  90                  95

Val Asn Tyr Phe Thr Tyr Lys Ala Val Arg Thr Val Leu His Gln Leu
            100                 105                 110

Tyr Glu Met Asn Pro Pro Gln Tyr Thr Trp Phe Tyr Asn His Ile Ile
        115                 120                 125

Thr Asn Arg Pro Thr Asp Gly Lys Arg Phe Leu Arg Ala Leu Gly Lys
    130                 135                 140

Glu Ser Gln Glu Leu Ala Glu Arg Val Met Ile Thr Arg Leu His Leu
145                 150                 155                 160

Tyr Gly Lys Trp Ile Lys Lys Cys Asp His Gly Lys Ile Tyr Gln Glu

165                 170                 175

Ile Ser Asp Glu Asn Leu Ala Leu Met Arg Glu Arg Leu Met Glu Thr
            180                 185                 190

Val Ile Trp Pro Ser Asp Asp Thr Asn Ser Arg
        195                 200

<210>  3
<211>  525
<212>  DNA
<213>  Arabidopsis thaliana

<220>
<221>  CDS
<222>  (1)..(525)

<400>  3
atg gag tca tct tct tca ctc ctt cat cac tct tac ctc tct tac ctt       48
Met Glu Ser Ser Ser Ser Leu Leu His His Ser Tyr Leu Ser Tyr Leu
1               5                   10                  15

aat cca aaa ttt gga aaa aga cct ttg gtt tct tat cca tta atg cag       96
Asn Pro Lys Phe Gly Lys Arg Pro Leu Val Ser Tyr Pro Leu Met Gln
            20                  25                  30

agt tcg cga aaa tgc aaa caa act cgc att tgt tct aac aag atg tat      144
Ser Ser Arg Lys Cys Lys Gln Thr Arg Ile Cys Ser Asn Lys Met Tyr
        35                  40                  45

gtt ccc ggc ttt gga gaa gct tca ccg gag gct aag gca gcg aag cat      192
Val Pro Gly Phe Gly Glu Ala Ser Pro Glu Ala Lys Ala Ala Lys His
    50                  55                  60

ctt cat gac ttc ttt act tac gtt gca gtg agg ata gtg tct gct cag      240
Leu His Asp Phe Phe Thr Tyr Val Ala Val Arg Ile Val Ser Ala Gln
65                  70                  75                  80

ctt gag agt tat aat cct gag gct tat atg gag ttg aga gaa ttt tta      288
Leu Glu Ser Tyr Asn Pro Glu Ala Tyr Met Glu Leu Arg Glu Phe Leu
                85                  90                  95

gat aca aac tct gta agt gac ggt gat aaa ttc tgc gcc act ctc atg      336
Asp Thr Asn Ser Val Ser Asp Gly Asp Lys Phe Cys Ala Thr Leu Met
            100                 105                 110

cgt cgc tct tca cgt cac atg aac tta gcc ctt cga att tta gag gta      384
Arg Arg Ser Ser Arg His Met Asn Leu Ala Leu Arg Ile Leu Glu Val
        115                 120                 125

cgg tct gct tat tgt aaa aac gat ttc gaa tgg gat aat atg aag cgc      432
Arg Ser Ala Tyr Cys Lys Asn Asp Phe Glu Trp Asp Asn Met Lys Arg
    130                 135                 140

ctc gcc ttc aag aac gta gat gat tcc aac aca aga ctc atg cgc gag      480
Leu Ala Phe Lys Asn Val Asp Asp Ser Asn Thr Arg Leu Met Arg Glu
145                 150                 155                 160

tac gtc ttg gag act agc cat gtc gaa acc gat tct gat aag tga          525

Tyr Val Leu Glu Thr Ser His Val Glu Thr Asp Ser Asp Lys
                165                 170

<210>  4
<211>  174
<212>  PRT
<213>  Arabidopsis thaliana

<400>  4

Met Glu Ser Ser Ser Ser Leu Leu His His Ser Tyr Leu Ser Tyr Leu
1               5                   10                  15

Asn Pro Lys Phe Gly Lys Arg Pro Leu Val Ser Tyr Pro Leu Met Gln
            20                  25                  30

Ser Ser Arg Lys Cys Lys Gln Thr Arg Ile Cys Ser Asn Lys Met Tyr
        35                  40                  45

Val Pro Gly Phe Gly Glu Ala Ser Pro Glu Ala Lys Ala Ala Lys His
    50                  55                  60

Leu His Asp Phe Phe Thr Tyr Val Ala Val Arg Ile Val Ser Ala Gln
65                  70                  75                  80

Leu Glu Ser Tyr Asn Pro Glu Ala Tyr Met Glu Leu Arg Glu Phe Leu
                85                  90                  95

Asp Thr Asn Ser Val Ser Asp Gly Asp Lys Phe Cys Ala Thr Leu Met
            100                 105                 110

Arg Arg Ser Ser Arg His Met Asn Leu Ala Leu Arg Ile Leu Glu Val
        115                 120                 125

Arg Ser Ala Tyr Cys Lys Asn Asp Phe Glu Trp Asp Asn Met Lys Arg
    130                 135                 140

Leu Ala Phe Lys Asn Val Asp Asp Ser Asn Thr Arg Leu Met Arg Glu
145                 150                 155                 160

Tyr Val Leu Glu Thr Ser His Val Glu Thr Asp Ser Asp Lys
                165                 170

<210>  5
<211>  588
<212>  DNA
<213>  Oryza sativa

<220>
<221>  CDS

<222>  (1)..(588)

<400>  5
atg gcc ggg gtg cag gtg atg ccg gcg gtg ggg gcg gtg gcg acg gcg        48
Met Ala Gly Val Gln Val Met Pro Ala Val Gly Ala Val Ala Thr Ala
1               5                   10                  15

gaa ggg agc tgc agg gcg gcg ggg cag agg agg ggc cgc ggc gtg agc        96
Glu Gly Ser Cys Arg Ala Ala Gly Gln Arg Arg Gly Arg Gly Val Ser
            20                  25                  30

agc ttg ttc gcc gga gac tgg agg cgg cgg cct cgc cgg gcg gcg tgc       144
Ser Leu Phe Ala Gly Asp Trp Arg Arg Arg Pro Arg Arg Ala Ala Cys
        35                  40                  45

acg gcc agg gtc agg ggc agg agg cag cag cag cag cag cag ggc ctc       192
Thr Ala Arg Val Arg Gly Arg Arg Gln Gln Gln Gln Gln Gln Gly Leu
    50                  55                  60

gcc gtc gtc tgc aac ctc ggt ggc acc tac gat gaa ggt ttc gag gac       240
Ala Val Val Cys Asn Leu Gly Gly Thr Tyr Asp Glu Gly Phe Glu Asp
65                  70                  75                  80

atc cac gtg caa ctg att aat gtg ttc acc tac aaa gcc gtc aag acc       288
Ile His Val Gln Leu Ile Asn Val Phe Thr Tyr Lys Ala Val Lys Thr
                85                  90                  95

gtg ctc acg cag ctc tac gaa atg aac cca cca agc tac cga tgg tta       336
Val Leu Thr Gln Leu Tyr Glu Met Asn Pro Pro Ser Tyr Arg Trp Leu
            100                 105                 110

tac aac ttc gtt gct gta aac aag cct acc gat ggc aaa gtg ttc ctt       384
Tyr Asn Phe Val Ala Val Asn Lys Pro Thr Asp Gly Lys Val Phe Leu
        115                 120                 125

cgt gct ctt gga aag gag aag cag gag ctt gca gag cga gtg atg ata       432
Arg Ala Leu Gly Lys Glu Lys Gln Glu Leu Ala Glu Arg Val Met Ile
    130                 135                 140

act agg ctt cac ttg tac agc aaa tgg atc aag aaa tgt gac cat gcg       480
Thr Arg Leu His Leu Tyr Ser Lys Trp Ile Lys Lys Cys Asp His Ala
145                 150                 155                 160

atg atg tac gag agg ata tct gac gag aac ctg gcg ttg atg cga gag       528
Met Met Tyr Glu Arg Ile Ser Asp Glu Asn Leu Ala Leu Met Arg Glu
                165                 170                 175

cgg ctc atg gag acg gtc atc tgg cca aca gac gac acc aac act gag       576
Arg Leu Met Glu Thr Val Ile Trp Pro Thr Asp Asp Thr Asn Thr Glu
            180                 185                 190

aag att ggc tga                                                       588
Lys Ile Gly
        195


<210>  6
<211>  195
<212>  PRT
<213>  Oryza sativa

<400>  6

Met Ala Gly Val Gln Val Met Pro Ala Val Gly Ala Val Ala Thr Ala
1               5                   10                  15

Glu Gly Ser Cys Arg Ala Ala Gly Gln Arg Arg Gly Arg Gly Val Ser
            20                  25                  30

Ser Leu Phe Ala Gly Asp Trp Arg Arg Arg Pro Arg Arg Ala Ala Cys
        35                  40                  45

Thr Ala Arg Val Arg Gly Arg Arg Gln Gln Gln Gln Gln Gln Gly Leu
    50                  55                  60

Ala Val Val Cys Asn Leu Gly Gly Thr Tyr Asp Glu Gly Phe Glu Asp
65                  70                  75                  80

Ile His Val Gln Leu Ile Asn Val Phe Thr Tyr Lys Ala Val Lys Thr
                85                  90                  95

Val Leu Thr Gln Leu Tyr Glu Met Asn Pro Pro Ser Tyr Arg Trp Leu
            100                 105                 110

Tyr Asn Phe Val Ala Val Asn Lys Pro Thr Asp Gly Lys Val Phe Leu
        115                 120                 125

Arg Ala Leu Gly Lys Glu Lys Gln Glu Leu Ala Glu Arg Val Met Ile
    130                 135                 140

Thr Arg Leu His Leu Tyr Ser Lys Trp Ile Lys Lys Cys Asp His Ala
145                 150                 155                 160

Met Met Tyr Glu Arg Ile Ser Asp Glu Asn Leu Ala Leu Met Arg Glu
                165                 170                 175

Arg Leu Met Glu Thr Val Ile Trp Pro Thr Asp Asp Thr Asn Thr Glu
            180                 185                 190

Lys Ile Gly
        195

<210>  7
<211>  582
<212>  DNA
<213>  Oryza sativa

<220>
<221>  CDS
<222>  (1)..(582)

<400>  7

atg gct gcc gtc gcg cgg atg gcc gcc gcg gtc gtc tgc ctt ccc tcc 48
Met Ala Ala Val Ala Arg Met Ala Ala Ala Val Val Cys Leu Pro Ser
1               5                   10                  15

tcg tcc tca tcc tcc tcc gcg tgc cgc gcg gcg cca ttg ccg tgg tcc 96
Ser Ser Ser Ser Ser Ser Ala Cys Arg Ala Ala Pro Leu Pro Trp Ser
            20                  25                  30

cgc ggc gtc gtc gtc ggg gtg agg cgg cgg cgc acg gtg gcg cgg gcg 144
Arg Gly Val Val Val Gly Val Arg Arg Arg Arg Thr Val Ala Arg Ala
        35                  40                  45

gcg cgg cgg agg ggg agg agg ccc ggc cgc cgc ggc ctc gtc atc gtg 192
Ala Arg Arg Arg Gly Arg Arg Pro Gly Arg Arg Gly Leu Val Ile Val
    50                  55                  60

gac gag ttc gcc ggg cag tac gag gag ggg ttc gaa gac gtg cac acg 240
Asp Glu Phe Ala Gly Gln Tyr Glu Glu Gly Phe Glu Asp Val His Thr
65                  70                  75                  80

gaa atc atg aat tac ttc act tac aag gcc aca agc acg gtg ctc cac 288
Glu Ile Met Asn Tyr Phe Thr Tyr Lys Ala Thr Ser Thr Val Leu His
                85                  90                  95

cag ttg tat gaa atg aat cca cct gct tat act tgg ctg tat aac tac 336
Gln Leu Tyr Glu Met Asn Pro Pro Ala Tyr Thr Trp Leu Tyr Asn Tyr
            100                 105                 110

gtc gtg gtt aac gat cca aag gag ggc aaa cat ttc ctc atc gca ctc 384
Val Val Val Asn Asp Pro Lys Glu Gly Lys His Phe Leu Ile Ala Leu
        115                 120                 125

gca aag gag agg caa gat ttg gca gag aga gtg atg atc aca cga ctc 432
Ala Lys Glu Arg Gln Asp Leu Ala Glu Arg Val Met Ile Thr Arg Leu
    130                 135                 140

cac ttg tac agc aaa tgg atc aag aaa tgt gac cat gca aaa atg tac 480
His Leu Tyr Ser Lys Trp Ile Lys Lys Cys Asp His Ala Lys Met Tyr
145                 150                 155                 160

gag aag att tcg aat gag aat ctg gag ata atg cgt cag agg tta atg 528
Glu Lys Ile Ser Asn Glu Asn Leu Glu Ile Met Arg Gln Arg Leu Met
                165                 170                 175

gaa act gta gcg tgg cca acg gac gac aca aac acc agc gac acg gcg 576
Glu Thr Val Ala Trp Pro Thr Asp Asp Thr Asn Thr Ser Asp Thr Ala
            180                 185                 190

aaa tga 582
Lys

<210> 8
<211> 193
<212> PRT
<213> Oryza sativa

<400> 8

Met Ala Ala Val Ala Arg Met Ala Ala Ala Val Val Cys Leu Pro Ser
1               5                   10                  15

Ser Ser Ser Ser Ser Ser Ala Cys Arg Ala Ala Pro Leu Pro Trp Ser
            20                  25                  30

Arg Gly Val Val Val Gly Val Arg Arg Arg Arg Thr Val Ala Arg Ala
        35                  40                  45

Ala Arg Arg Arg Gly Arg Arg Pro Gly Arg Arg Gly Leu Val Ile Val
    50                  55                  60

Asp Glu Phe Ala Gly Gln Tyr Glu Glu Gly Phe Glu Asp Val His Thr
65                  70                  75                  80

Glu Ile Met Asn Tyr Phe Thr Tyr Lys Ala Thr Ser Thr Val Leu His
                85                  90                  95

Gln Leu Tyr Glu Met Asn Pro Pro Ala Tyr Thr Trp Leu Tyr Asn Tyr
            100                 105                 110

Val Val Val Asn Asp Pro Lys Glu Gly Lys His Phe Leu Ile Ala Leu
        115                 120                 125

Ala Lys Glu Arg Gln Asp Leu Ala Glu Arg Val Met Ile Thr Arg Leu
    130                 135                 140

His Leu Tyr Ser Lys Trp Ile Lys Lys Cys Asp His Ala Lys Met Tyr
145                 150                 155                 160

Glu Lys Ile Ser Asn Glu Asn Leu Glu Ile Met Arg Gln Arg Leu Met
                165                 170                 175

Glu Thr Val Ala Trp Pro Thr Asp Asp Thr Asn Thr Ser Asp Thr Ala
            180                 185                 190

Lys

<210>  9
<211>  561
<212>  DNA
<213>  Oryza sativa

<220>
<221>  CDS
<222>  (1)..(561)

<400>  9
atg gcg tct cct cgc tgc gcc gcc gtc gcc ctc ctc cat ccc gcc gga        48
Met Ala Ser Pro Arg Cys Ala Ala Val Ala Leu Leu His Pro Ala Gly
1               5                   10                  15

gtc gcc gcc ggc ggc gga gct cgc cgg cgt gtc ctc ctc ctc gac caa          96
Val Ala Ala Gly Gly Gly Ala Arg Arg Arg Val Leu Leu Leu Asp Gln
            20                  25                  30

gag cgg ccg ttg tgg ggg act gag gtg cgc cgg cgc cgg cgc cgg cgt          144
Glu Arg Pro Leu Trp Gly Thr Glu Val Arg Arg Arg Arg Arg Arg Arg
        35                  40                  45

ttc tcg agc ctc gag acg ccg ccg cgg tgc agc aag atg tac gta ccc          192
Phe Ser Ser Leu Glu Thr Pro Pro Arg Cys Ser Lys Met Tyr Val Pro
    50                  55                  60

ggc ttc gga gag gga tcg ccg gag aag aag gca gca aga aac ctg cag          240
Gly Phe Gly Glu Gly Ser Pro Glu Lys Lys Ala Ala Arg Asn Leu Gln
65                  70                  75                  80

cac ttc ttc aac tac att gct gtc agg gtt gtg ctc aca cag ctt gag          288
His Phe Phe Asn Tyr Ile Ala Val Arg Val Val Leu Thr Gln Leu Glu
                85                  90                  95

agt tac aac cgg gaa gca tat ggt gag ctg atg gat ttc gtg aac cga          336
Ser Tyr Asn Arg Glu Ala Tyr Gly Glu Leu Met Asp Phe Val Asn Arg
            100                 105                 110

aac tcc ctc aac gac gct gat act ttc tgc aag aag ttg atc cgc gag          384
Asn Ser Leu Asn Asp Ala Asp Thr Phe Cys Lys Lys Leu Ile Arg Glu
        115                 120                 125

tct cca agg cac aag cag cta gca atg agg atc ttg gag gtc cga tct          432
Ser Pro Arg His Lys Gln Leu Ala Met Arg Ile Leu Glu Val Arg Ser
    130                 135                 140

gct tat gtc aag cat gat ttc gag tgg gat aat ctg aaa agg tta tct          480
Ala Tyr Val Lys His Asp Phe Glu Trp Asp Asn Leu Lys Arg Leu Ser
145                 150                 155                 160

ttc aag atg gtt gat gag gcc aac aca aag ctc atg agg gac tat gtc          528
Phe Lys Met Val Asp Glu Ala Asn Thr Lys Leu Met Arg Asp Tyr Val
                165                 170                 175

ttg gag acc agc cac atc gaa gac gat aac tga                              561
Leu Glu Thr Ser His Ile Glu Asp Asp Asn
            180                 185


<210>  10
<211>  186
<212>  PRT
<213>  Oryza sativa

<400>  10

Met Ala Ser Pro Arg Cys Ala Ala Val Ala Leu Leu His Pro Ala Gly
1               5                   10                  15


Val Ala Ala Gly Gly Gly Ala Arg Arg Arg Val Leu Leu Leu Asp Gln
            20                  25                  30


Glu Arg Pro Leu Trp Gly Thr Glu Val Arg Arg Arg Arg Arg Arg Arg
        35                  40                  45

Phe Ser Ser Leu Glu Thr Pro Pro Arg Cys Ser Lys Met Tyr Val Pro
    50                  55                  60

Gly Phe Gly Glu Gly Ser Pro Glu Lys Lys Ala Ala Arg Asn Leu Gln
65                  70                  75                  80

His Phe Phe Asn Tyr Ile Ala Val Arg Val Val Leu Thr Gln Leu Glu
                85                  90                  95

Ser Tyr Asn Arg Glu Ala Tyr Gly Glu Leu Met Asp Phe Val Asn Arg
            100                 105                 110

Asn Ser Leu Asn Asp Ala Asp Thr Phe Cys Lys Lys Leu Ile Arg Glu
        115                 120                 125

Ser Pro Arg His Lys Gln Leu Ala Met Arg Ile Leu Glu Val Arg Ser
    130                 135                 140

Ala Tyr Val Lys His Asp Phe Glu Trp Asp Asn Leu Lys Arg Leu Ser
145                 150                 155                 160

Phe Lys Met Val Asp Glu Ala Asn Thr Lys Leu Met Arg Asp Tyr Val
                165                 170                 175

Leu Glu Thr Ser His Ile Glu Asp Asp Asn
            180                 185

<210>  11
<211>  678
<212>  DNA
<213>  Physcomitrella patens

<220>
<221>  CDS
<222>  (1)..(678)

<400>  11
atg gtt gga gcc act cta gtt ggc gct agc atg gct ggt gcg gtg cag       48
Met Val Gly Ala Thr Leu Val Gly Ala Ser Met Ala Gly Ala Val Gln
1               5                   10                  15

gct gtc agc ctc gca ggg ccc cct tgc agg tgc aca agc ttt gag atg       96
Ala Val Ser Leu Ala Gly Pro Pro Cys Arg Cys Thr Ser Phe Glu Met
            20                  25                  30

gct gct ggt gga gta gtc gta tca agg aag caa ggt cat tcg tgc tcc      144
Ala Ala Gly Gly Val Val Val Ser Arg Lys Gln Gly His Ser Cys Ser
        35                  40                  45

atg att caa agg aag agc aat act att gtg cac ttg tac agc aca atg      192
Met Ile Gln Arg Lys Ser Asn Thr Ile Val His Leu Tyr Ser Thr Met

50                  55                  60

aca cag gct tgg caa gat tct cgt cta act gct cgg gct ctg gct cat        240
Thr Gln Ala Trp Gln Asp Ser Arg Leu Thr Ala Arg Ala Leu Ala His
65                  70                  75                  80

acc att cct tgc tct act agt ggc att cat cat gct ccc atg gtg gtg        288
Thr Ile Pro Cys Ser Thr Ser Gly Ile His His Ala Pro Met Val Val
                85                  90                  95

aac gag ctc ggc ggc cag tat gag gat act ttt gat gat gtg gaa aaa        336
Asn Glu Leu Gly Gly Gln Tyr Glu Asp Thr Phe Asp Asp Val Glu Lys
            100                 105                 110

cac ttg ctc gac tac ttc acg tat aaa gcc gtc aaa aca gtg ctg gct        384
His Leu Leu Asp Tyr Phe Thr Tyr Lys Ala Val Lys Thr Val Leu Ala
        115                 120                 125

cag ctt tat gag atg aat cca cct gaa tac gct tgg ttt tac aat ttt        432
Gln Leu Tyr Glu Met Asn Pro Pro Glu Tyr Ala Trp Phe Tyr Asn Phe
    130                 135                 140

gta gtt aac aac aaa cct caa gac agt aag ctc ttt att cga gtg cta        480
Val Val Asn Asn Lys Pro Gln Asp Ser Lys Leu Phe Ile Arg Val Leu
145                 150                 155                 160

gtg aag gaa aag cag gaa ctg gct gaa agg gtt atg gtg acc cgg ttg        528
Val Lys Glu Lys Gln Glu Leu Ala Glu Arg Val Met Val Thr Arg Leu
                165                 170                 175

cat ctc ttt aac aaa tgg gtc aag aaa tac aat cac ata caa atg tat        576
His Leu Phe Asn Lys Trp Val Lys Lys Tyr Asn His Ile Gln Met Tyr
            180                 185                 190

cag gcc att aaa gac caa aac ttg cag ttg atg cgg gag agg ctg att        624
Gln Ala Ile Lys Asp Gln Asn Leu Gln Leu Met Arg Glu Arg Leu Ile
        195                 200                 205

caa aca gta aag ctg acc tct gat ggg gac aac att gat agg cct ccg        672
Gln Thr Val Lys Leu Thr Ser Asp Gly Asp Asn Ile Asp Arg Pro Pro
    210                 215                 220

ttg tga                                                                678
Leu
225

<210>  12
<211>  225
<212>  PRT
<213>  Physcomitrella patens

<400>  12

Met Val Gly Ala Thr Leu Val Gly Ala Ser Met Ala Gly Ala Val Gln
1               5                   10                  15

Ala Val Ser Leu Ala Gly Pro Pro Cys Arg Cys Thr Ser Phe Glu Met
            20                  25                  30

Ala Ala Gly Gly Val Val Val Ser Arg Lys Gln Gly His Ser Cys Ser

35                    40                    45

Met Ile Gln Arg Lys Ser Asn Thr Ile Val His Leu Tyr Ser Thr Met
    50                  55                  60

Thr Gln Ala Trp Gln Asp Ser Arg Leu Thr Ala Arg Ala Leu Ala His
65                  70                  75                  80

Thr Ile Pro Cys Ser Thr Ser Gly Ile His His Ala Pro Met Val Val
                85                  90                  95

Asn Glu Leu Gly Gly Gln Tyr Glu Asp Thr Phe Asp Asp Val Glu Lys
            100                 105                 110

His Leu Leu Asp Tyr Phe Thr Tyr Lys Ala Val Lys Thr Val Leu Ala
        115                 120                 125

Gln Leu Tyr Glu Met Asn Pro Pro Glu Tyr Ala Trp Phe Tyr Asn Phe
    130                 135                 140

Val Val Asn Asn Lys Pro Gln Asp Ser Lys Leu Phe Ile Arg Val Leu
145                 150                 155                 160

Val Lys Glu Lys Gln Glu Leu Ala Glu Arg Val Met Val Thr Arg Leu
                165                 170                 175

His Leu Phe Asn Lys Trp Val Lys Lys Tyr Asn His Ile Gln Met Tyr
            180                 185                 190

Gln Ala Ile Lys Asp Gln Asn Leu Gln Leu Met Arg Glu Arg Leu Ile
        195                 200                 205

Gln Thr Val Lys Leu Thr Ser Asp Gly Asp Asn Ile Asp Arg Pro Pro
    210                 215                 220

Leu
225

<210>  13
<211>  624
<212>  DNA
<213>  Oryza sativa

<220>
<221>  CDS
<222>  (1)..(624)

<400>  13
atg gca gcc act atc aga agc act ggg tgt tgt gcg att gcg aat gct        48

Met Ala Ala Thr Ile Arg Ser Thr Gly Cys Cys Ala Ile Ala Asn Ala
1               5                   10                  15

cga agt ttg agc cag act cag tac tca agc aag caa gaa agg tgg gaa      96
Arg Ser Leu Ser Gln Thr Gln Tyr Ser Ser Lys Gln Glu Arg Trp Glu
            20                  25                  30

aaa tcg tct agt gct gga tgt gtt ggt ggg aca ctg ggg gga acg cat      144
Lys Ser Ser Ser Ala Gly Cys Val Gly Gly Thr Leu Gly Gly Thr His
        35                  40                  45

gcc acg ggt tta gcg tct gtc cta ttc tgc aat aag acg ttc ctt ctg      192
Ala Thr Gly Leu Ala Ser Val Leu Phe Cys Asn Lys Thr Phe Leu Leu
    50                  55                  60

ccg aag aat aga agt cgg ccg aat ttt agg ttt gga cat gtt agt cct      240
Pro Lys Asn Arg Ser Arg Pro Asn Phe Arg Phe Gly His Val Ser Pro
65                  70                  75                  80

atc cgc agc aaa atg ttt gtg cca ggg ttt ggg gag gcg tct cct gaa      288
Ile Arg Ser Lys Met Phe Val Pro Gly Phe Gly Glu Ala Ser Pro Glu
                85                  90                  95

gcc aaa gca gct gca gca ttg cac aac ttt ttt act tat gtt gca gtc      336
Ala Lys Ala Ala Ala Ala Leu His Asn Phe Phe Thr Tyr Val Ala Val
            100                 105                 110

aaa ata gta gca tcc cag ctt gag gat tat aac aaa gaa gca tat gta      384
Lys Ile Val Ala Ser Gln Leu Glu Asp Tyr Asn Lys Glu Ala Tyr Val
        115                 120                 125

gac ctg atg gag ttt ttg gag aag atg cca ctg aat gat ggt gac aag      432
Asp Leu Met Glu Phe Leu Glu Lys Met Pro Leu Asn Asp Gly Asp Lys
    130                 135                 140

ttc att gca gca ttg atg cga gag tct ttc cgc cat aag aac ttg gct      480
Phe Ile Ala Ala Leu Met Arg Glu Ser Phe Arg His Lys Asn Leu Ala
145                 150                 155                 160

cta cgc atc atg gaa gtt aga tca gca tat gcc aat cag gac ttt gaa      528
Leu Arg Ile Met Glu Val Arg Ser Ala Tyr Ala Asn Gln Asp Phe Glu
                165                 170                 175

tgg gac aat ctc caa agc ttg tct ttt aaa caa ctc gag gag ggt aat      576
Trp Asp Asn Leu Gln Ser Leu Ser Phe Lys Gln Leu Glu Glu Gly Asn
            180                 185                 190

aag aca cta atg cgc aaa tac ctc acc gag acc agc aat atg gaa tga      624
Lys Thr Leu Met Arg Lys Tyr Leu Thr Glu Thr Ser Asn Met Glu
        195                 200                 205

<210>  14
<211>  207
<212>  PRT
<213>  Oryza sativa

<400>  14

Met Ala Ala Thr Ile Arg Ser Thr Gly Cys Cys Ala Ile Ala Asn Ala
1               5                   10                  15

Arg Ser Leu Ser Gln Thr Gln Tyr Ser Ser Lys Gln Glu Arg Trp Glu
            20                  25                  30

Lys Ser Ser Ser Ala Gly Cys Val Gly Gly Thr Leu Gly Gly Thr His
        35                  40                  45

Ala Thr Gly Leu Ala Ser Val Leu Phe Cys Asn Lys Thr Phe Leu Leu
    50                  55                  60

Pro Lys Asn Arg Ser Arg Pro Asn Phe Arg Phe Gly His Val Ser Pro
65                  70                  75                  80

Ile Arg Ser Lys Met Phe Val Pro Gly Phe Gly Glu Ala Ser Pro Glu
                85                  90                  95

Ala Lys Ala Ala Ala Ala Leu His Asn Phe Phe Thr Tyr Val Ala Val
            100                 105                 110

Lys Ile Val Ala Ser Gln Leu Glu Asp Tyr Asn Lys Glu Ala Tyr Val
        115                 120                 125

Asp Leu Met Glu Phe Leu Glu Lys Met Pro Leu Asn Asp Gly Asp Lys
    130                 135                 140

Phe Ile Ala Ala Leu Met Arg Glu Ser Phe Arg His Lys Asn Leu Ala
145                 150                 155                 160

Leu Arg Ile Met Glu Val Arg Ser Ala Tyr Ala Asn Gln Asp Phe Glu
                165                 170                 175

Trp Asp Asn Leu Gln Ser Leu Ser Phe Lys Gln Leu Glu Glu Gly Asn
            180                 185                 190

Lys Thr Leu Met Arg Lys Tyr Leu Thr Glu Thr Ser Asn Met Glu
        195                 200                 205

<210>  15
<211>  573
<212>  DNA
<213>  Chlamydomonas reinhardtii

<220>
<221>  CDS
<222>  (1)..(573)

<400>  15
atg atg agc ctc agc gca aga gcg gct ttt cgc gcg ccc ctc agc cat        48
Met Met Ser Leu Ser Ala Arg Ala Ala Phe Arg Ala Pro Leu Ser His
1               5                   10                  15

cat cgg ccg cgc ccc caa gcc tac ccc cgg gct gtt gtc acg ccg tgc     96
His Arg Pro Arg Pro Gln Ala Tyr Pro Arg Ala Val Val Thr Pro Cys
            20                  25                  30

gca agg atg cat atc cct gcg gat tct ttt tcg ggg gct tcg cca gag     144
Ala Arg Met His Ile Pro Ala Asp Ser Phe Ser Gly Ala Ser Pro Glu
        35                  40                  45

cgt aaa gct gct gta gcc ctg cgg tcg ctg ttc acg ttt gtt gca gct     192
Arg Lys Ala Ala Val Ala Leu Arg Ser Leu Phe Thr Phe Val Ala Ala
    50                  55                  60

cgg gtg gtg ctg gag cag ctg cag ggc ccc ggc ggc cct gag acc acc     240
Arg Val Val Leu Glu Gln Leu Gln Gly Pro Gly Gly Pro Glu Thr Thr
65                  70                  75                  80

tac aac cag cag gca tac cta gac ctg atg gac ttc ctg ggc acg ccc     288
Tyr Asn Gln Gln Ala Tyr Leu Asp Leu Met Asp Phe Leu Gly Thr Pro
                85                  90                  95

atg aag ggc gat ggc ggc gac gag tgg atg gcc gct gtc atg agg aag     336
Met Lys Gly Asp Gly Gly Asp Glu Trp Met Ala Ala Val Met Arg Lys
            100                 105                 110

aac cac gct ttg gcc ctg cgc ctg atg gag gtg cgc gag gcc tac ctg     384
Asn His Ala Leu Ala Leu Arg Leu Met Glu Val Arg Glu Ala Tyr Leu
        115                 120                 125

gac gag ttt gag tgg gga aag acc atg gag atg gcc agc cgc gag acg     432
Asp Glu Phe Glu Trp Gly Lys Thr Met Glu Met Ala Ser Arg Glu Thr
    130                 135                 140

cgc gag gcc aac aca cgc ctc atg cgc gcg gcg gcc atg gcc agc ctg     480
Arg Glu Ala Asn Thr Arg Leu Met Arg Ala Ala Ala Met Ala Ser Leu
145                 150                 155                 160

cag gcg tct ctg acg gag ccg gtg ggc ggc ggt gcc ggc gcc ggc tgc     528
Gln Ala Ser Leu Thr Glu Pro Val Gly Gly Gly Ala Gly Ala Gly Cys
                165                 170                 175

atg tct atg gag gac ctg gac ggc ccc ggc aag ggt gcc gcg tga         573
Met Ser Met Glu Asp Leu Asp Gly Pro Gly Lys Gly Ala Ala
            180                 185                 190

<210>  16
<211>  190
<212>  PRT
<213>  Chlamydomonas reinhardtii

<400>  16

Met Met Ser Leu Ser Ala Arg Ala Ala Phe Arg Ala Pro Leu Ser His
1               5                   10                  15

His Arg Pro Arg Pro Gln Ala Tyr Pro Arg Ala Val Val Thr Pro Cys
            20                  25                  30

Ala Arg Met His Ile Pro Ala Asp Ser Phe Ser Gly Ala Ser Pro Glu
        35                  40                  45

Arg Lys Ala Ala Val Ala Leu Arg Ser Leu Phe Thr Phe Val Ala Ala
    50                  55                  60

Arg Val Val Leu Glu Gln Leu Gln Gly Pro Gly Gly Pro Glu Thr Thr
65                  70                  75                  80

Tyr Asn Gln Gln Ala Tyr Leu Asp Leu Met Asp Phe Leu Gly Thr Pro
                85                  90                  95

Met Lys Gly Asp Gly Gly Asp Glu Trp Met Ala Ala Val Met Arg Lys
            100                 105                 110

Asn His Ala Leu Ala Leu Arg Leu Met Glu Val Arg Glu Ala Tyr Leu
        115                 120                 125

Asp Glu Phe Glu Trp Gly Lys Thr Met Glu Met Ala Ser Arg Glu Thr
    130                 135                 140

Arg Glu Ala Asn Thr Arg Leu Met Arg Ala Ala Ala Met Ala Ser Leu
145                 150                 155                 160

Gln Ala Ser Leu Thr Glu Pro Val Gly Gly Gly Ala Gly Ala Gly Cys
                165                 170                 175

Met Ser Met Glu Asp Leu Asp Gly Pro Gly Lys Gly Ala Ala
            180                 185                 190

<210>  17
<211>  585
<212>  DNA
<213>  Zea mays

<220>
<221>  CDS
<222>  (1)..(585)

<400>  17
atg tcc gga gtt cag gcg atg ccg gcg gcc gtg ggc gtg gcg ggg ctg        48
Met Ser Gly Val Gln Ala Met Pro Ala Ala Val Gly Val Ala Gly Leu
1               5                   10                  15

aga ggg tca ggg ttg gtt agc agc agc agt acc ggc ttg ttc gcc ggc        96
Arg Gly Ser Gly Leu Val Ser Ser Ser Ser Thr Gly Leu Phe Ala Gly
            20                  25                  30

gac tgc aag agg aag gct cgt cgc ccg gcg tgc tcg gcc tgg gcc ctc       144
Asp Cys Lys Arg Lys Ala Arg Arg Pro Ala Cys Ser Ala Trp Ala Leu
        35                  40                  45

agg cag agc cgg atc ggc agc agg agc agg agc aag agg ggc ctc ggc       192
Arg Gln Ser Arg Ile Gly Ser Arg Ser Arg Ser Lys Arg Gly Leu Gly
    50                  55                  60

atc gtc tgc aac ctc gcg gga cag tac gag gat tcc ttc gac gac gtc     240
Ile Val Cys Asn Leu Ala Gly Gln Tyr Glu Asp Ser Phe Asp Asp Val
65                  70                  75                  80

cag cgg caa ctg atg aac tac ttc acc tac aag gcc gtg agg acc gtg     288
Gln Arg Gln Leu Met Asn Tyr Phe Thr Tyr Lys Ala Val Arg Thr Val
                85                  90                  95

ctg acc cag ctc tac gag ctg aac ccg ccg agc tac cgc tgg ttc tac     336
Leu Thr Gln Leu Tyr Glu Leu Asn Pro Pro Ser Tyr Arg Trp Phe Tyr
            100                 105                 110

aac ttc gtc gct gtc aac aag cct acc gac ggc aag cat ttc ctc cgt     384
Asn Phe Val Ala Val Asn Lys Pro Thr Asp Gly Lys His Phe Leu Arg
        115                 120                 125

gct ctc ggc aag gag agg cag gag ctc gca gag cga gtg atg gtc acc     432
Ala Leu Gly Lys Glu Arg Gln Glu Leu Ala Glu Arg Val Met Val Thr
    130                 135                 140

agg ctt cac ctg tac ggg aaa tgg ata aag aaa tgc gac cat gcc aag     480
Arg Leu His Leu Tyr Gly Lys Trp Ile Lys Lys Cys Asp His Ala Lys
145                 150                 155                 160

acg tac gag aag atc tcc gac gag aac ctg gcg ctg atg cgg gag cgg     528
Thr Tyr Glu Lys Ile Ser Asp Glu Asn Leu Ala Leu Met Arg Glu Arg
                165                 170                 175

ctg atg gag acg gtc atc tgg cca acc gac gac agc agc acg gag aag     576
Leu Met Glu Thr Val Ile Trp Pro Thr Asp Asp Ser Ser Thr Glu Lys
            180                 185                 190

atc ggc tga                                                         585
Ile Gly

<210>  18
<211>  194
<212>  PRT
<213>  Zea mays

<400>  18

Met Ser Gly Val Gln Ala Met Pro Ala Ala Val Gly Val Ala Gly Leu
1               5                   10                  15

Arg Gly Ser Gly Leu Val Ser Ser Ser Ser Thr Gly Leu Phe Ala Gly
            20                  25                  30

Asp Cys Lys Arg Lys Ala Arg Arg Pro Ala Cys Ser Ala Trp Ala Leu
        35                  40                  45

Arg Gln Ser Arg Ile Gly Ser Arg Ser Arg Ser Lys Arg Gly Leu Gly
    50                  55                  60

Ile Val Cys Asn Leu Ala Gly Gln Tyr Glu Asp Ser Phe Asp Asp Val
65                  70                  75                  80

Gln Arg Gln Leu Met Asn Tyr Phe Thr Tyr Lys Ala Val Arg Thr Val
                85                  90                  95

Leu Thr Gln Leu Tyr Glu Leu Asn Pro Pro Ser Tyr Arg Trp Phe Tyr
            100                 105                 110

Asn Phe Val Ala Val Asn Lys Pro Thr Asp Gly Lys His Phe Leu Arg
        115                 120                 125

Ala Leu Gly Lys Glu Arg Gln Glu Leu Ala Glu Arg Val Met Val Thr
    130                 135                 140

Arg Leu His Leu Tyr Gly Lys Trp Ile Lys Lys Cys Asp His Ala Lys
145                 150                 155                 160

Thr Tyr Glu Lys Ile Ser Asp Glu Asn Leu Ala Leu Met Arg Glu Arg
                165                 170                 175

Leu Met Glu Thr Val Ile Trp Pro Thr Asp Asp Ser Ser Thr Glu Lys
            180                 185                 190

Ile Gly